# EUROPÄISCHE PATENTSCHRIFT

(11) **EP 0 715 629 B1**
(45) Veröffentlichungstag und Bekanntmachung des Hinweises auf die Patenterteilung: **29.01.1997**
(21) Anmeldenummer: 94924855.3
(22) Anmeldetag: 08.08.1994
(51) Int. Cl.: C07D 521/00, A01N 47/36, C07D 401/12

(54) **N-HETEROARYL-N-(PYRID-2-YL-SULFONYL)-HARNSTOFFE, VERFAHREN ZU IHRER HERSTELLUNG UND IHRE VERWENDUNG ALS HERBIZIDE UND PFLANZENWACHSTUMSREGULATOREN**
N-HETEROARYL-N'(PYRID-2-YL-SULPHONYL) UREAS, PROCESSES FOR THE MANUFACTURE THEREOF, USES THEREOF AS HERBICIDES AND PLANT GROWTH REGULATORS
N-HETEROARYL-N'-(PYRID-2-YL-SULFONYL)UREES, LEUR PROCEDE DE PREPARATION ET LEUR UTILISATION COMME HERBICIDES ET REGULATEURS DE CROISSANCE VEGETALE

(30) Priorität: 24.08.1993 DE 4328397; 19.10.1993 DE 4335587
(43) Veröffentlichungstag der Anmeldung: 12.06.1996
(73) Patentinhaber: Hoechst Schering AgrEvo GmbH, 13509 Berlin (DE)
(72) Erfinder: HAAF, Klaus, D-65779 Kelkheim (DE); KEHNE, Heinz, D-65719 Hofheim am Taunus (DE); BAUER, Klaus, D-63456 Hanau (DE); BIERINGER, Hermann, D-65817 Eppstein/Taunus (DE)
(86) Internationale Anmeldenummer: EP9402628
(87) Internationale Veröffentlichungsnummer: WO9506049

(56) Entgegenhaltungen:
- WO-A-91/10660

## Beschreibung

Die Erfindung betrifft das technische Gebiet der Herbizide und Pflanzenwachstumsregulatoren, insbesondere der Herbizide zur selektiven Bekämpfung von Unkräutern und Ungräsern in Nutzpflanzenkulturen.

Es ist bekannt, daß einige 2-Pyridylsulfonylharnstoffe herbizide und pflanzenwachstumsregulierende Eigenschaften besitzen; vergleiche EP-A-13 480, EP-A-272 855, EP-A-84224, US-A-4421550, EP-A-103543 (US-A-4 579 583) US-A-4487626, EP-A-125864, WO 88/04297 und WO 91/10660 (ZA 91/0173).

Es wurden nun weitere 2-Pyridylsulfonylharnstoffe mit speziellen Resten in 3-Position des Pyridylrestes gefunden, die als Herbizide und Pflanzenwachstumsregulatoren geeignet sind.

Gegenstand der vorliegenden Erfindung sind Verbindungen der Formel (I) oder deren Salze, 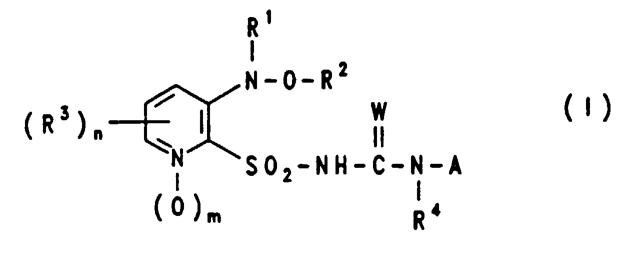 worin
- R¹: H, (C₁-C₆)Alkyl, das unsubstituiert oder durch einen oder mehrere Reste aus der Gruppe Halogen, Nitro, (C₁-C₄)Alkoxy, (C₃-C₆)Cycloalkyl, Cyano, Aryl und substituiertes Aryl substituiert ist, (C₃-C₆)Cycloalkyl, das unsubstituiert oder durch einen oder mehrere Reste aus der Gruppe Halogen, (C₁-C₄)Alkyl und (C₁-C₄)Alkoxy substituiert ist, oder (C₂-C₆)Alkenyl oder (C₂-C₆)Alkinyl, wobei jeder der beiden letztgenannten Reste unsubstituiert oder durch einen oder mehrere Reste aus der Gruppe Halogen, (C₁-C₄)Alkoxy und (C₃-C₆)Cycloalkyl substituiert ist, oder Aryl, substituiertes Aryl, oder einen Acylrest der Formel

-CO-R*,

worin
R* H, (C₁-C₆)Alkyl, (C₂-C₆)Alkenyl oder (C₂-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, Nitro, Cyano, Thiocyanato, Aryl und substituiertes Aryl substituiert ist, oder (C₁-C₄)Alkoxy, (C₂-C₄)Alkenyloxy, (C₂-C₄)Alkinyloxy oder (C₁-C₄)Alkylthio, wobei jeder der letztgenannten vier Reste unsubstituiert oder durch einen oder mehrere Reste aus der Gruppe Halogen, (C₁-C₄)Alkoxy, (C₁-C₄)Alkylthio, Aryl und substituiertes Aryl substituiert ist, oder (C₃-C₆)Cycloaykyl oder (C₃-C₆)Cycloalkoxy, wobei jeder der letztgenannten beiden Reste unsubstituiert oder durch einen oder mehrere Reste aus der Gruppe (C₁-C₄)Alkyl, (C₁-C₄)Alkoxy, (C₁-C₄)Alkylthio, (C₁-C₄)Haloalkyl, und Halogen substituiert ist, oder einen Rest der Formel NR^{a}R^{b} bedeutet,
- R²: H, (C₁-C₆)Alkyl, das unsubstituiert oder durch einen oder mehrere Reste aus der Gruppe Halogen, Nitro, (C₁-C₄)Alkoxy, (C₃-C₆)Cycloalkyl, Aryl und substituiertes Aryl substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, (C₁-C₄)Haloalkyl, Aryl und substituiertes Aryl substituiert ist, oder (C₂-C₆)Alkenyl oder (C₂-C₆)Alkinyl, wobei jeder der letztgenannten zwei Reste unsubstituiert oder durch einen oder mehrere Reste aus der Gruppe (C₁-C₄)Alkoxy, (C₁-C₄)Haloalkyl, (C₃-C₆)Cycloalkyl und Halogen substituiert ist, oder Aryl, substituiertes Aryl oder einen Rest der Formel R'R"R"'Si, worin R', R", R"' unabhängig voneinander (C₁-C₄)Alkyl bedeuten, oder einen Acylrest der Formel

-CO-R**,

worin
R** H, (C₁-C₆)Alkyl, (C₂-C₆)Alkenyl oder (C₂-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, Nitro, Cyano, Thiocyanato, Aryl und substituiertes Aryl substituiert ist, oder (C₁-C₄)Alkoxy, (C₂-C₄)Alkenyloxy oder (C₂-C₄)Alkinyloxy, wobei jeder der letztgenannten drei Reste unsubstituiert oder durch einen oder mehrere Reste aus der Gruppe Halogen, (C₁-C₄)Alkoxy, (C₁-C₄)Alkylthio, Aryl und substituiertes Aryl substituiert ist, oder (C₃-C₆)Cycloalkyl oder (C₃-C₆)Cycloalkoxy, wobei jeder der letztgenannten beiden Reste unsubstituiert oder durch einen oder mehrere Reste aus der Gruppe Halogen, (C₁-C₄)Alkyl, (C₁-C₄)Alkoxy, (C₁-C₄)Alkylthio und (C₁-C₄)Haloalkyl substituiert ist, oder einen Rest der Formel NR^{c}R^{d} bedeutet,
- R³: (C₁₋C₄)Alkyl, (C₁-C₃)Haloalkyl, Halogen, NO₂, CN, (C₁-C₃)Alkoxy, (C₁-C₃)Haloalkoxy, (C₁-C₃)Alkylthio, (C₁-C₃)Alkoxy-(C₁-C₃)alkyl, [(C₁-C₃)Alkoxy]-carbonyl, (C₁-C₃)Alkylamino, Di[(C₁-C₃)alkyl]-amino, (C₁-C₃)-Alkylsulfinyl, (C₁-C₃)Alkylsulfonyl, SO₂NR^{e}R^{f} oder C(O)NR^{g}R^{h},
R^{a}, R^{b}, R^{c}, R^{d}, R^{e}, R^{f}, R^{g} und R^{h} unabhängig voneinander H, (C₁-C₄)Alkyl, (C₃-C₆)Alkenyl, (C₃-C₆)Alkinyl, [(C₁-C₄)Alkyl]-carbonyl, Arylcarbonyl, das im Arylrest unsubstituiert oder durch einen oder mehrere Reste aus der Gruppe Halogen, (C₁-C₄)Alkyl und (C₁-C₄)Alkoxy substituiert ist, oder paarweise R^{a} und R^{b}, R^{c} und R^{d}, R^{e} und R^{f} bzw. R^{g} und R^{h} gemeinsam mit dem sie verbindenden N-Atom einen heterocyclischen gesättigten oder ungesättigten Ring mit 3 bis 7 Ringatomen, der neben dem N-Atom 1 oder 2 weitere Heteroatome aus der Gruppe N, O und S in den möglichen Oxidationsstufen enthalten kann und der unsubstituiert oder substituiert ist,
- R⁴: H oder (C₁-C₄)Alkyl, vorzugsweise H oder CH₃, insbesondere H,
- m: 0 oder 1, vorzugsweise 0,
- n: 0, 1 oder 2, vorzugsweise 0 oder 1,
- A: einen Rest der Formel 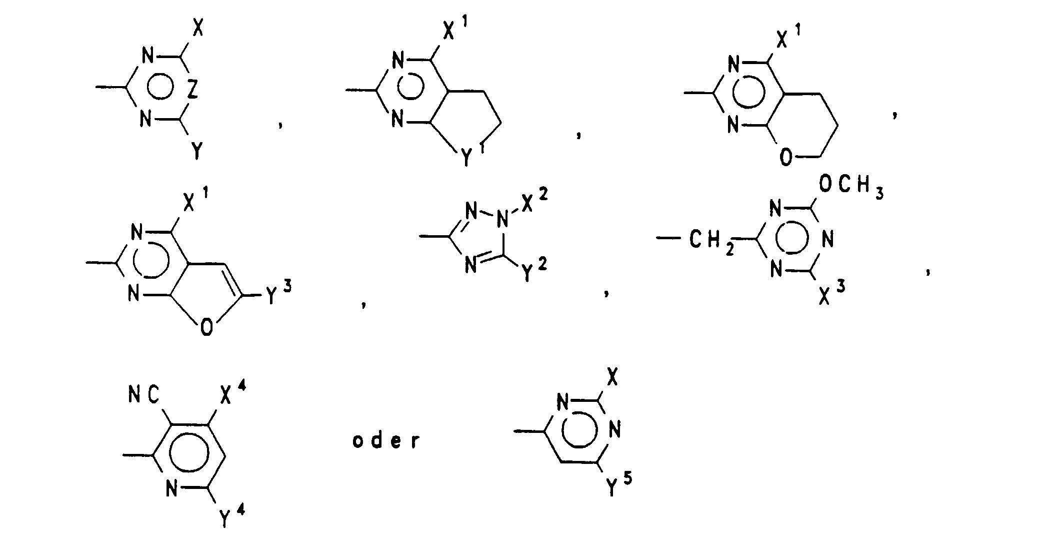
- X,Y: unabhängig voneinander H, Halogen, (C₁-C₃)Alkyl, (C₁-C₃)Alkoxy oder (C₁-C₃)Alkylthio, wobei die vorgenannten alkylhaltigen Reste unsubstituiert oder ein- oder mehrfach durch Halogen oder ein- oder zweifach durch (C₁-C₃)Alkoxy oder (C₁-C₃)Alkylthio substituiert sind, oder einen Rest der Formel NR⁵R⁶, (C₃-C₆)-Cycloalkyl, (C₂-C₄)Alkenyl, (C₂-C₄)Alkinyl, (C₃-C₄)-Alkenyloxy oder (C₃-C₄)Alkinyloxy,
- R⁵,R⁶: unabhängig voneinander H, (C₁-C₃)Alkyl oder (C₃-C₄)Alkenyl,
- W: O oder S, vorzugsweise O,
- Z: CH oder N, vorzugsweise CH,
- X¹: CH₃, OCH₃, OC₂H₅ oder OCF₂H,
- Y¹: -O- oder -CH₂-,
- X²: CH₃, C₂H₅ oder CH₂CF₃,
- Y²: OCH₃, OC₂H₅, SCH₃, SC₂H₅, CH₃ oder C₂H₅,
- X³: CH₃ oder OCH₃,
- Y³: H oder CH₃,
- X⁴: CH₃, OCH₃, OC₂H₅, CH₂OCH₃ oder Cl,
- Y⁴: CH₃, OCH₃, OC₂H₅ oder Cl,
- Y⁵: CH₃, C₂H₅, OCH₃ oder Cl
bedeuten.

In der Formel (I) und im folgenden können kohlenwasserstoffhaltige Reste wie z.B. Alkyl-, Alkoxy-, Haloalkyl- und Alkylthioreste sowie die entsprechenden ungesättigten und/oder substituierten Reste im Kohlenwasserstofftell jeweils geradkettig oder verzweigt sein. Alkylreste, auch in den zusammengesetzten Bedeutungen wie Alkoxy, Haloalkyl usw., bedeuten Methyl, Ethyl, n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet Fluor, Chlor, Brom oder Jod. Haloalkyl bedeutet Alkyl, das durch ein oder mehrere Atome aus der Gruppe Halogen substituiert ist; Haloalkyl ist beispielsweise CF₃, CHF₂, CH₂CF₃. Aryl ist beispielsweise Phenyl, Naphthyl, Tetrahydronaphthyl, Indanyl, Fluorenyl und ähnliches, vorzugsweise Phenyl. Substituiertes Aryl oder substituiertes Phenyl bedeutet vorzugsweise Aryl bzw. Phenyl, das durch einen oder mehrere, vorzugsweise 1 bis 3 Reste aus der Gruppe Halogen, Alkyl, Haloalkyl, Haloalkoxy, Nitro, Cyano, Alkoxycarbonyl, Alkanoyl, Carbamoyl, Mono- und Di-alkylaminocarbonyl, Mono- und Dialkylamino, Alkylsulfinyl oder Alkylsulfonyl substituiert ist, wobei bei den alkylhaltigen Resten solche mit 1 bis 4 C-Atomen, insbesondere 1 bis 2 C-Atomen bevorzugt sind; besonders bevorzugt sind dabei Methyl, Methoxy und Chlor. Beispiele für heterocyclische Reste NR^{a}R^{b}, NR^{c}R^{d}, NR^{e}R^{f} oder NR^{g}R^{h} sind gegebenenfalls substituiertes Pyrrol, Imidazol, Pyrazol, Triazol, Pyrazolon, Oxazol, Oxazolon, Propansultam, Butansultam, Pyrrolidon, Piperidin und Morpholin. Als Substituenten in substituierten heterocyclischen Resten kommen solche in Frage, die für Cycloalkyl- und Aryl-Reste genannt wurden, insbesondere (C₁-C₄)Alkyl, (C₁-C₄)Alkoxy, (C₁-C₄)Haloalkyl,(C₁-C₄)Haloalkoxy und Halogen.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formeln (I) nicht gesondert angegeben sind. Die durch ihre spezifische Raumform definierten möglichen Stereoisomeren, wie Enantiomere, Diastereomere, Z- und E-lsomere sind jedoch alle von den Formeln (I) umfaßt und können nach üblichen Methoden aus Gemischen der Stereoisomeren erhalten werden oder auch durch stereoselektive Reaktionen in Kombination mit dem Einsatz von stereochemisch reinen Ausgangsstoffen hergestellt werden.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metallsalze, insbesondere Alkalisalze (Na-, K-Salze) oder Erdalkalisalze, oder auch Ammoniumsalze oder Salze mit organischen Aminen. Ebenso kann Salzbildung durch Anlagerung einer starken Säure an den Pyridinteil der Verbindung der Formel (I) erfolgen. Geeignete Säuren hierfür sind starke anorganische und organische Säuren, beispielsweise HCI, HBr, H₂SO₄ oder HNO₃.

Von besonderem Interesse sind erfindungsgemäße Verbindungen (I), worin R¹ H, (C₁-C₄)Alkyl, das unsubstituiert oder durch einen oder mehrere Reste aus der Gruppe Halogen, vorzugsweise F, Cl und Br, (C₁-C₄)Alkoxy, (C₃-C₆)Cycloalkyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, oder (C₃-C₆)Cycloalkyl, (C₂-C₄)Alkenyl, (C₂-C₄)Haloalkenyl,(C₂-C₄)Alkinyl, Phenyl, substituiertes Phenyl oder einen Rest der Formel

-CO- R*,

worin
- R*: H, (C₁-C₄)Alkyl, (C₂-C₄)Alkenyl oder (C₂-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylsulfonyl, Phenyl und substituiertes Phenyl substituiert ist, oder (C₁-C₄)Alkoxy, das unsubstituiert oder durch einen oder mehrere Reste aus der Gruppe Halogen, (C₁-C₄)Alkoxy, (C₁-C₄)Alkylthio, Phenyl und substituiertes Aryl substituiert ist, oder (C₃-C₆)Cycloalkyl oder (C₃-C₆)Cycloalkoxy, wobei jeder der letztgenannten beiden Reste unsubstituiert oder durch einen oder mehrere Reste aus der Gruppe (C₁-C₂)Alkyl, (C₁-C₂)Alkoxy und Halogen substituiert ist, oder einen Rest der Formel NR^{a}R^{b}
bedeutet, bedeutet.

Bevorzugt sind erfindungsgemäße Verbindungen der Formel (I) oder deren Salze, in denen R¹ Wasserstoff, (C₁-C₄)Alkyl, das unsubstituiert oder durch 1 bis 3 Reste aus der Gruppe Halogen oder durch (C₁-C₂)Alkoxy oder (C₃-C₅)Cycloalkyl substituiert ist, bedeutet.

Weiterhin sind erfindungsgemäße Verbindungen der Formel (I) oder deren Salze bevorzugt, in denen R¹ einen Rest der Formel -CO-R* bedeutet, worin R* Wasserstoff, (C₁-C₄)Alkyl, das unsubstituiert oder durch einen oder mehrere Halogenatome oder durch (C₁-C₂)Alkoxy substituiert ist, oder (C₂-C₄)Alkenyl, (C₂-C₃)Alkinyl, (C₃-C₅)Cycloalkyl, (C₁-C₄)Alkoxy, das unsubstituiert oder durch ein oder mehrere Halogenatome oder durch Phenyl substituiert ist, oder einen Rest der Formel NR^{a}R^{b}, worin R^{a} und R^{b} unabhängig voneinander H oder (C₁-C₄)Alkyl sind, bedeutet.

Von besonderem Interesse sind auch erfindungsgemäße Verbindungen der Formel (I) und deren Salze, worin
- R²: H, (C₁-C₄)Alkyl, das unsubstituiert oder durch einen oder mehrere Reste aus der Gruppe Halogen, (C₁-C₄)Alkoxy, (C₃-C₆)Cycloalkyl, Phenyl und substituiertes Phenyl substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy oder (C₁-C₄)Haloalkyl substituiert ist, oder (C₂-C₄)Alkenyl oder (C₂-C₄)Alkinyl, wobei jeder der letztgenannten zwei Reste unsubstituiert oder durch einen oder mehrere Reste aus der Gruppe (C₁-C₄)Alkoxy, (C₁-C₄)Haloalkyl und Halogen substituiert ist, oder Phenyl, substituiertes Phenyl oder einen Rest der Formel R'R"R"'Si, worin R', R", R"' unabhängig voneinander (C₁-C₄)Alkyl bedeuten, oder einen Acylrest der Formel

-O-R**,

worin
R ** H, (C₁-C₄)Alkyl, (C₂-C₄)Alkenyl oder (C₂-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)-Alkylsulfonyl, Phenyl und substituiertes Phenyl substituiert ist, oder (C₁-C₄)Alkoxy, (C₃-C₄)Alkenyloxy oder (C₃-C₄)Alkinyloxy, wobei jeder der letztgenannten drei Reste unsubstituiert oder durch einen oder mehrere Reste aus der Gruppe Halogen, (C₁-C₄)Alkoxy, (C₁-C₄)Alkylthio, Phenyl und substituiertes Phenyl substituiert ist, oder (C₃-C₆)Cycloalkyl oder (C₃-C₆)Cycloalkoxy, wobei jeder der letztgenannten beiden Reste unsubstituiert oder durch einen oder mehrere Reste aus der Gruppe Halogen, (C₁-C₄)Alkyl, (C₁-C₄)Alkoxy, und (C₁-C₄)Haloalkyl substituiert ist, oder einen Rest der Formel NR^{c}R^{d} bedeutet, bedeutet.

Weiter bevorzugt sind erfindungsgemäße Verbindungen der Formel (I) und deren Salze, worin
- R²: H, (C₁-C₄)Alkyl, (₁-C₄)Haloalkyl, [(C₁-C₄)Alkoxy]-(C₁-C₄)Alkyl oder einen Rest der Formel -CO-R**, worin
R** H, (C₁-C₄)Alkyl, (C₁-C₄)Haloalkyl, (C₃-C₄)Alkenyl, (C₃-C₄)Alkinyl, (C₁-C₄)Alkoxy, [(C₁-C₂)Alkoxy)-(C₁-C₂)Alkyl, (C₃-C₅)Cycloalkyl oder einen Rest der Formel NR^{c}R^{d}, worin R^{c} und R^{d} unabhängig voneinander H oder (C₁-C₄)Alkyl bedeuten, bedeutet,
bedeutet.
- R³: ist vorzugsweise (C₁-C₃)Alkyl, (C₁-C₃)Haloalkyl, Halogen, NO₂, (C₁-C₃)Alkoxy, (C₁-C₃)Haloalkoxy, (C₁-C₂)Alkoxy-(C₁-C₂)alkyl, Acetyl, Propionyl, (C₁-C₂)Alkylamino, Di[(C₁-C₂)alkyl]-amino, (C₁-C₃)Alkylsulfonyl, SO₂NR^{e}B^{f} oder C(O)NR^{g}R^{h}.

R^{a}, R^{b}, R^{c}, R^{d}, R^{e}, R^{f}, R^{g} und R^{h} sind unabhängig voneinander vorzugsweise H, (C₁-C₄)Alkyl, Allyl, But-2-enyl, But-3-enyl, Propargyl, But-2-inyl, But-3-inyl, Propionyl, Acetyl, Phenylcarbonyl, das im Phenylrest unsubstituiert oder durch einen oder mehrere Reste aus der Gruppe Halogen, (C₁-C₄)Alkyl und (C₁-C₄)Alkoxy substituiert ist, oder paarweise R^{a} und R^{b}, R^{c} und R^{d}, R^{e} und R^{f} bzw. R^{g} und R^{h} gemeinsam mit dem sie verbindenden N-Atom einen heterocyclischen gesättigten oder ungesättigten Ring mit 5 oder 6 Ringatomen, der neben dem N-Atom 1 oder 2 weitere Heteroatome aus der Gruppe N, O und S in den möglichen Oxidationsstufen enthalten kann und der unsubstituiert oder durch einen oder mehrere Reste aus der Gruppe Methyl, Ethyl, Methoxy, Ethoxy, Halogen, (C₁-C₂)Haloalkyl substituiert ist.

Weiter bevorzugt sind erfindungsgemäße Verbindungen der Formel (I) oder deren Salze, worin
- R³: (C₁-C₄)Alkyl, (C₁-C₃)Haloalkyl, Halogen, (C₁-C₃)Alkoxy oder Nitro und
- n: 0, 1 oder 2, vorzugsweise 0 oder 1, bedeuten.

Weiter bevorzugt sind Verbindungen der Formel (I) oder deren Salze, worin
- R¹: H, (C₁-C₄)Alkyl, (C₁C₄)Haloalkyl oder [(C₁-C₂)Alkoxy]-(C₁-C₂)alkyl, oder einen Rest der Formel -CO-R*, worin
R* H, (C₁-C₄)Alkyl, Halo-(C₁-C₄)alkyl, (C₂-C₄)Alkenyl, (C₂-C₄)Alkinyl, (C₁-C₄)Alkoxy, [(C₁-C₂)Alkoxy]-(C₁-C₂)alkyl, (C₃-C₅)Cycloalkyl oder einen Rest der Formel NR^{a}R^{b}, worin R^{e} und R^{b} unabhängig voneinander H oder (C₁-C₄)Alkyl bedeuten,
- R²: H, (C₁-C₄)Alkyl, (₁-C₄)Haloalkyl, [(C₁-C₂)Alkoxy]-(Cᵢ-C₂)Alkyl oder einen Rest der Formel -CO-R**, worin
R** H, (C₁-C₄)Alkyl, (C₁-C₄)Haloalkyl, (C₃-C₄)Alkenyl, (C₃-C₄)Alkinyl, (C₁-C₄)Alkoxy, [(C₁-C₄)Alkoxy]-(C₁-C₂)Alkyl, (C₃-C₅)Cycloalkyl oder einen Rest der Formel NR^{c}R^{d}, worin R^{c} und R^{d} unabhängig voneinander H oder (C₁-C₄)Alkyl bedeuten,
- R³: (C₁-C₄)Alkyl, Halogen, Nitro oder (C₁-C₄)Alkoxy und
- n: 0 oder 1 bedeuten.

Weiterhin bevorzugt sind dabei erfindungsgemäße Verbindungen der Formel (I) oder deren Salze, worin R¹ H, CH₃ oder C₂H₅ und R* H, (C₁-C₄)Alkyl, (C₁-C₂)Haloalkyl, Vinyl, Cyclopropyl, Cyclobutyl, (C₁-C₂)Alkoxy oder N(CH₃)₂, insbesondere H, bedeuten.

Weiterhin bevorzugt sind erfindungsgemäße Verbindungen der Formel (I) oder deren Salze, worin A ein Rest der Formel 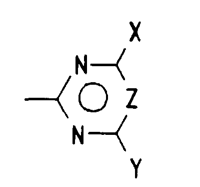 ist.

Vorzugsweise ist einer der Reste X und Y (C₁-C₃)Alkyl, (C₁-C₃)Alkoxy, (C₁-C₃)Haloalkyl, (C₁-C₃)Haloalkoxy oder [(C₁-C₂)Alkoxy]-(C₁-C₂)alkyl und der andere Rest Y bzw. X (C₁-C₃)Alkyl, (C₁-C₃)Alkoxy, (C₁-C₃)Alkylthio, wobei jeder der letztgenannten 3 Reste unsubstituiert oder ein- oder mehrfach durch Halogen oder ein- oder zweifach durch (C₁-C₃)Alkoxy oder (C₁-C₃)Alkylthio substituiert ist, oder Halogen oder ein Rest der Formel NR⁵R⁶, worin R⁵ und R⁶ unabhängig voneinander H, (C₁-C₃)Alkyl oder (C₃-C₄)Alkenyl sind, oder (C₃-C₆)Cycloalkyl, (C₂-C₄)Alkenyl, (C₂-C₄)Alkinyl, (C₃-C₄)Alkenyloxy oder (C₃-C₄)Alkinyloxy.

Noch mehr bevorzugt bedeutet einer der Reste X und Y (C₁-C₂)Alkyl, (C₁-C₂)Alkoxy oder OCF₂H und der andere Rest Y bzw. X (C₁-C₂)Alkyl, (C₁-C₂)Alkoxy, Halogen, OCF₂H, OCH₂CF₃ oder CF₃.

Insbesondere sind X und Y unabhängig voneinander (C₁-C₂)Alkyl oder (C₁-C₂)Alkoxy.

Bevorzugt sind auch erfindungsgemäße Verbindungen der Formel (I) oder deren Salze, die eine Kombination von zwei oder mehreren der bevorzugt genannten Bedeutungen (Merkmale) aufweisen.

Weiterer Gegenstand der vorliegenden Erfindung sind Verfahren zur Herstellung der erfindungsgemäßen Verbindungen der Formel (I) oder deren Salze, dadurch gekennzeichnet, daß man
a) eine Verbindung der Formel (II), 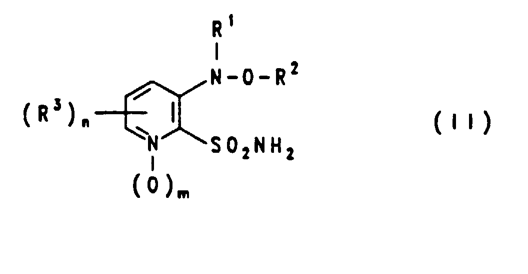 mit einem heterocyclischen Carbamat der Formel (III),

   R°-O-CO-NR⁴-A (III)

   worin R° gegebenenfalls substituiertes Phenyl oder (C₁-C₄)Alkyl bedeutet, umsetzt oder
b) ein Pyridylsulfonylcarbamat der Formel (IV), 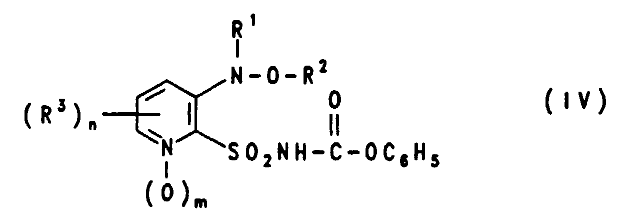 mit einem Aminoheterocyclus der Formel (V)

   H-NR⁴-A (V)

   umsetzt oder
c) ein Sulfonylisocyanat der Formel (Vl) 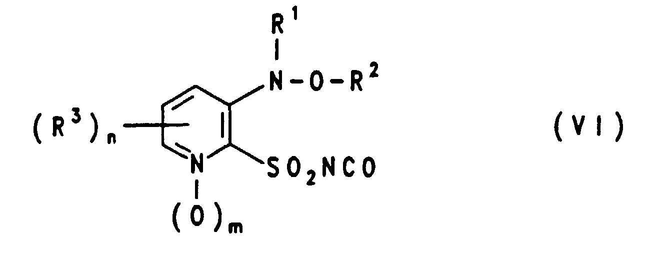 mit einem Aminoheterocyclus der Formel (V)

   H-NR⁴-A (V)

   umsetzt oder
d) in einer Eintopfreaktion zunächst einen Aminoheterocyclus der Formel H-NR⁴-A (V) in Gegenwart einer Base, wie z.B. Triethylamin, mit Phosgen umsetzt und das gebildete Intermediat mit einem Pyridinsulfonamid der Formel (II) umsetzt, oder
e) ein Sulfonamid der genannten Formel (II) mit einem (Thio-)lsocyanat der Formel (VII) 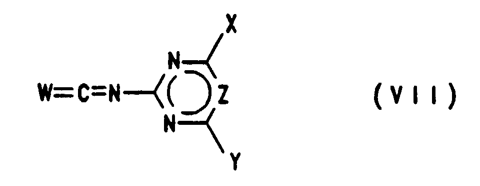 in Gegenwart einer Base, z.B. Kaliumcarbonat oder Triethylamin, umsetzt,

wobei in den Formeln (II)-(VII) die Reste R¹, R², R³, R⁴, A, W, X, Y und Z sowie m und n wie in Formel (I) definiert sind.

Die Umsetzung der Verbindungen der Formeln (II) und (III) erfolgt vorzugsweise basenkatalysiert in einem inerten organischen Lösungsmittel, wie z. B. Dichlormethan, Acetonitril, Dioxan oder THF bei Temperaturen zwischen 0° C und dem Siedepunkt des Lösungsmittels. Als Base wird beispielsweise 1,8-Diazabicyclo-[5.4.0]undec-7-en (DBU), Triethylamin oder Trimethylaluminium oder Triethylaluminium verwendet.

Die Sulfonamide (II) können aus N-geschützten 3-(N-Hydroxylamino)-pyrid-2-yl-sulfonamiden (VIII) 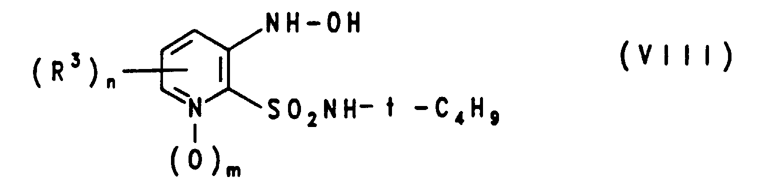 hergestellt werden.

Die Sulfonamide (II) und die geschützten Sulfonamide (VIII) sind neue Verbindungen. Sie und ihre Herstellung sind ebenfalls Gegenstand der Erfindung.

Man erhält die Verbindungen der Formel (II) z. B. ausgehend von Verbindungen der Formel (VIII) durch Umsetzung mit Carbonsäurehalogeniden der Formel Hal-CO-R* (Hal = Halogen, vorzugsweise Chlor) oder symmetrischen oder gemischten Carbonsäureanhydriden der Formel R*-CO-O-COR* oder der Formel R**-CO-O-COR⁺, worin R⁺ analog R* bzw. R** definiert ist oder ein anderer aliphatischer oder aromatischer Rest ist, wobei in einem ersten Schritt die O-Acylverbindungen gebildet werden und bei weiterer Zugabe des Acylierungsreagenzes die N,O-Diacylverbindungen entstehen, (R¹ = COR*, bzw. R² = COR**). Die zunächst erhaltenen diacylierten t-butylgeschützten Sulfonamide werden mit Säuren, z.B. mit Trifluoressigsäure (s.u.) in die freien Sulfonamide (II) (R¹ = COR*, R² = COR**) überführt.

Strukturen der Formel (II), bei denen R¹ = COR* und R² = H bedeuten, erhält man, indem die oben beschriebenen N,O-Diacylverbindungen z.B. mit verdünnter Natronlauge (z.B. 2n NaOH) verseift werden. Strukturen der Formel (II), bei denen R¹ = H und R² = Alkyl bedeuten, erhält man, indem die Verbindungen der Formel (VIII) mit Alkylierungsreagenzien wie z.B. Methyliodid oder Dimethylsulfat unter Zusatz geeigneter Basen wie z.B. Kaliumhydroxid, DBU, Pyridin, Triethylamin oder Natriumethanolat umgesetzt werden. Diese könnten dann in einem weiteren Schritt mit Acylierungsreagentien, wie oben beschrieben, zu Verbindungen umgesetzt werden, bei denen R¹ = COR* und R² = Alkyl oder substituiertes Alkyl bedeuten.

Eine weitere Derivatisierung ist analog literaturbekannter Methoden (siehe Houben-Weyl, Methoden der organischen Chemie, Band E 16a/Teil 1, S. 1-419, Dieter Klamann, GeorgThieme Verlag Stuttgart, New York 1990) möglich.

Die so erhaltenen N-tert.-Butylpyridylsulfonamide können in die Verbindungen der allgemeinen Formel (II) durch Umsetzung mit einer starken Säure (z.B. Trifluoressigsäure) überführt werden.

Die einzelnen Reaktionsstufen können analog üblichen Verfahren durchgeführt werden. Die Sulfonamide der Formel (VIII) sind nach literaturbekannten Verfahren aus gegebenenfalls substituierten 2-Chlor-3-nitropyridinen herstellbar. Dabei erfolgt zunächst die Umsetzung mit Schwefel-Nucleophilen, wie z. B. Benzylmercaptan, und anschließender oxidativer Chlorierung des Schwefelatoms mit Natriumhypochlorit oder Chlor (Bildung der Sulfochloride analog EP-A-272 855) sowie Umsetzung der erhaltenen Sulfochloride mit tert. Butylamin zu 3-Nitro-2-N-tert.-butylsulfonylamidopyridinen. Die Nitrogruppe kann anschließend zum Hydroxylamin (II) (R¹ =R² =H) reduziert werden und dann in geeigneter Form, z.B. durch N- oder O- Acylierung, zu anderen Verbindungen der Formel (II) derivatisiert werden.

Alternativ können Verbindungen der Formel (II) aus 2-Chlor-3-nitro-pyridinen durch Reduktion zum Hydroxylamin und anschließender Derivatisierung hergestellt werden, wobei die Derivatisierung z.B. eine N- oder O- Acylierung bzw. N- oder O- Alkylierung, dann Bildung der Sulfochloride wie vorstehend beschrieben und anschließende Umsetzung der Sulfochloride direkt mit Ammoniak nach oder analog üblichen Methoden beinhaltet.

Die Carbamate der Formel (III) können nach Methoden hergestellt werden, die in den südafrikanischen Patentanmeldungen 82/5671 und 82/5045 bzw. EP-A 70804 (US-A-4 480 101) oder Research Disclosure RD 275056 beschrieben sind.

Die Umsetzung der Verbindungen (IV) mit den Aminoheterocyclen (V) führt man vorzugsweise in inerten, aprotischen Lösungsmitteln wie z. B. Dioxan, Acetonitril oder Tetrahydrofuran bei Temperaturen zwischen 0° C und der Siedetemperatur des Lösungsmittels durch. Die Pyridylsulfonylcarbamate der Formel (IV) erhält man analog EP-A-44 808 oder EP-A-237 292.

Die Pyridylsulfonylisocyanate der Formel (Vl) lassen sich analog EP-A-184 385 herstellen und mit den Aminoheterocyclen der Formel (V) umsetzen.

Die (Thio-)lsocyanate der Formel (VII) sind nach literaturbekannten Verfahren erhältlich (EP-A-232067, EP-A-166516). Die Umsetzung der (Thio)-lsocyanate (VII) mit Verbindungen (II) erfolgt bei -10°C und 100°C, vorzugsweise 20 bis 100°C, in einem inerten aprotischen Lösungsmittel, wie z.B. Aceton oder Acetonitril, in Gegenwart einer geeigneten Base, z.B. N(C₂H₅)₃ oder K₂CO₃.

Die Salze der Verbindungen der Formel (I) werden vorzugsweise in inerten Lösungsmitteln wie z. B. Wasser, Methanol oder Aceton bei Temperaturen von 0 bis 100° C hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin. Als Säuren zur Salzbildung eignen sich besonders HCI, HBr, H₂SO₄ oder HNO₃.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) oder deren Salze weisen eine ausgezeichnete herbizide Wirksamkeit gegen ein breites Spektrum wirtschaftlicher wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielsweise einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Verbindungen der Formel (I) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchstauchung, eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen der Formel (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) oder deren Salze enthalten.

Die Verbindungen der Formel (I) oder deren Salze können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell,, Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläse mühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdisbergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen enthalten in der Regel 0,1 bis 99 Gew.-%, insbesondere 0,1 bis 95 Gew.-%, Wirkstoff der Formel (I) oder deren Salze.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff. versprühbare Lösungen etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 9th edition, The British Crop Protection Council, 1990/91, Bracknell, England, und dort zitierter Literatur beschrieben sind. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet): acetochlor; acifluorfen; aclonifen; AKH 7088, d. h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d. h. Ammoniumsulfamat; anilofos; asulam; atrazin; aziprotryn; barban; BAS 516 H, d. h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; carbetamide; CDAA, d. h. 2-Chlor-N,N-di-2-propenylacetamid; CDEC, d. h. Diethyldithiocarbaminsäure-2-chlorallylester; CGA 184927, d. h. 2-[4-[(5-Chlor-3-fluor-2-pyridinyl)-oxy]-phenoxy]-propansäure und 2-propynylester; chlomethoxyfen; chloramben; chlorazifop-butyl, pirifenop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clomazone; clomeprop; cloproxydim; clopyralid; cyanazine; cycloate; cycloxydim; cycluron; cyperquat; cyprazine; cyprazole; 2,4-DB; dalapon; desmediphan; desmetryn; di-allate; dicamba; dichlobenil; dichlorprop; diclofop-methyl; diethatyl; difenoxuron; difenzoquat; diflufenican; dimefuron; dimethachlor; dimethametryn; dimethazone, clomazon; dimethipin; dimetrasulfuron, cinosulfuron; dinitramine; dinoseb; dinoterb; diphenamid; dipropetryn; diquat; dithiopyr; diuron; DNOC; eglinazine-ethyl; EL 177, d. h. 5-Cyano-1-(1,1-dimethylethyl)-N-methyl-3H-pyrazole-4-carboxamid; endothal; EPTC; esprocarb; ethalfluralin; ethametsulfuron-methyl; ethidimuron; ethiozin; ethofumesate; F5231, d. h. N-[2-Chlor-4-fluor-5-[4-(3-fluorpropyl)-4,5-dihydro-5-oxo-1H-tetrazol-1-yl]-phenyl]-ethansulfonamid; F6285, d. h. 1-[5-(N-Methylsulfonyl)-amino-2,4-dichlorophenyl]-3-methyl-4-difluoromethyl-1,2,4-triazol-5-on; fenoprop; fenoxan, s. clomazon; fenoxaprop-ethyl; fenuron; flamprop-methyl; flazasulfuron; fluazifop und dessen Esterderivate; fluchloralin; flumetsulam; N-[2,6-Difluorphenyl]-5-methyl-(1,2,4)-triazolo[1,5a]pyrimidin-2-sulfonamid; flumeturon; flumipropyn; fluorodifen; fluoroglycofen-ethyl; fluridone; flurochloridone; fluroxypyr; flurtamone; fomesafen; fosamine; furyloxyfen; glufosinate; glyphosate; halosaten; haloxyfop und dessen Esterderivate; hexazinone; Hw 52, d. h. N-(2,3-Dichlorphenyl)-4-(ethoxymethoxy)-benzamid; imazamethabenz-methyl; imazapyr; imazaquin; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metobromuron; metolachlor; metoxuron; metribuzin; metsulfuron-methyl; MH; molinate; monalide; monocarbamide dihydrogensulfate; monolinuron; monuron; MT 128, d. h. 6-Chlor-N-(3-chlor-2-propenyl)-5-methyl-N-phenyl-3-pyridazinamin; MT 5950, d. h. N-[3-Chlor-4-(1-methylethyl)-phenyl]-2-methylpentanamid; naproanilide; napropamide; naptalam; NC 310, d. h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiazon; oxyfluorfen; paraquat; pebulate; pendimethalin; perfluidone; phenmedipham; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Esterderivate; propazine; propham; propyzamide; prosulfalin; prosulfocarb; prynachlor; pyrazolinate; pyrazon; pyrazosulfuron-ethyl; pyrazoxyfen; pyridate; quinclorac; quinmerac; quinofop und dessen Esterderivate, quizalofop und dessen Esterderivate; quizalofop-ethyl; quizalofop-p-tefuryl; renriduron; dymron; S 275, d. h. 2-[4-Chlor-2-fluor-5-(2-propynyloxy)-phenyl]-4,5,6,7-tetrahydro-2H-indazol; S 482, d. h. 2-[7-Fluor-3,4-dihydro-3-oxo-4-(2-propynyl)-2H-1,4-benzoxazin-6-yl]-4,5,6, 7-tetrahydro- 1 H-isoindol- 1,3(2H)-dion; secbumeton; sethoxydim; siduron; simazine; simetryn; SN 106279, d. h. 2-[[7-[2-Chlor-4-(trifluor-methyl)-phenoxy]-2-naphthalenyl]-oxy]-propansäure und -methylester; sulfometuron-methyl; sulfazuron; flazasulfuron; TCA; tebutam; tebuthiuron; terbacil; terbucarb; terbuchlor; terbumeton; terbuthylazine; terbutryn; TFH 450, d. h. N,N-Diethyl-3-[(2-ethyl-6-methylphenyl)-sulfonyl]-1H-1,2,4-triazol-1-carboxamid; thiazafluron; thifensulfuron-methyl; thiobencarb; tiocarbazil; tralkoxydim; tri-allate; triasulfuron; triazofenamide; tribenuron-methyl; triclopyr; tridiphane; trietazine; trifluralin; trimeturon; vernolate; WL 110547, d. h. 5-Phenoxy-1-[3-(trifluormethyl)-phenyl]-1H-tetrazol.

Zur Anwendung werden die in handelsüblicher Form vorliegenden Formulierungen gegebenenalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Die erfindungsgemäßen Verbindungen können beispielsweise direkt auf die Schadpflanzen oder Schadpflanzen und Nutzpflanzen zugleich im Nachauflaufverfahren oder auf die Fläche, auf der die Pflanzen wachsen, z.B. auf Ackerboden mit Pflanzensamen bzw. aufgelaufenen Pflanzen oder auf Anbauflächen, wie z.B. eine Reisanbaufläche, im Vor- oder Nachauflaufverfahren appliziert werden.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### Beispiele

### a) 2-Benzylthio-3-nitropyridin

39,1 g (0,315 mol) Benzylmercaptan werden in 200 ml Acetonitril vorgelegt und mit 47,8 g (0,346 mol) K₂CO₃ 40 min bei 60° C gerührt. Anschließend tropft man 50,0 g (0,315 mol) 2-Chlor-3-nitropyridin in 150 ml Acetonitril gelöst hinzu und erhitzt 4 h zum Rückfluß. Das Acetonitril wird unter reduziertem Druck abdestilliert, der Rückstand in Dichlormethan aufgenommen, die organische Phase jeweils einmal mit gesättigter Natriumhydrogencarbonatlösung und mit 1 n Salzsäure gewaschen, die organische Phase über Magnesiumsulfat getrocknet, vom Trockenmittel abfiltriert und das Dichlormethan unter reduziertem Druck entfernt. Nach Umkristallisieren aus Methanol erhält man 60,9 g (79 % d. Th.) 2-Benzyl-3-nitropyridin vom Schmelzpunkt 72° C.

### b) 3-Nitro-2-pyridin-tert.-butylsulfonamid

24,6 g (0,1 mol) 2-Benzylthio-3-nitropyridin werden in 360 ml CH₂Cl₂ gelöst. 280 ml Wasser hinzugefügt und bei 0° C 44,9 ml konz. HCI hinzugetropft. Anschließend läßt man 550 ml 5 %ige Natriumhypochloritlösung so langsam zulaufen, daß die Innentemperatur 5° C nicht überschreitet. Nach 30 min weiteren Rührens bei 0° C wird der Ansatz auf 500 ml Wasser gegeben, die organische Phase abgetrennt und die wäßrige Phase bei 0° C mit CH₂Cl₂ nachgewaschen. Die vereinigten organischen Phasen werden mit NaCl-Lösung gewaschen und über MgSO₄ getrocknet. Man tropft dann bei -70° C 32,1 g (0,44 mol) t-Butylamin hinzu. Man läßt den Ansatz auf Raumtemperatur aufwärmen, gießt in Wasser und stellt mit 1 n HCI auf pH 2-3. Nach Abtrennen der organischen Phase wird die wäßrige Phase mit CH₂Cl₂ nachgewaschen, die organischen Phasen vereinigt, getrocknet und das Lösungsmittel unter reduziertem Druck abgezogen. Nach Ausrühren des Rückstandes mit Diethylether und Abfiltrieren erhält man 14,7 g (57 % d. Th.) 3-Nitro-2-pyridin-tert.-butylsulfonamid vom Schmelzpunkt 134° C.

### c) 3-N-Hydroxylamino-2-pyridin-tert.-butylsulfonamid

Zur homogenen Lösung von 38,40 g (148,3 mmol) 3-Nitro-2-pyridin-tert.-butylsulfonamid und 21,89 g (409,2 mmol) Ammoniumchlorid in 600 ml Ethanol und 150 ml Wasser gab man unter Eiskühlung portionsweise 45,70 g Zinkstaub. Nach Ende der Zugabe wurde 30 min nachgerührt, anschließend von überschüssigem Zink abfiltriert und das Filtrat im Vakuum bis zur Trockene eingeengt. Anorganische Bestandteile der Rückstände konnten abgetrennt werden, indem man den Rückstand in wenig Essigester suspendierte und über eine Kieselgelsäule (h = 5 cm, Ø = 5 cm) in Essigester abfiltrierte. Das Filtrat wurde eingeengt und aus Essigester, Petrolether (1:3) kristallisiert. Man erhielt 32,9 g 3-N-Hydroxylamino-2-pyridin-tert.butylsulfonamid (Ausbeute 90 %) mit einem Schmelzpunkt von 129°C.

### d) 3-N-(O-Acetyl)-hydroxylamino-2-pyridin-tert.-butylsulfonamid

In 10 ml wasserfreiem Tetrahydrofuran wurden 0,88 g (4,10 mmol) 3-N-Hydroxylamino-2-pyridin-tert.-butylsulfonamid zusammen mit 0,50 (4,9 mmol) Triethylamin gelöst und diese Lösung mit 0,32 g (4,1 mmol) Acetylchlorid versetzt. Nach 30 min setzte man 100 ml Essigester zu, extrahierte zweimal mit 2n HCI, trocknete die organische Phase mit Na₂SO₄, filtrierte vom Lösungsmittel ab und engte das Filtrat am Rotationsverdampfer ein. Das Rohprodukt wurde aus Essigester/n-Heptan kristallisiert. Man erhielt 1,10 g 3-N-(O-Acetyl)-hydroxylamino-2-pyridin-tert.-butylsulfonamid als kristallinen Feststoff (Schmp. 147°C, Ausbeute 93 %).

### e) 3-N-(O-Acetyl)-hydroxylamino-2-pyridinsulfonamid

2,00 (6,97 mmol) 3-N-(O-Acetyl)-hydroxylamino-2-pyridin-tert.-butylsulfonamid wurden in 3,0 ml Trifluoressigsäure 3 h auf 60°C erwärmt. Nach Entfernen des Lösungsmittels im Vakuum konnte der Rückstand aus Essigester/Ether kristallisiert werden. Man erhielt 1,21 g (75 % Ausbeute) 3-N-(O-Acetyl)-hydroxylamino-2-pyridinsulfonamid mit einem Schmelzpunkt von 121°C.

### f) 3-(4,6-Dimethoxypyrimidin-2-yl)-1-[(3-N-(O-acetyl)-hydroxylamino)-2-pyridylsulfonylharnstoff

Zu einer Lösung von 1,20 g (5,10 mmol) 3-N-(O-Acetyl)-hydroxylamino-2-pyridylsulfonamid und 2,00 g (7,3 mmol) 1-Phenyl-3-4,6-Dimethoxypyrimidin-2-yl)-carbamat in 20 ml Acetonitril gab man 844 mg (6,12 mmol) 1,5-Diazabicyclo-[5.4.0]undec-7-en (DBU). Nach 30 min Rühren bei Raumtemperatur wurde mit 2n Salzsäure angesäuert, mehrfach mit Essigsäureethylester extrahiert, über Na₂SO₄ getrocknet, filtriert und am Rotationsverdampfer eingeengt. Das Produkt kristallisierte aus Essigester/Ethanol 1:10. Man erhielt 0,93 g (44 % Ausbeute) mit einem Schmelzpunkt von 122°C.

### g) 3-N-(O-Methyl)-hydroxylamino-2-pyridin-tert.-butylsulfonamid

6,00 g (24,5 mmol) 3-N-Hydroxylamino-2-pyridin-tert.-butylsulfonamid wurden in 200 ml wasserfreiem THF zusammen mit 3,39 g (26,9 mmol) Dimethylsulfat gelöst, auf -20°C gekühlt und anschließend portionsweise mit 5,54 g (49,0 mmol) Kalium-tert.-butylat versetzt. Die Reaktionslösung erwärmte sich innerhalb von 2 h auf Raumtemperatur. Anschließend setzte man 100 ml Wasser zu und extrahierte mehrfach mit Ether. Nach Trocknen der etherischen Phasen über Natriumsulfat erhielt man ein Rohprodukt, das über Kieselgel im Laufmittel Essigester/n-Heptan 1:2 chromatographiert wurde. Man erhielt 2,56 g (40 %) 3-N-(O-Methyl)-hydroxylamino-2-pyridin-tert.-butylsulfonamid als farblose Kristalle mit dem Schmelzpunkt 128°C.

### h) 3-N-(O-Methyl)hydroxylamino-2-pyrid insulfonamid

1,00 g (3,86mmol) 3-N-(OMethyl)-hydroxylamino-2-pyridin-tert.-butylsulfonamid wurden in 15 ml Trifluoressigsäure gelöst und 8 h auf 50°C erwärmt. Anschließend entfernte man das Lösungsmittel im Vakuum und kristallisierte den Rückstand aus Essigester/Cyclohexan. Man erhielt 470 mg (61 %) 3-N-(O-Methyl)hydroxylamino-2-pyridinsulfonamid mit einem Schmelzpunkt von 149°C.

### i) 3-(4,6-Dimethoxypyrimidin-2-yl)1-[3-N-[O-methyl)-hydroxylamino)-2-pyridylsulfonyl]harnstoff

430 mg (2,11 mmol) 3-N-(O-Methyl)-hydroxylamino-2-pyridinsulfonamid wurden zusammen mit 7,57mg (2,75 mmol)1-Phenyl-3(4,6-dimethoxypyrimidin-2-yl)-carbamat in 20 ml Acetonitril gelöst und bei Raumtemperatur mit 0,350 g (2,53 mmol) 1,8-Diazabicyclo[5.4.O]undec-7-en versetzt. Nach 30 min säuerte mit 2n Salzsäure an, extrahierte mit Essigsäureethylester, trocknete über Na₂SO₄, filtrierte und engte am Rotationsverdampfer ein. Das Produkt kristallisierte aus Methylenchlorid/Ether. Man erhielt 450 mg (55 % d.Th.) 3-(4,6-Dimethoxypyrimidin-2-yl)-1-[3-N[O-methyl]hydroxylamino)-2-pyridylsulfonyl]-harnstoff mit einem Schmelzpunkt von 138°C.

Die anderen Verbindungen der nachfolgenden Tabellen 1-5 werden analog zu den Verfahren der Beispiele a bis i erhalten.

Abkürzungen zu Tabellen 1-5:
Schmp. = Smp. = Fp. = Schmelzpunkt in °C
Ph = Phenyl
Bzl = Benzyl
Me = Methyl
Et = Ethyl
Pr = Propyl
Bu = Butyl
n-Alkyl = geradkettiges Alkyl = Alkyl
i-Alkyl = iso-Alkyl (z.B. i-Bu = Isobutyl)
s-Alkyl = sekundäres Alkyl
t-Alkyl = tertiäres Alkyl
c-Alkyl = Cycloalkyl (z.B. c-Pr = Cyclopropyl)

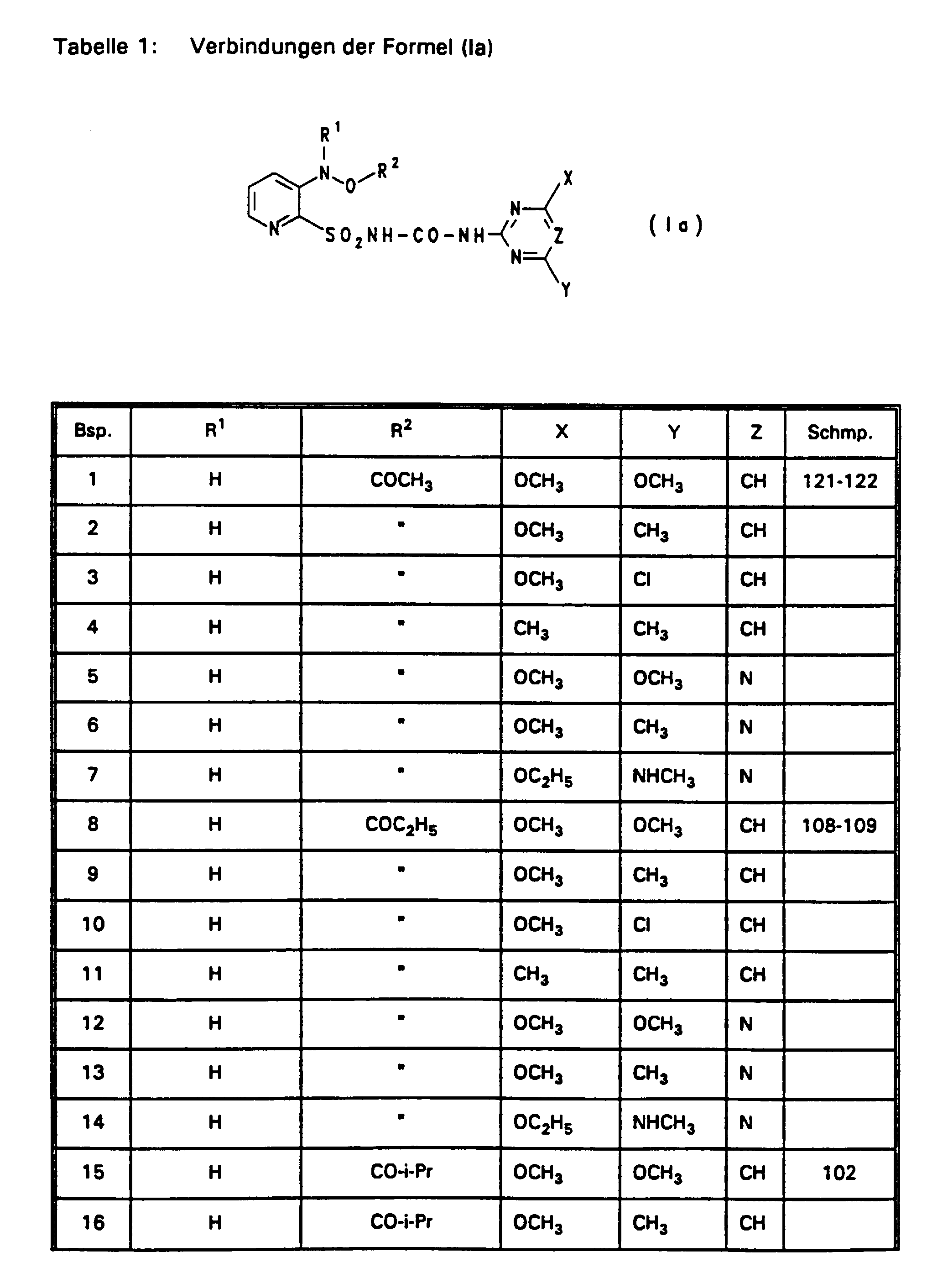

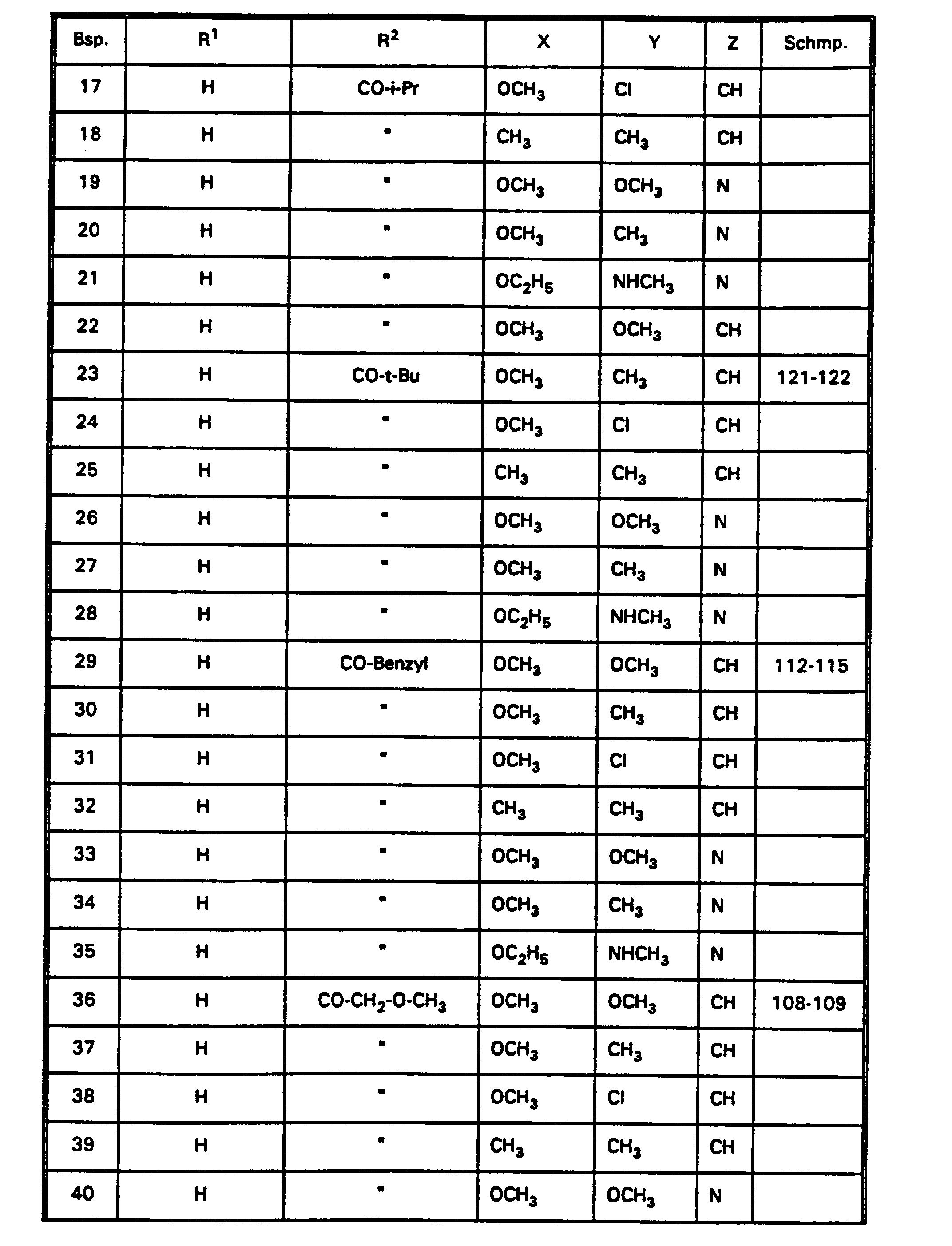

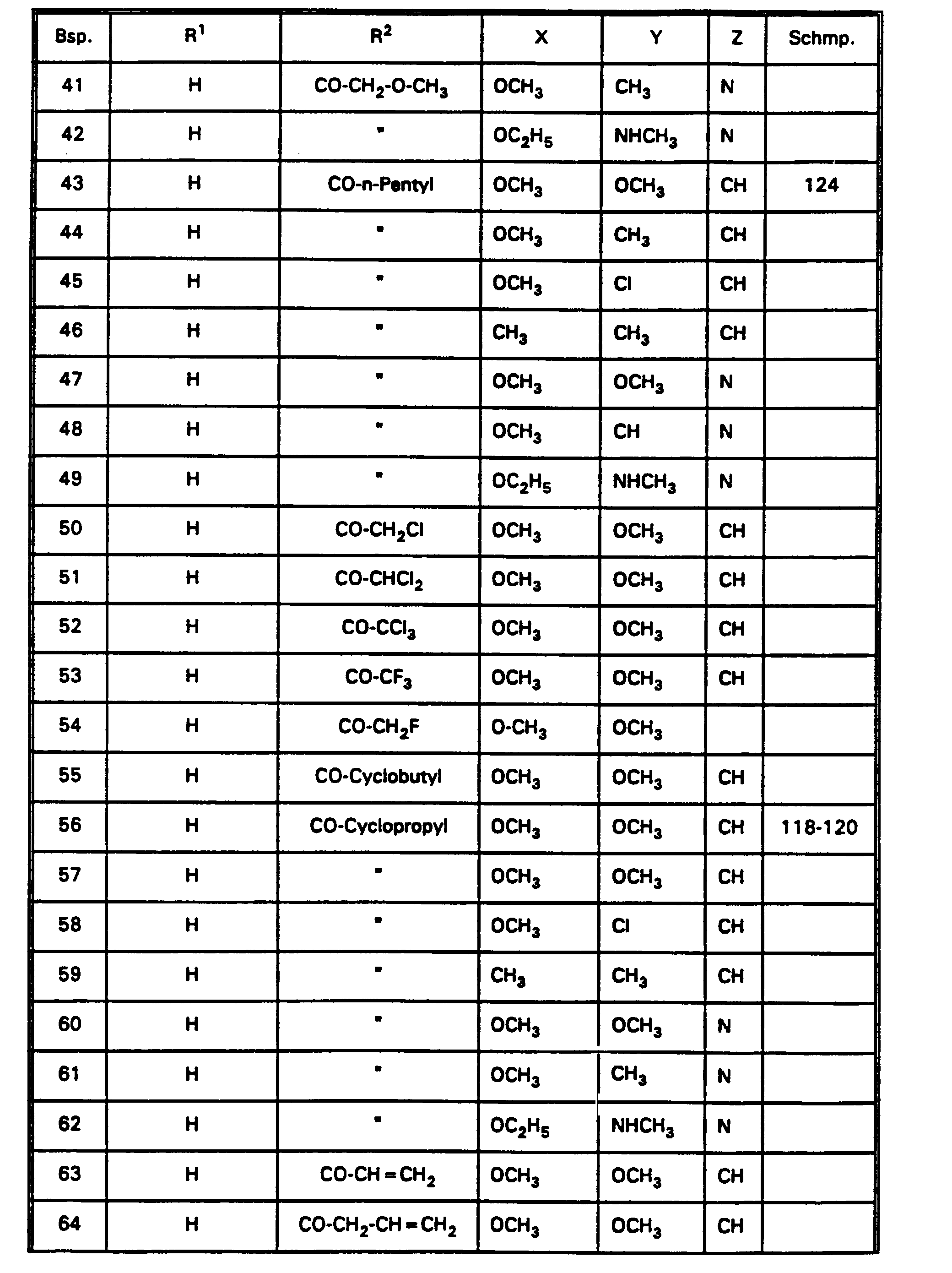

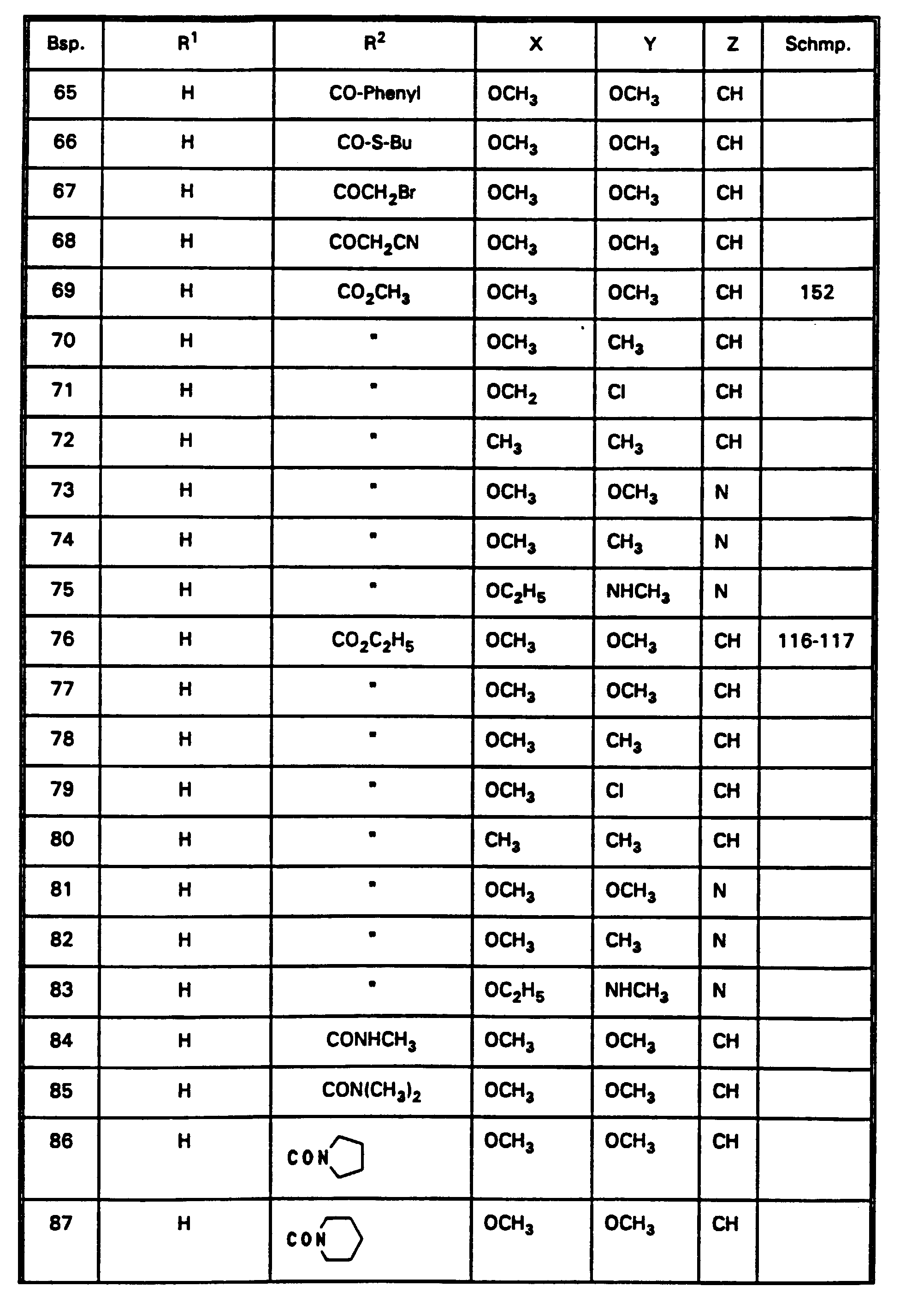

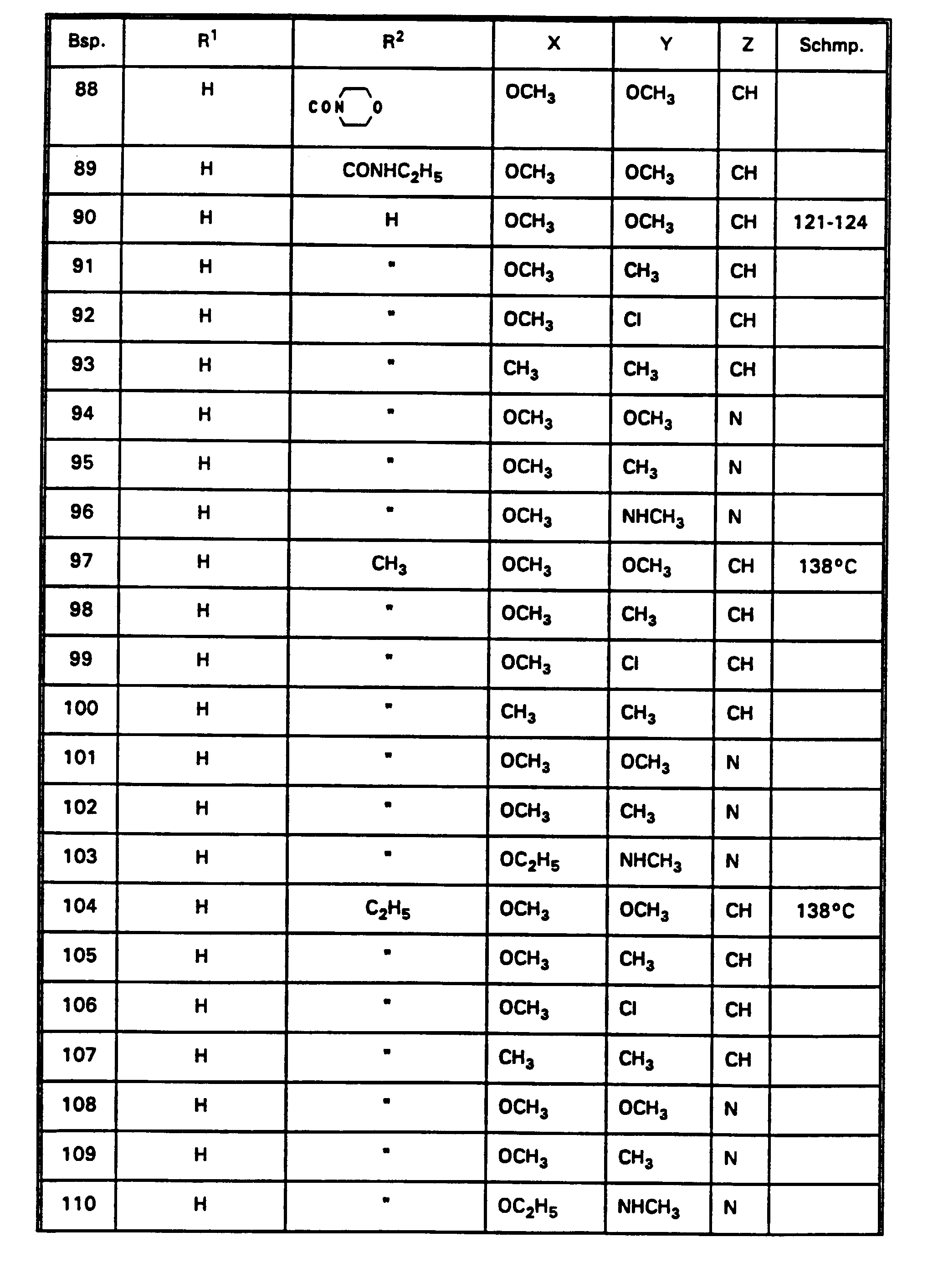

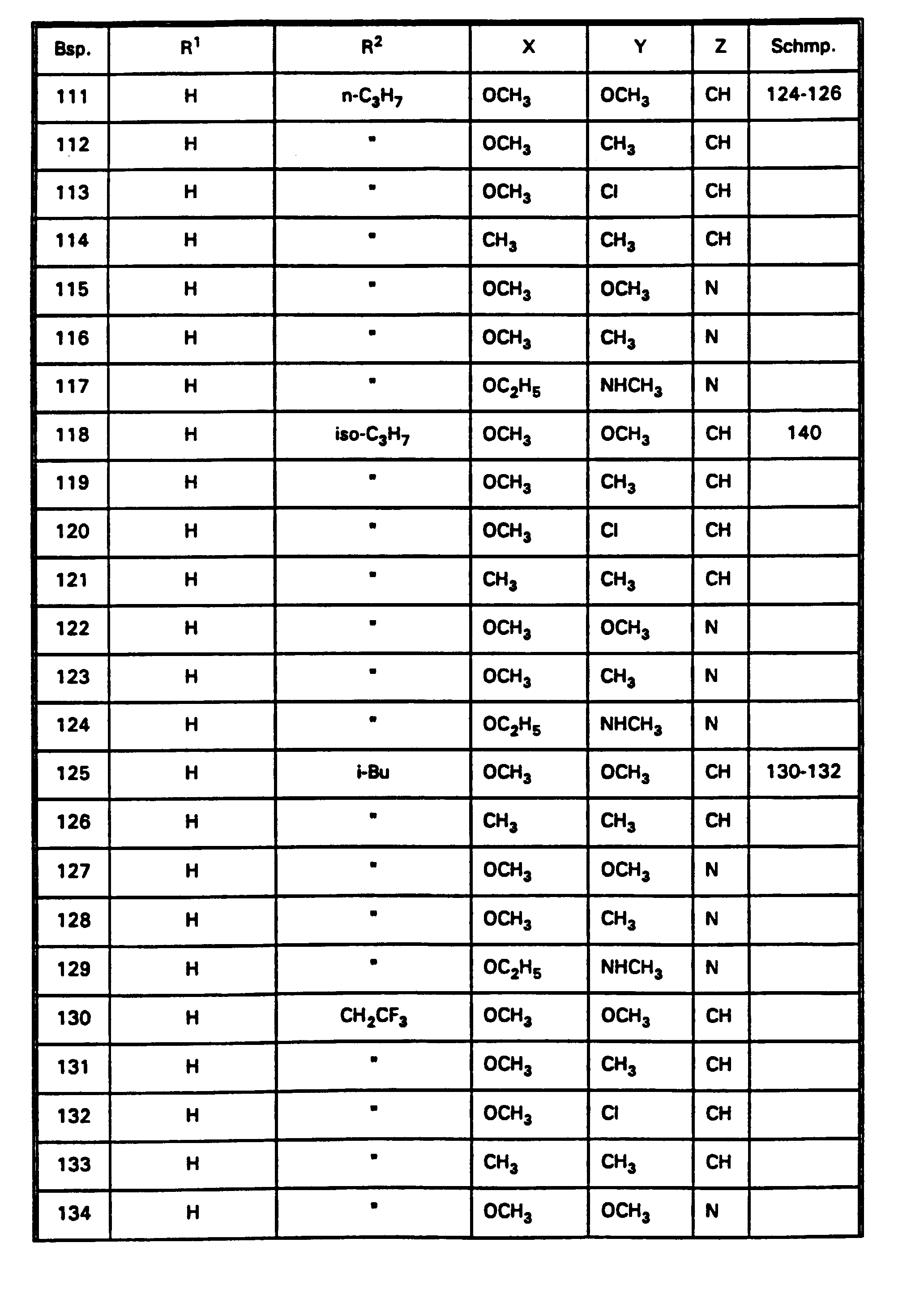

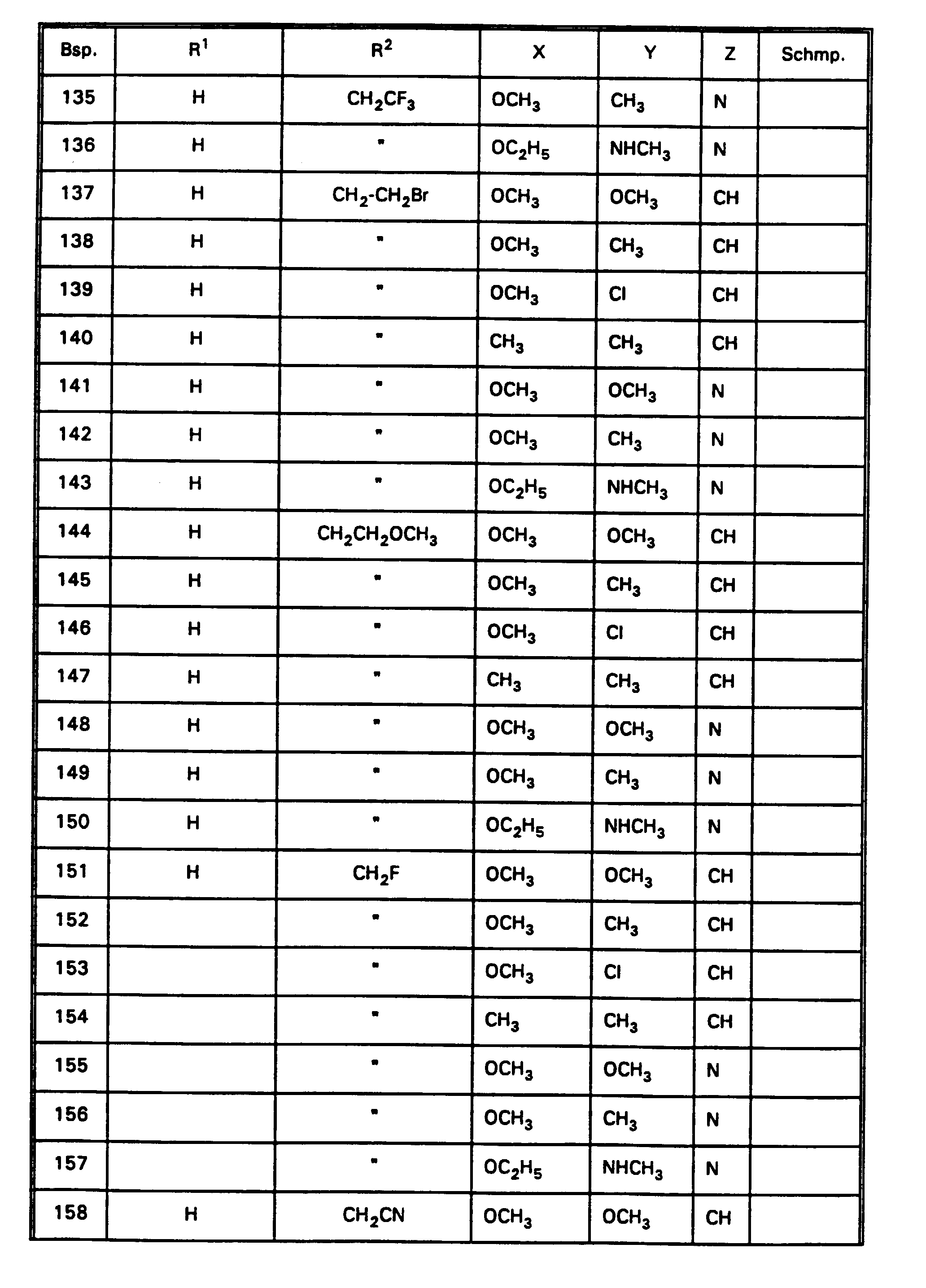

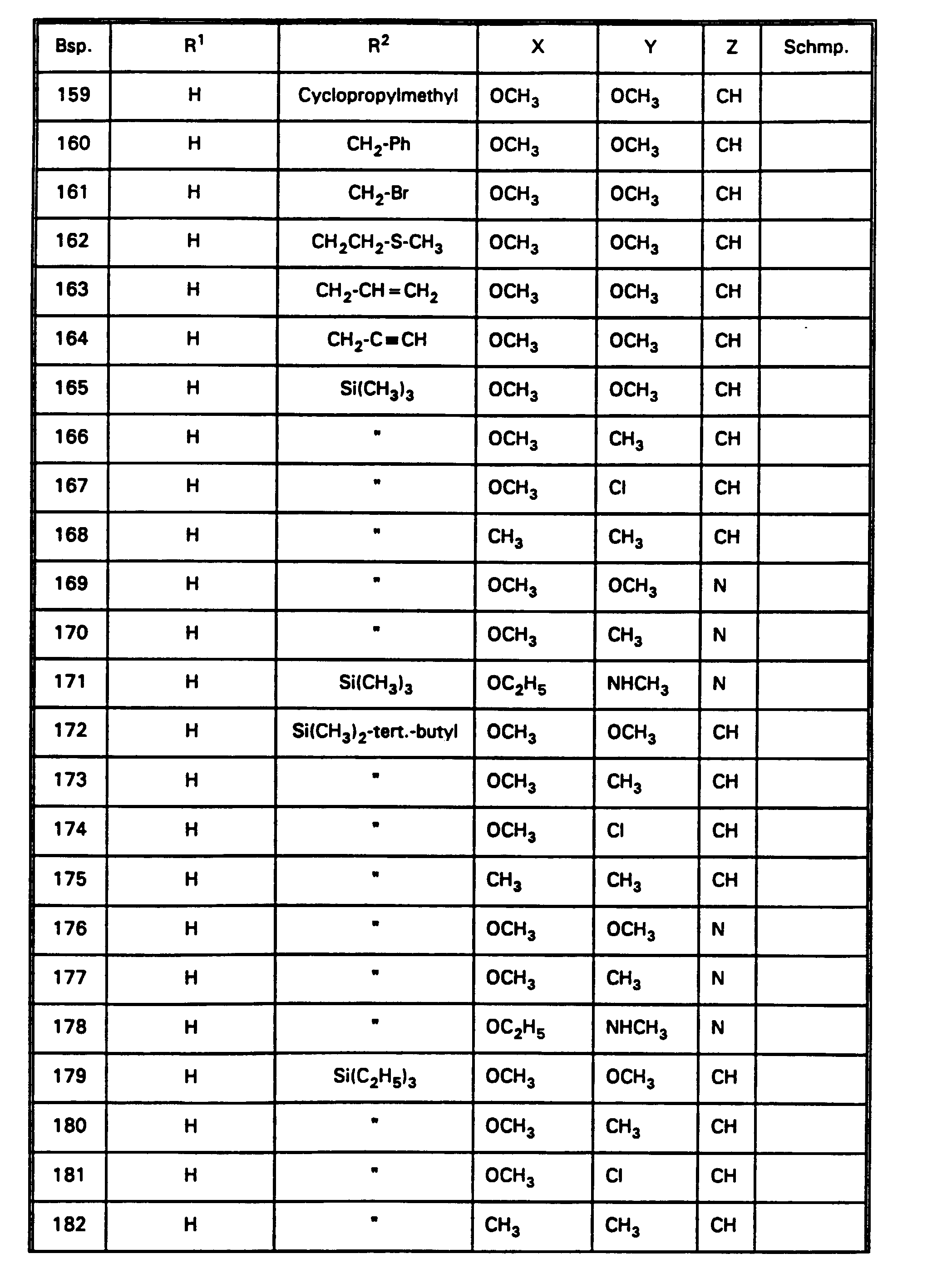

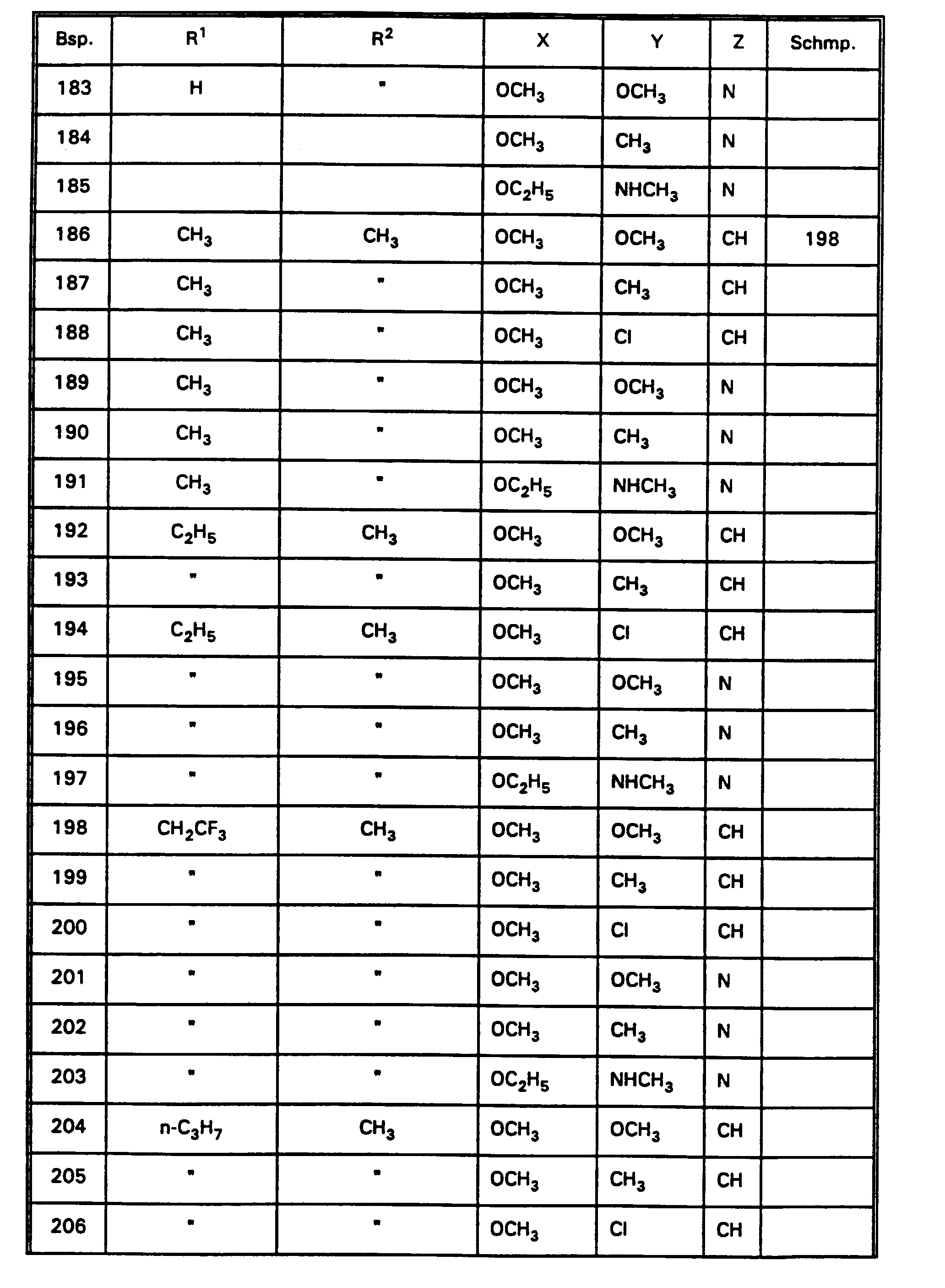

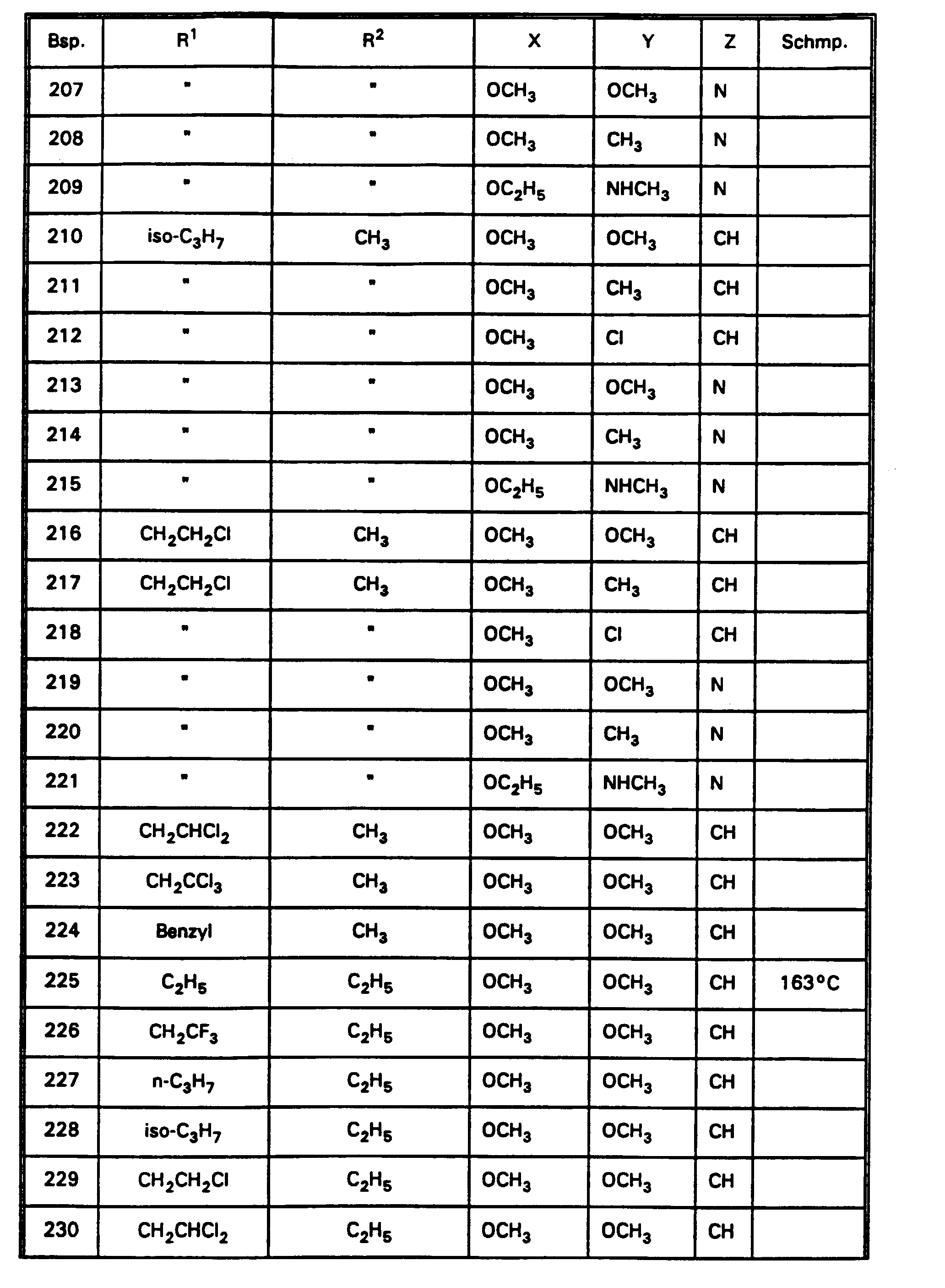

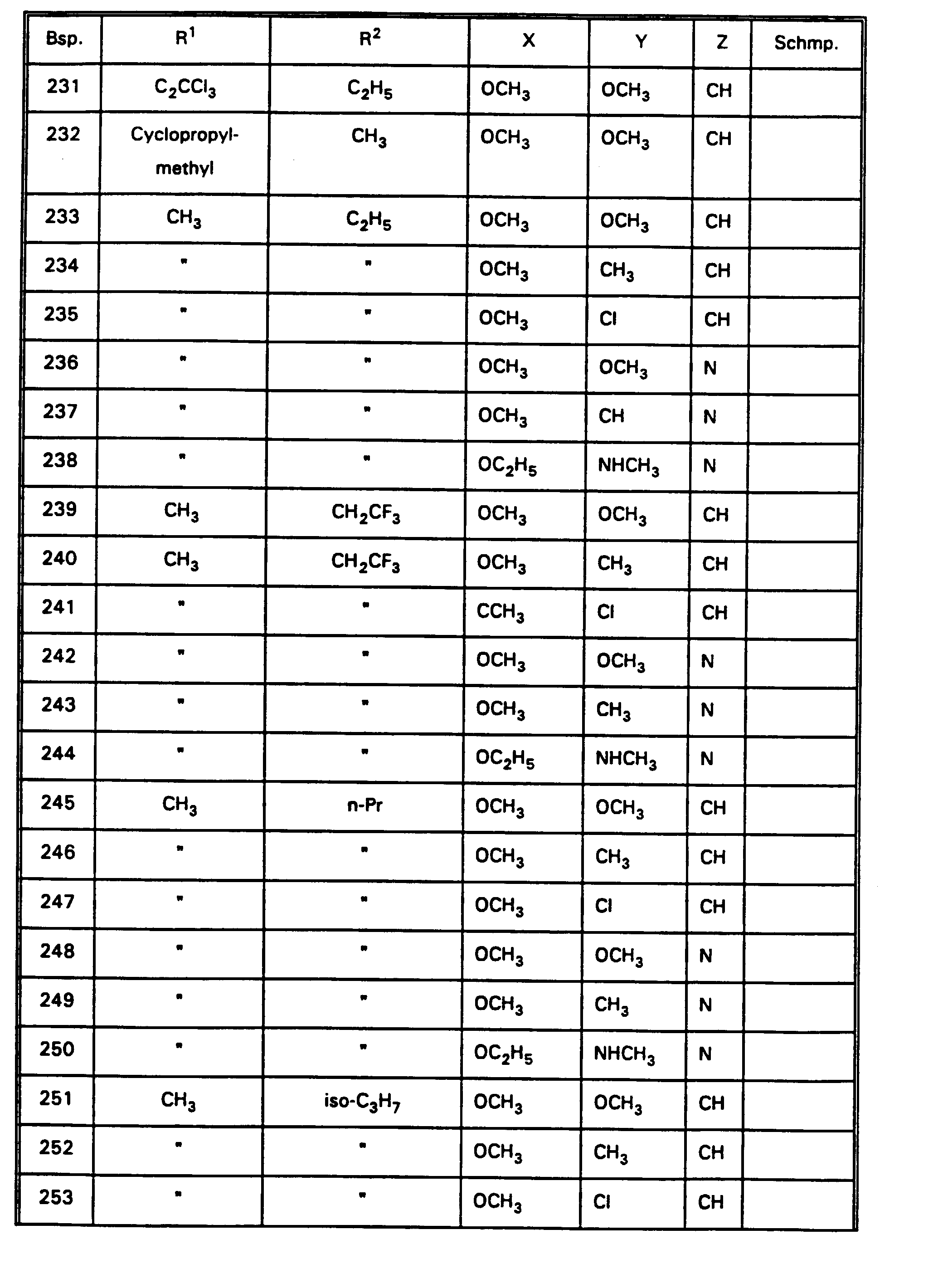

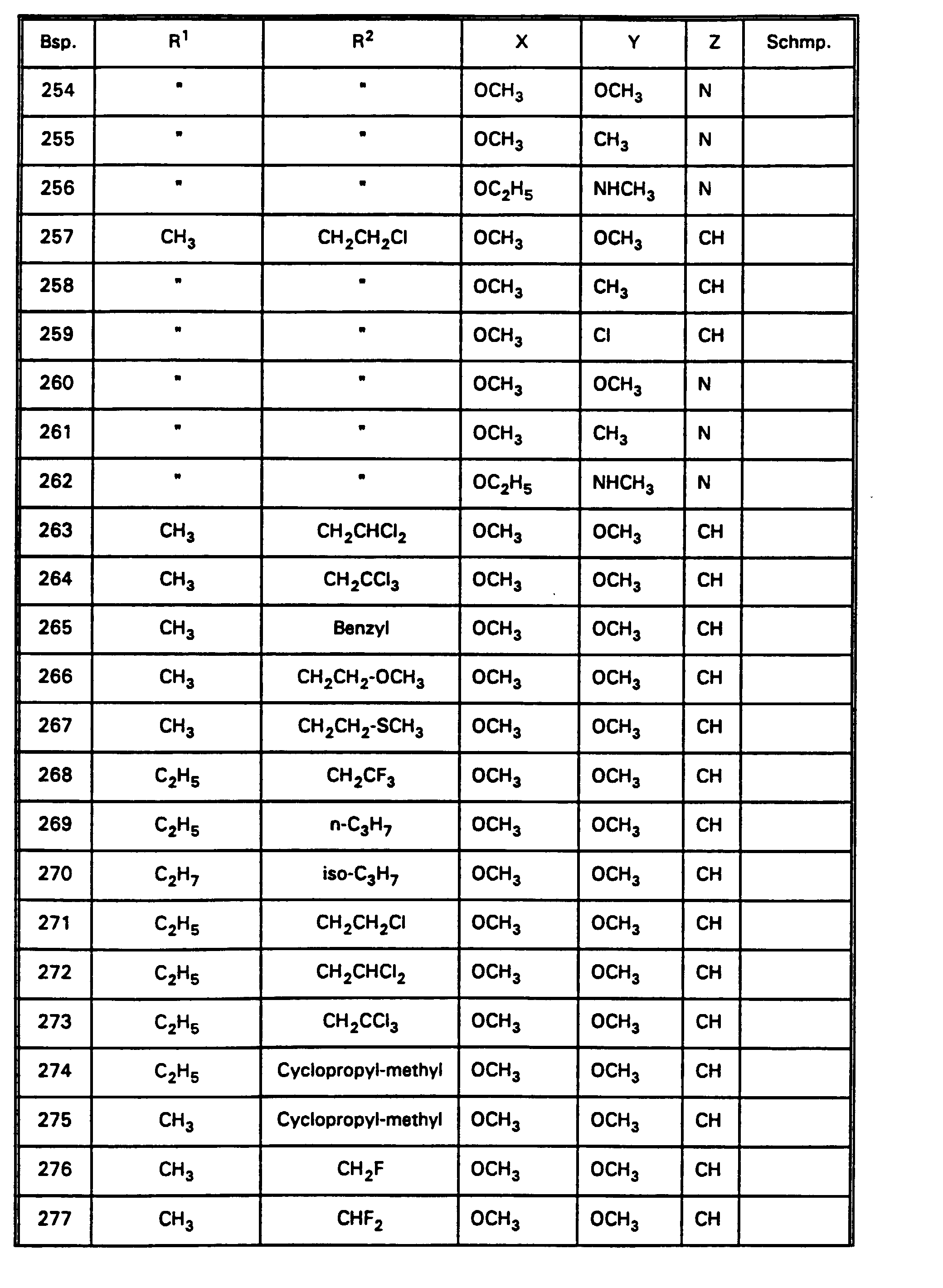

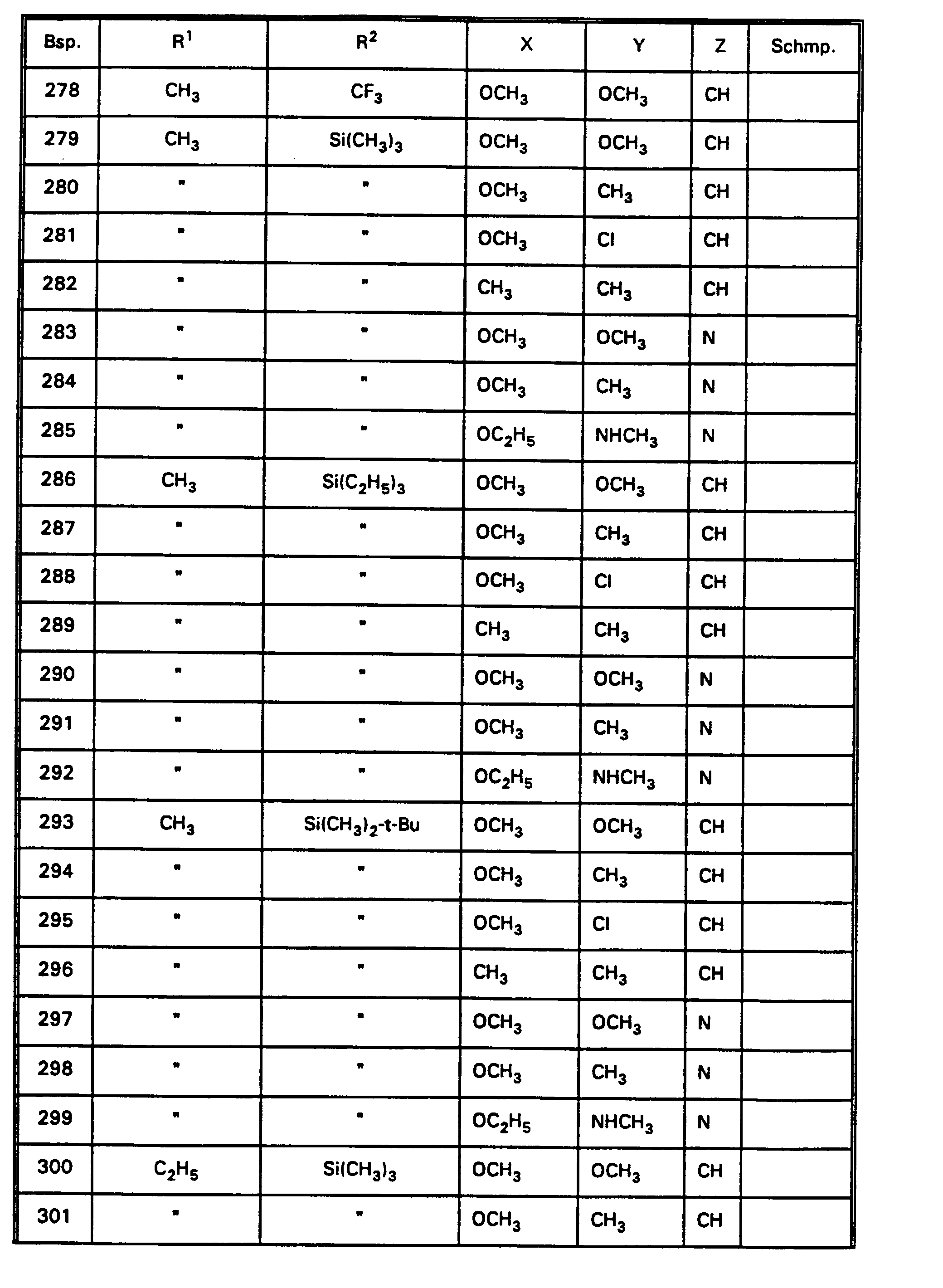

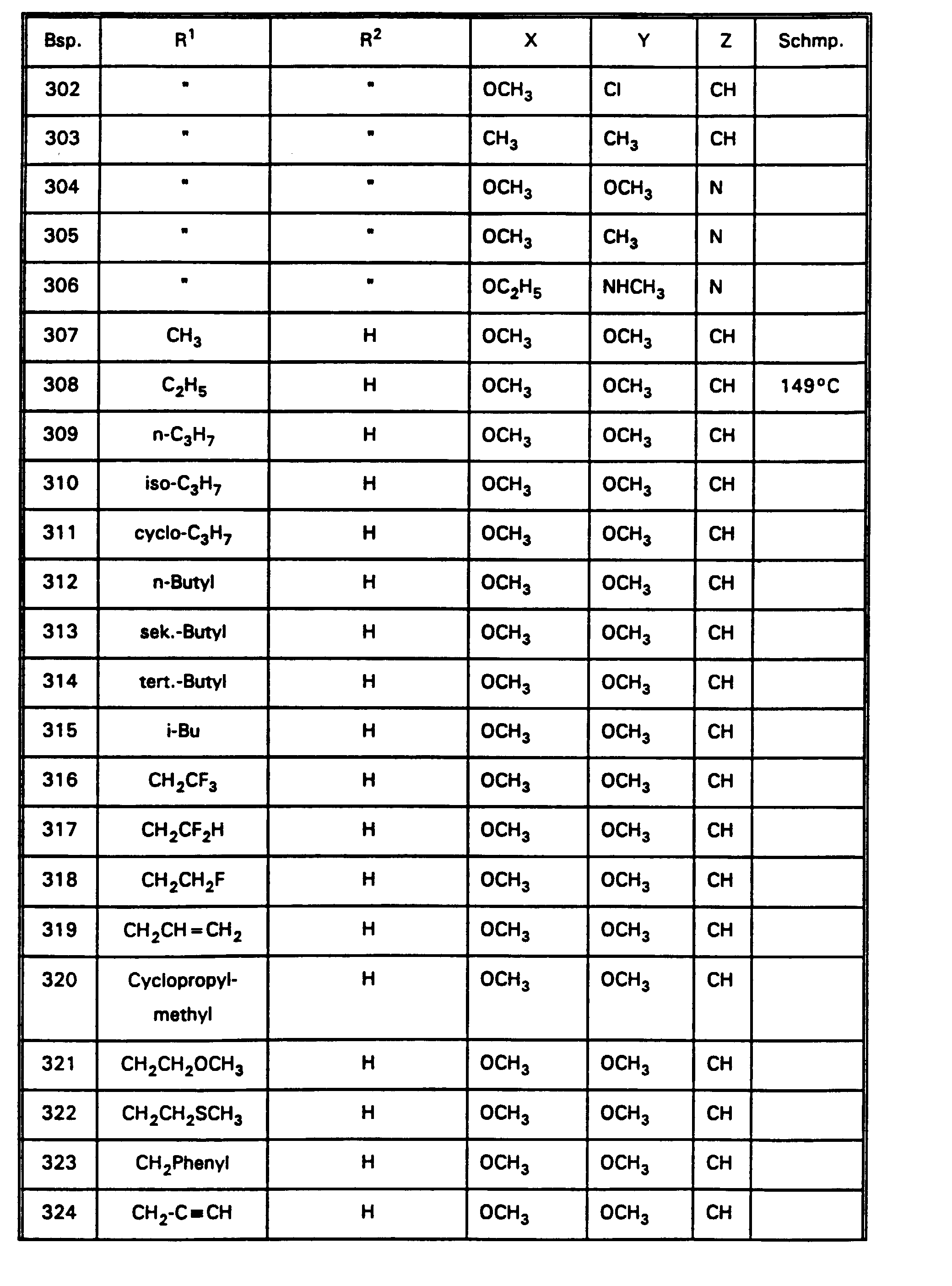

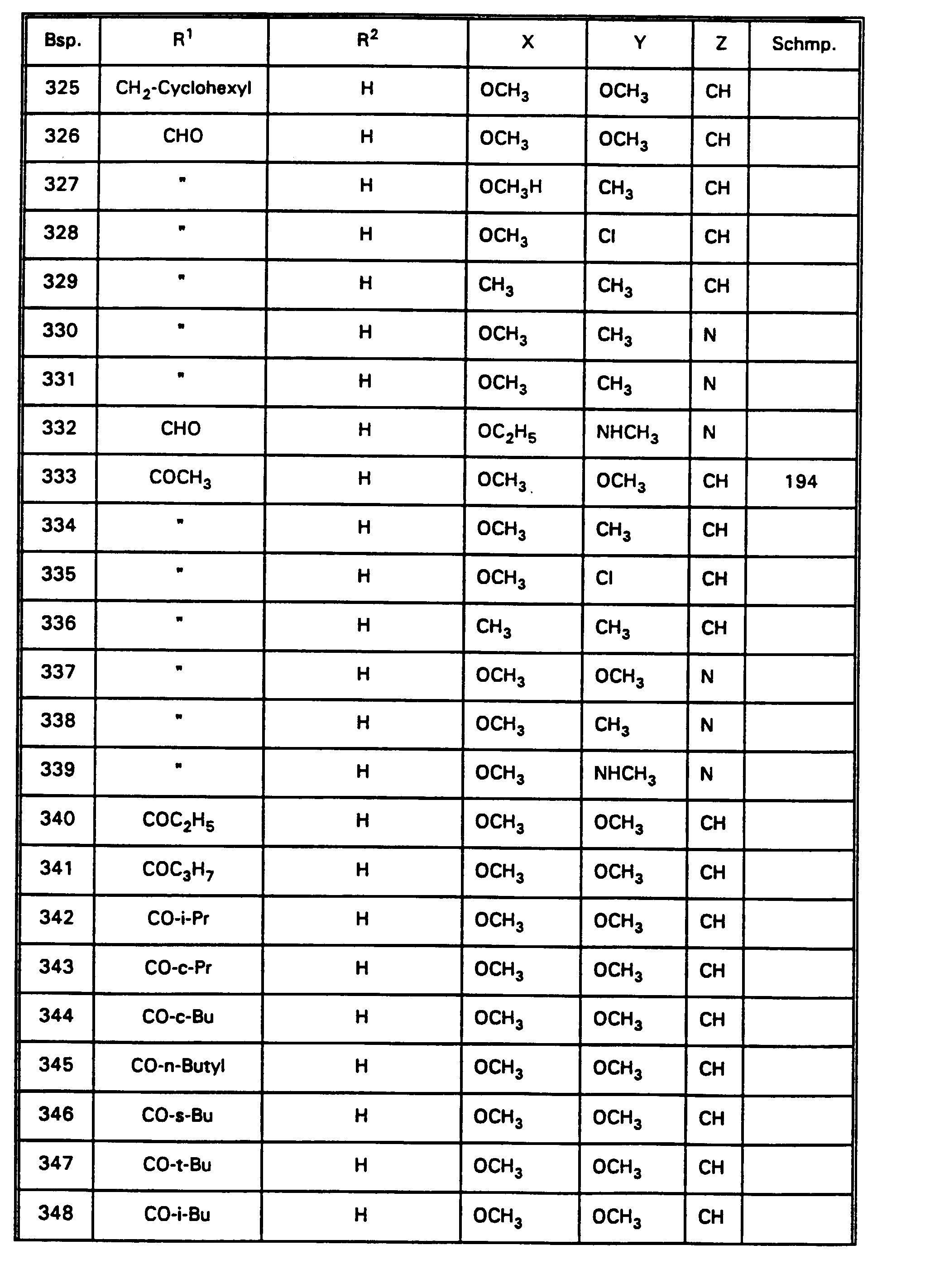

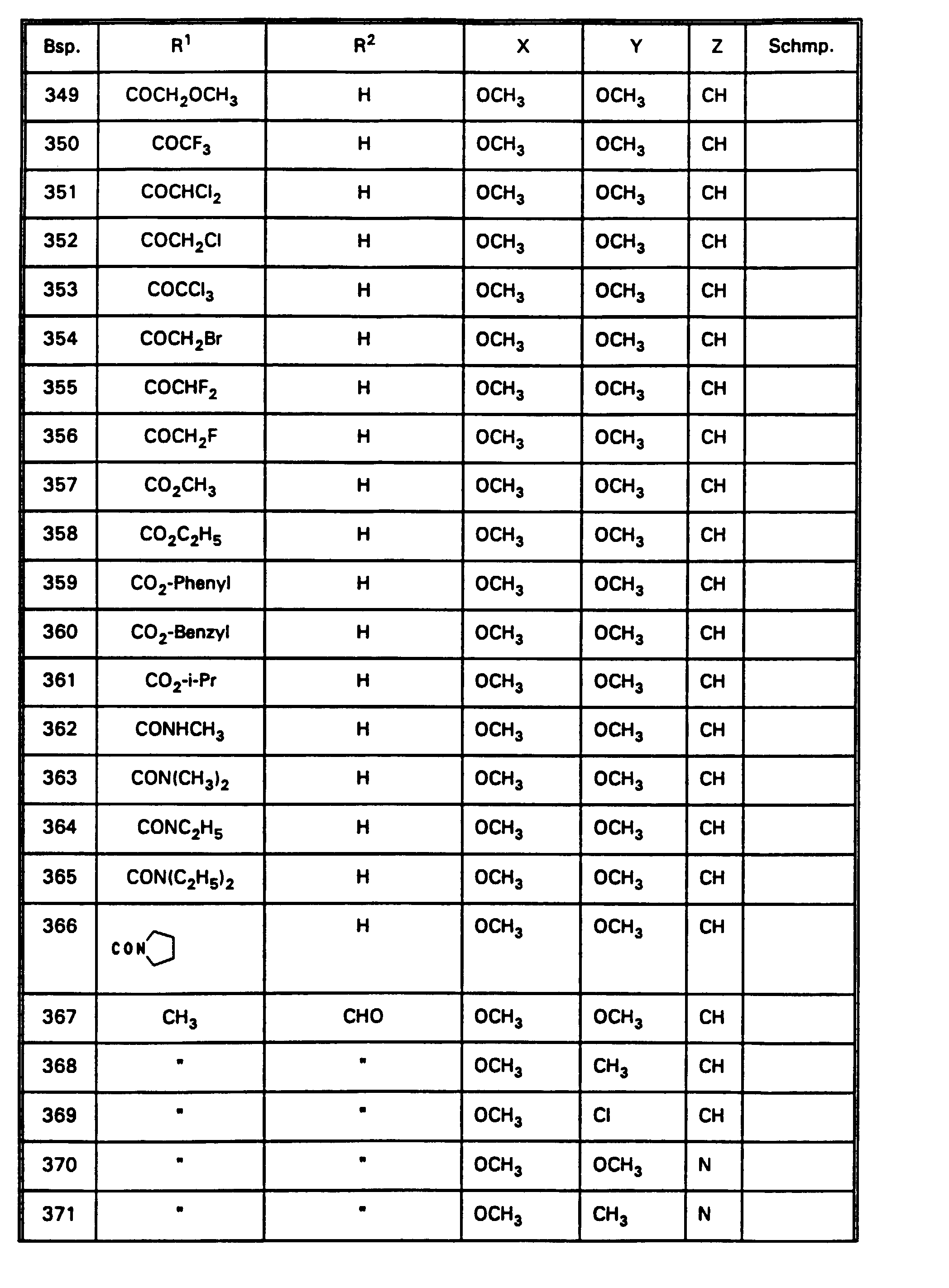

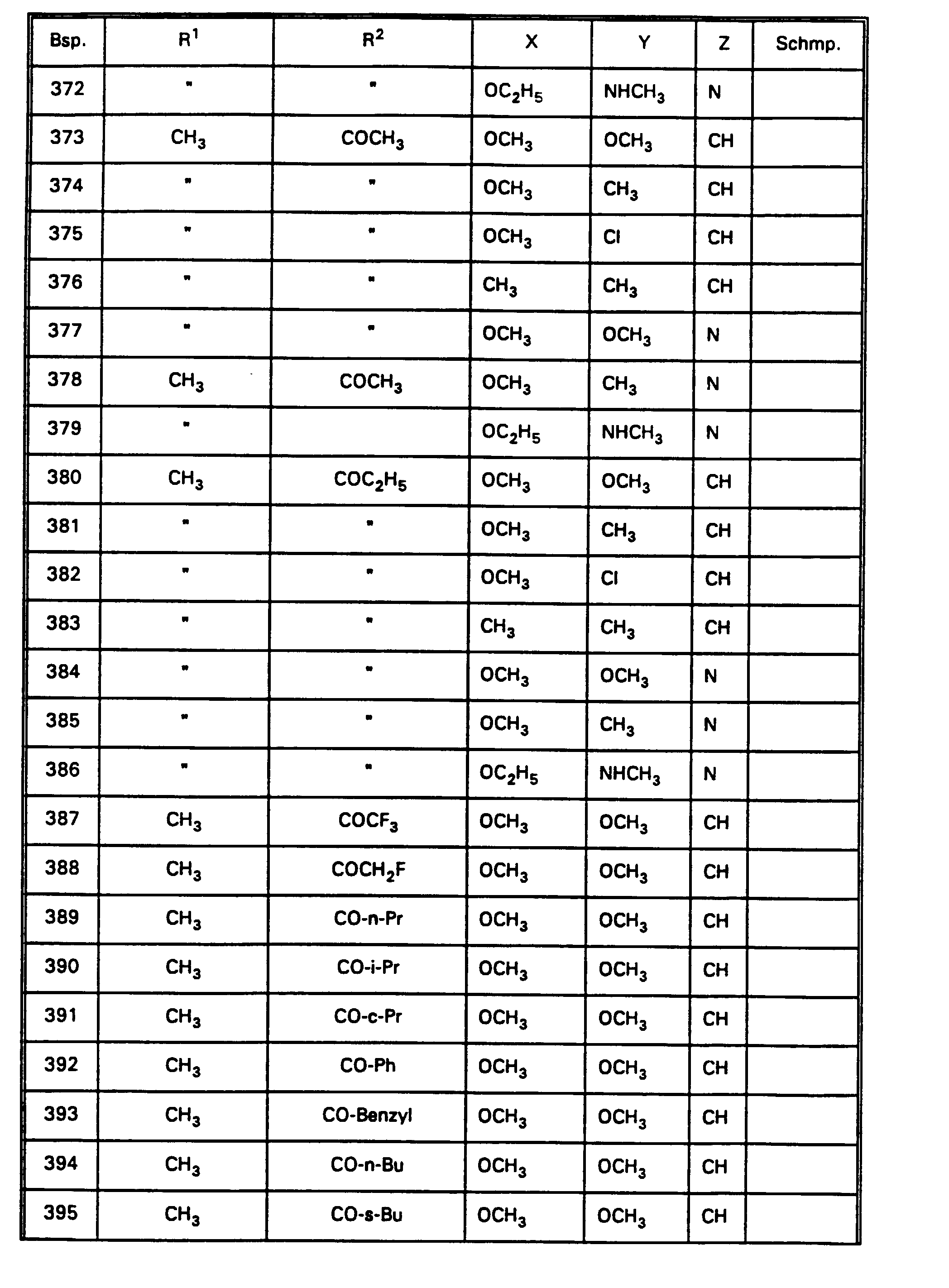

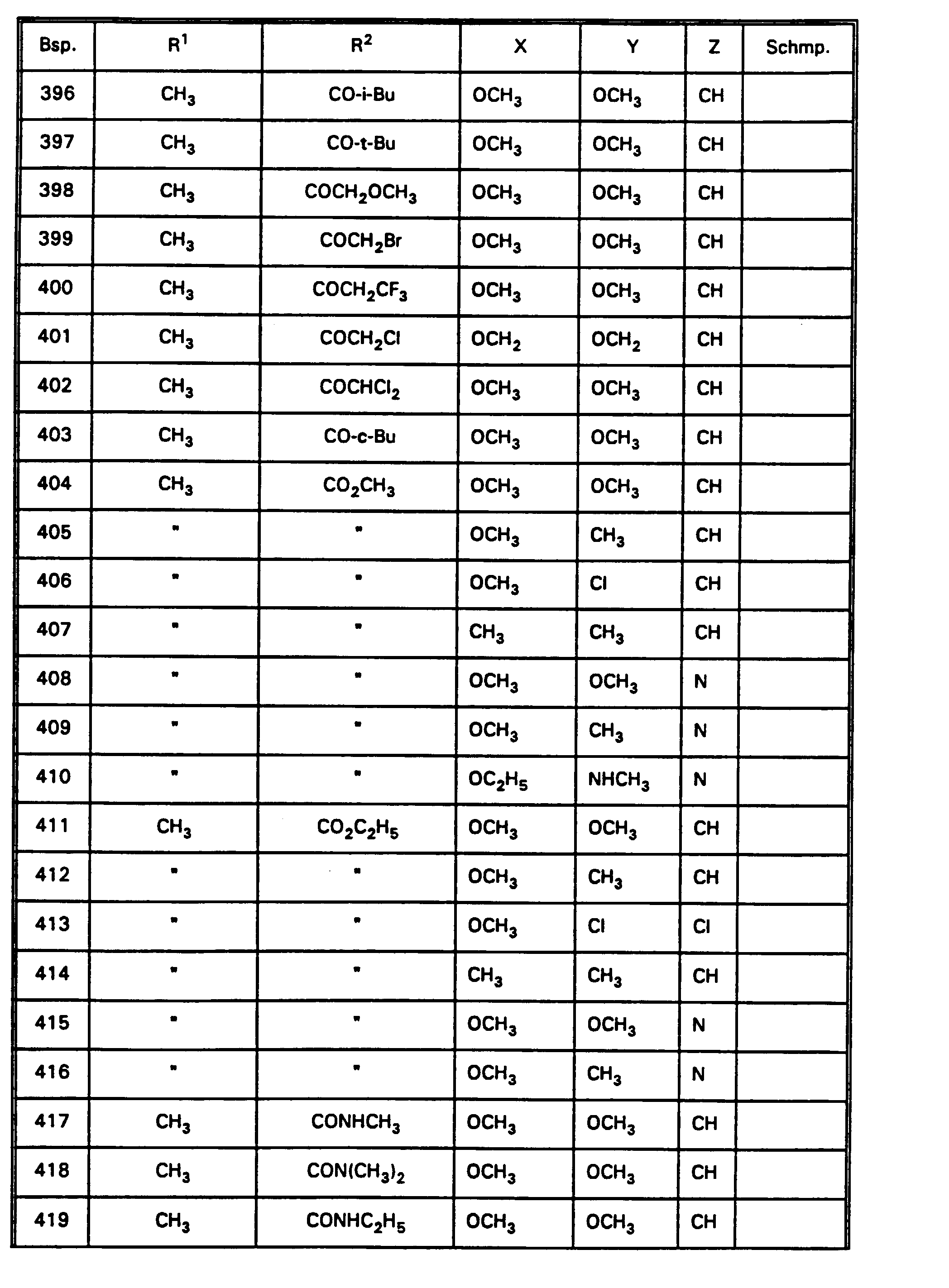

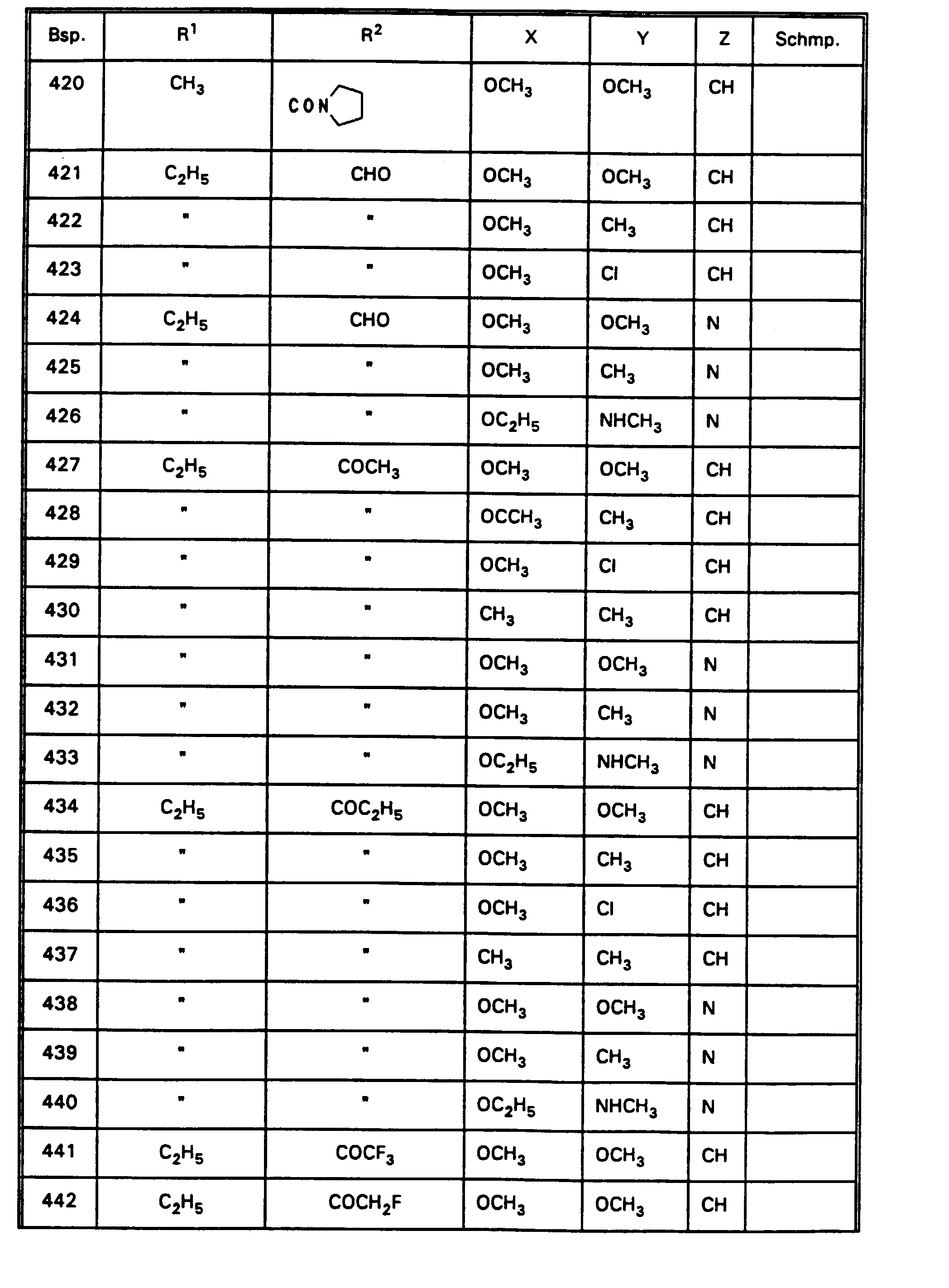

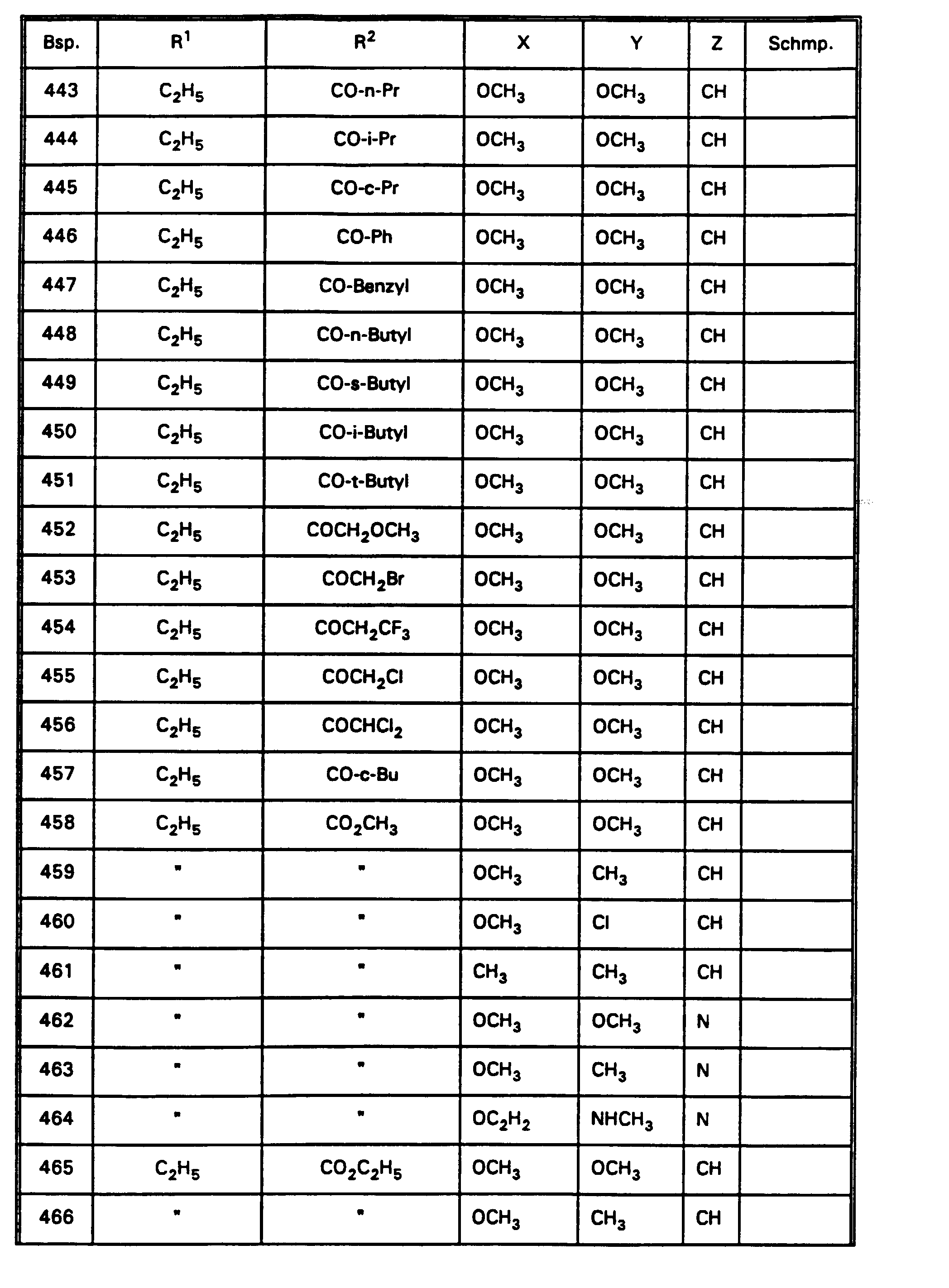

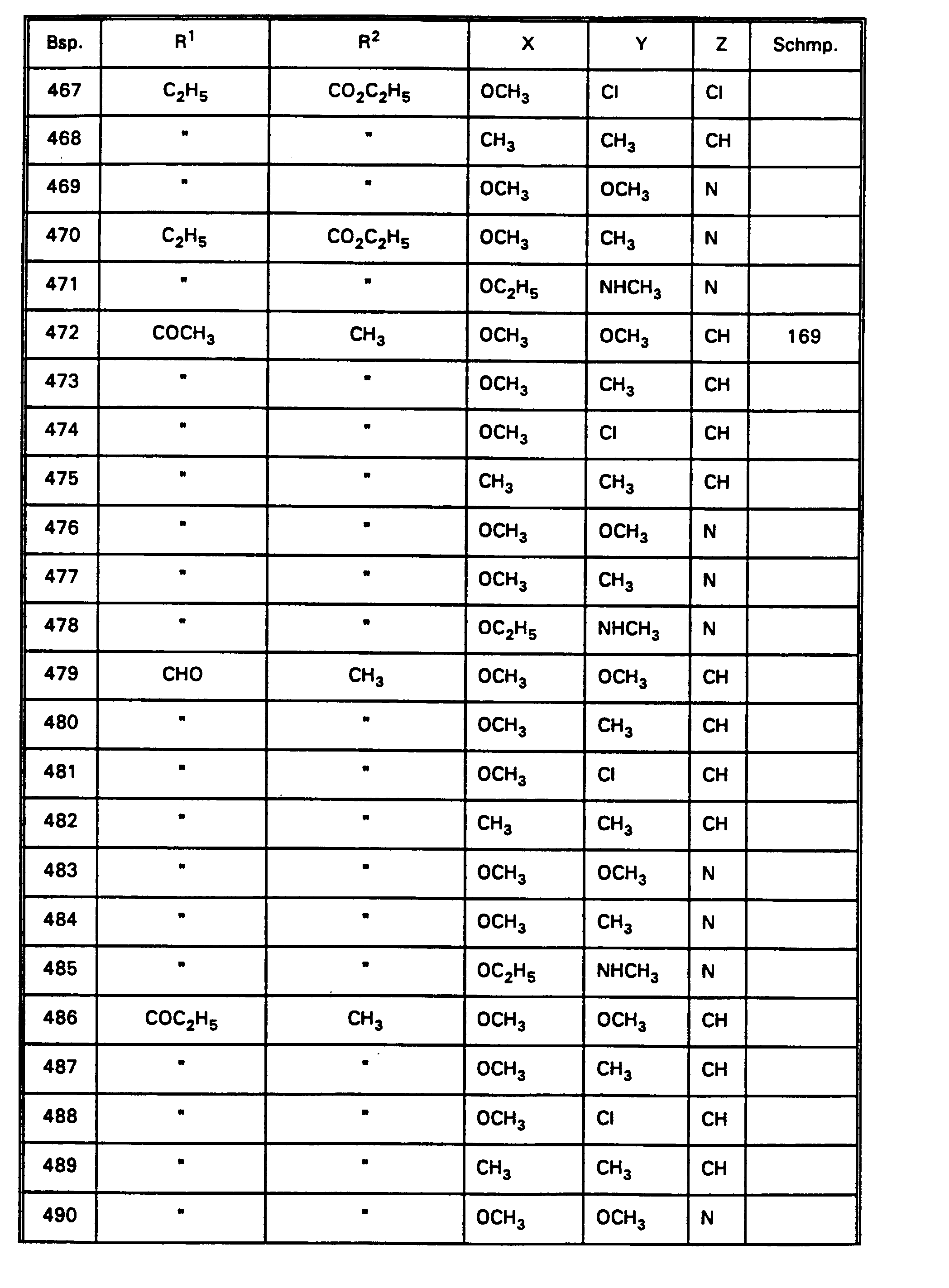

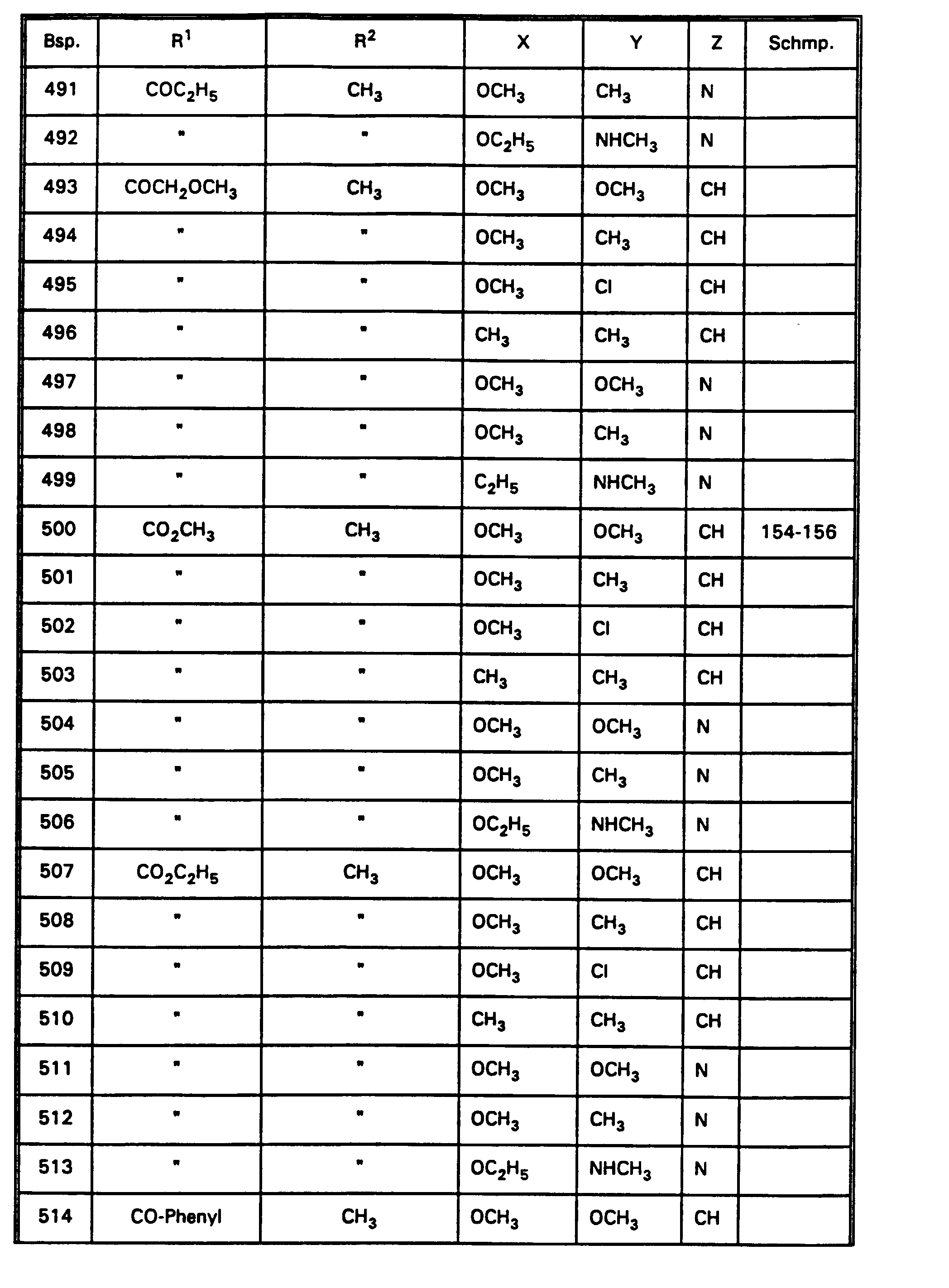

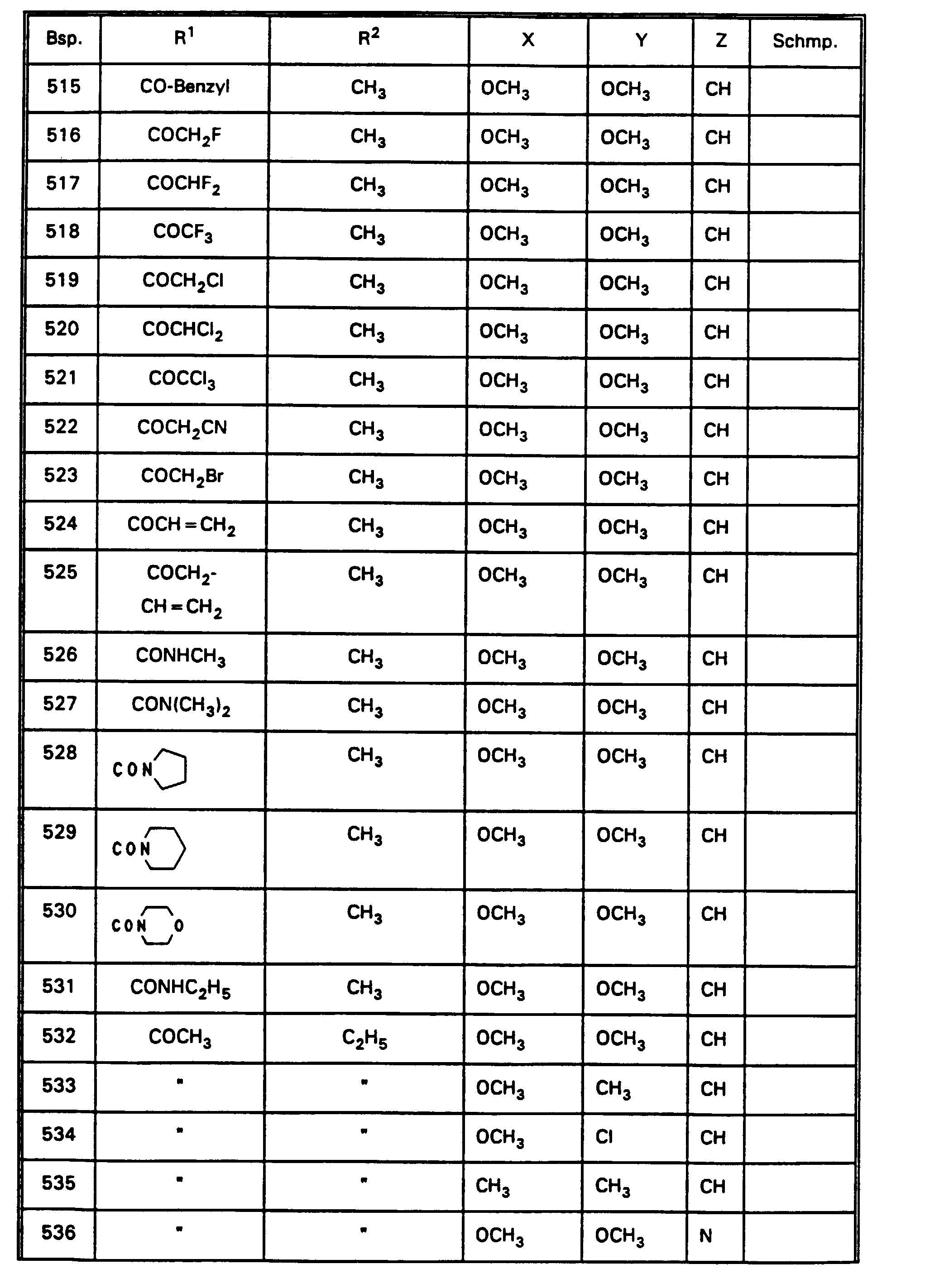

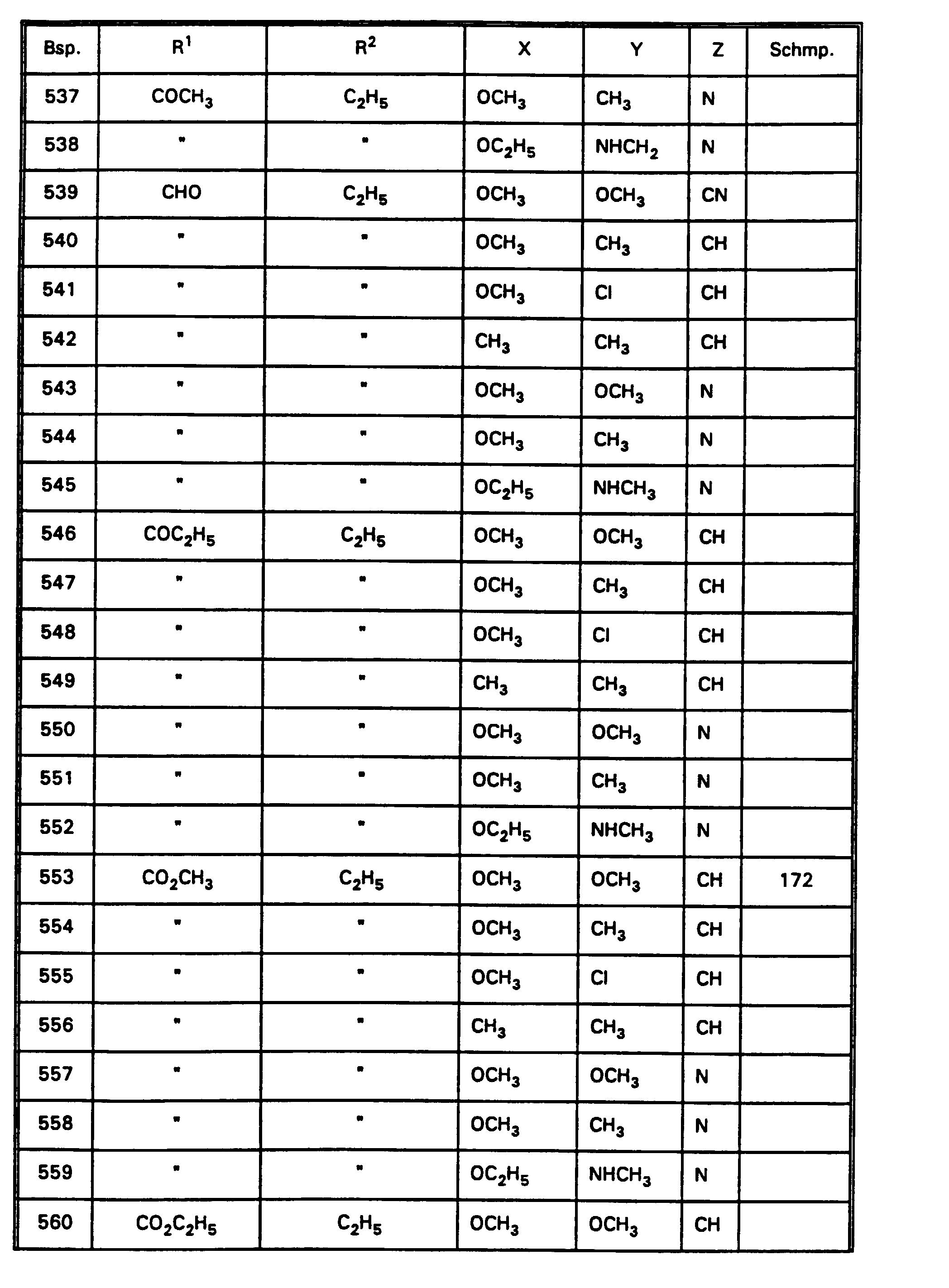

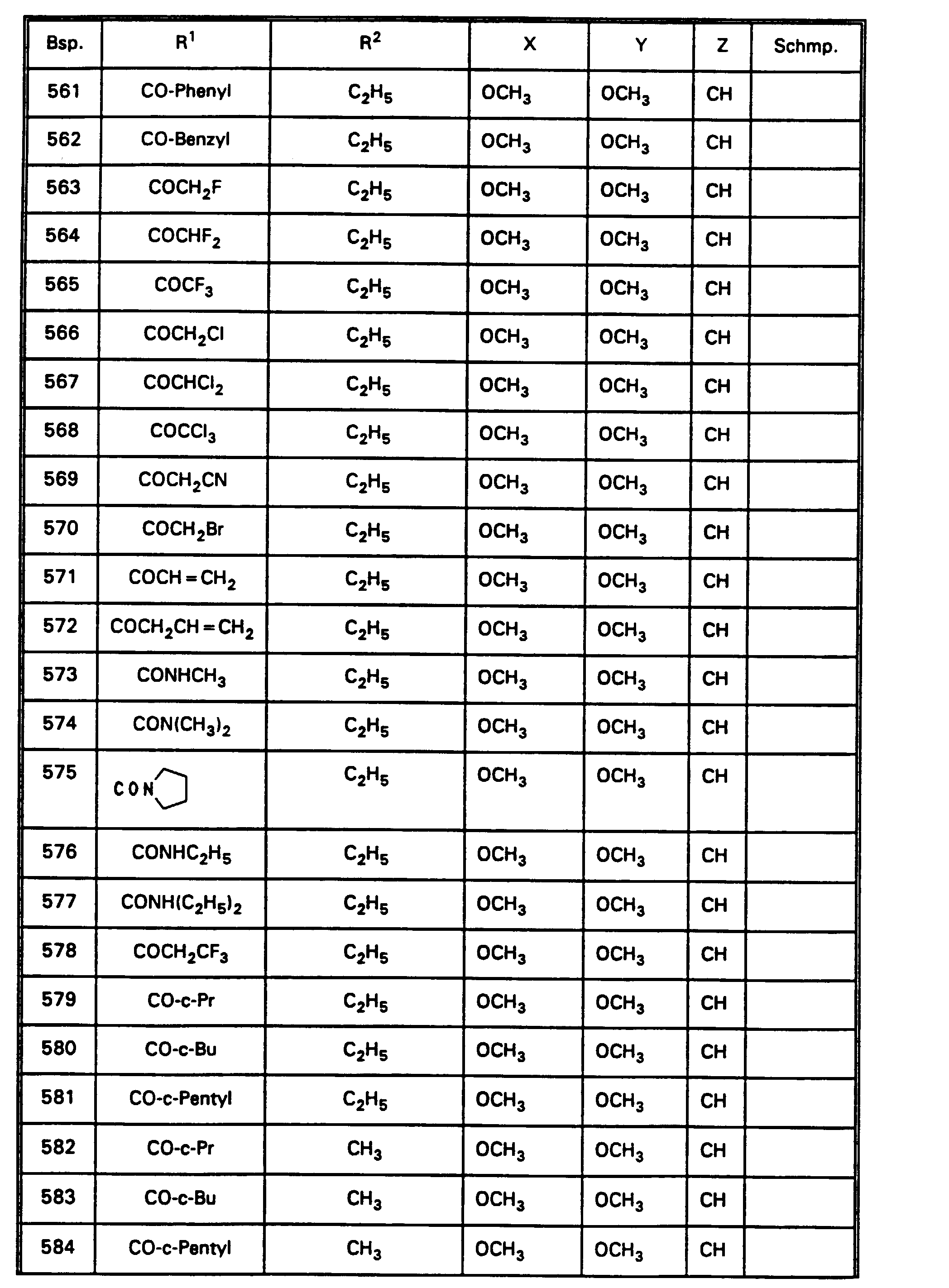

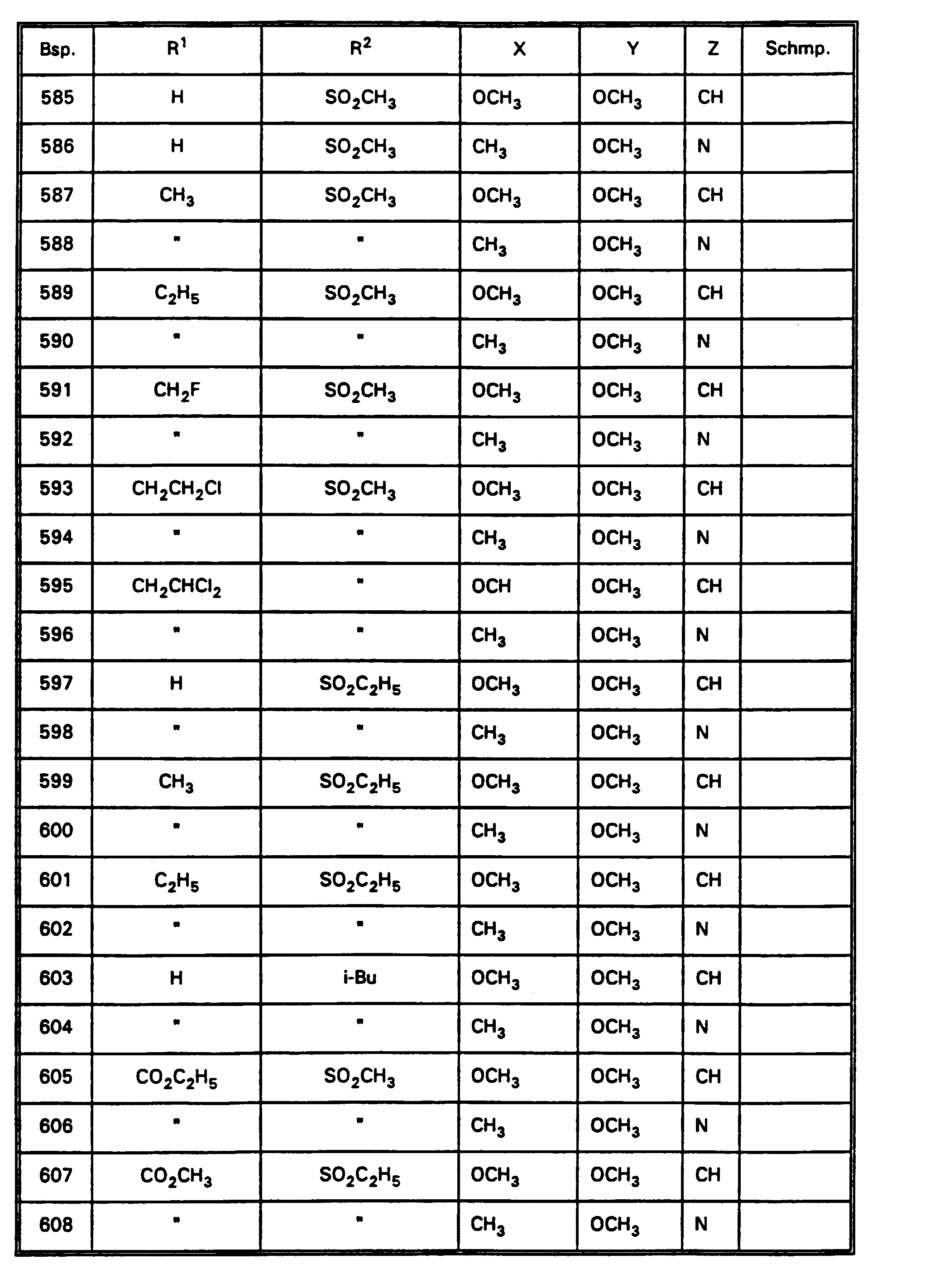

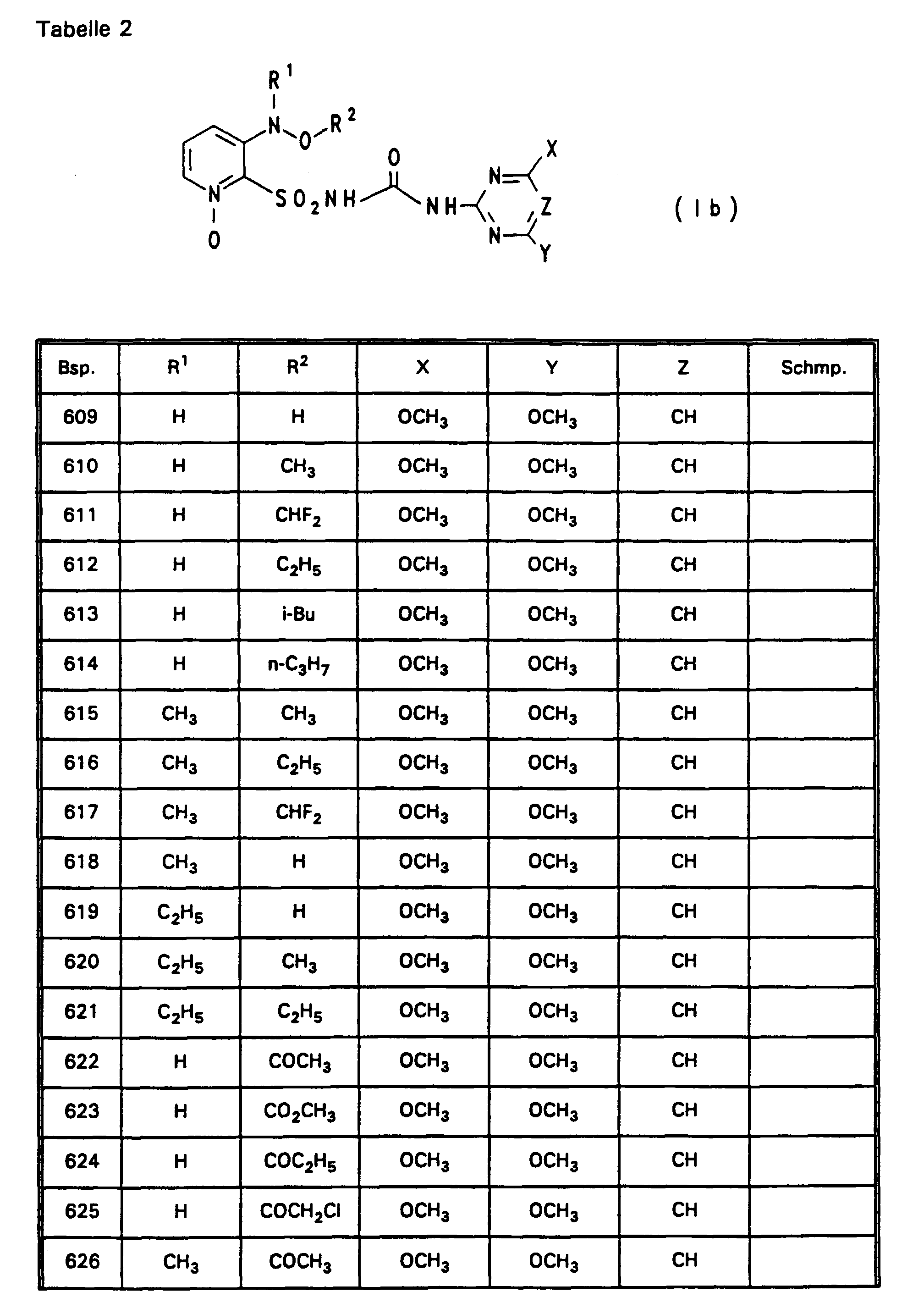

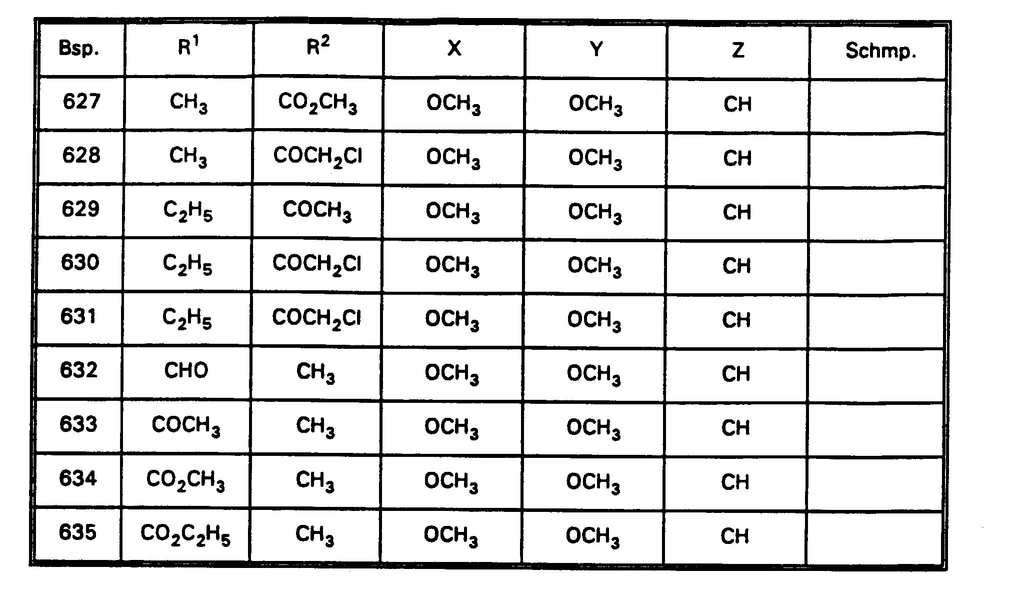

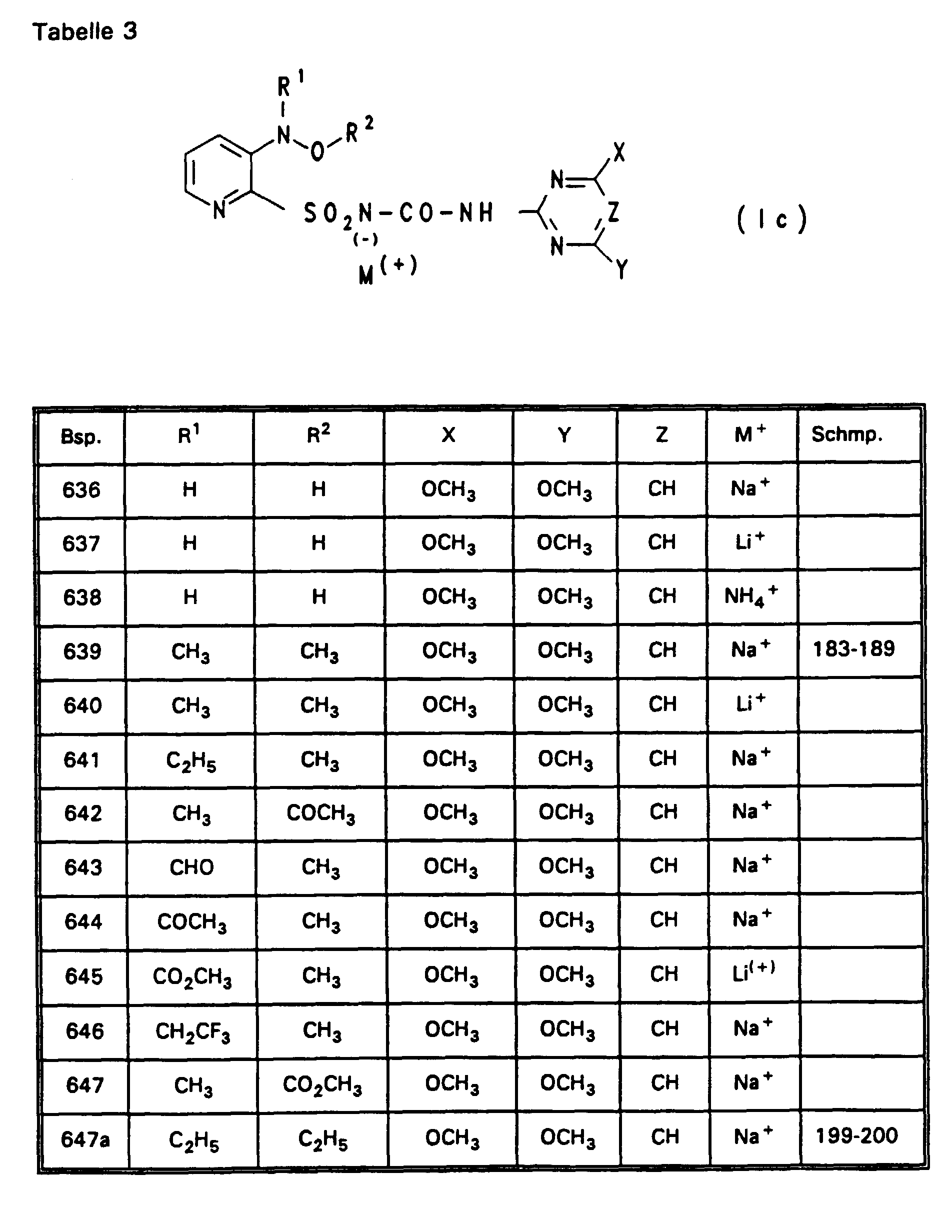

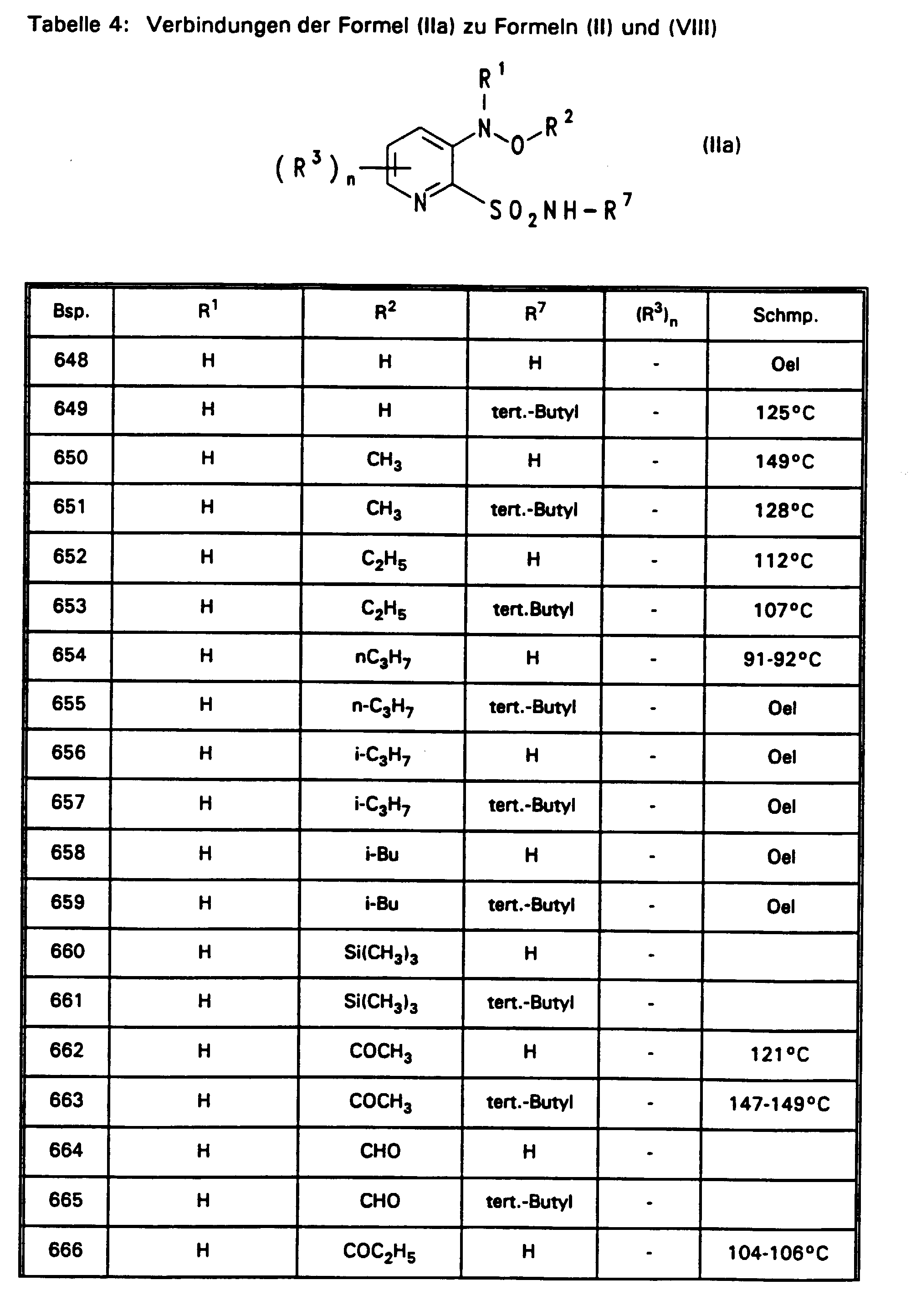

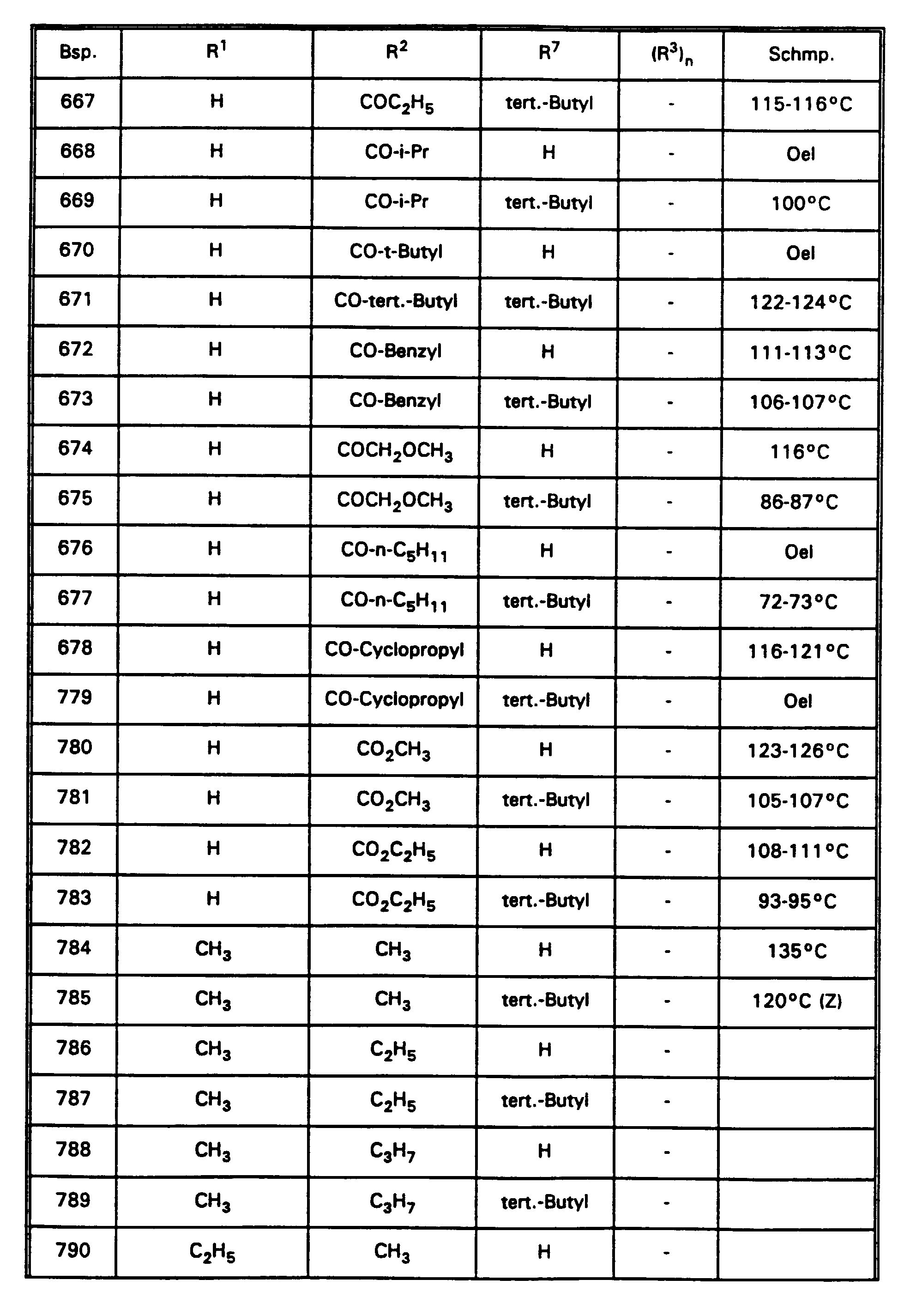

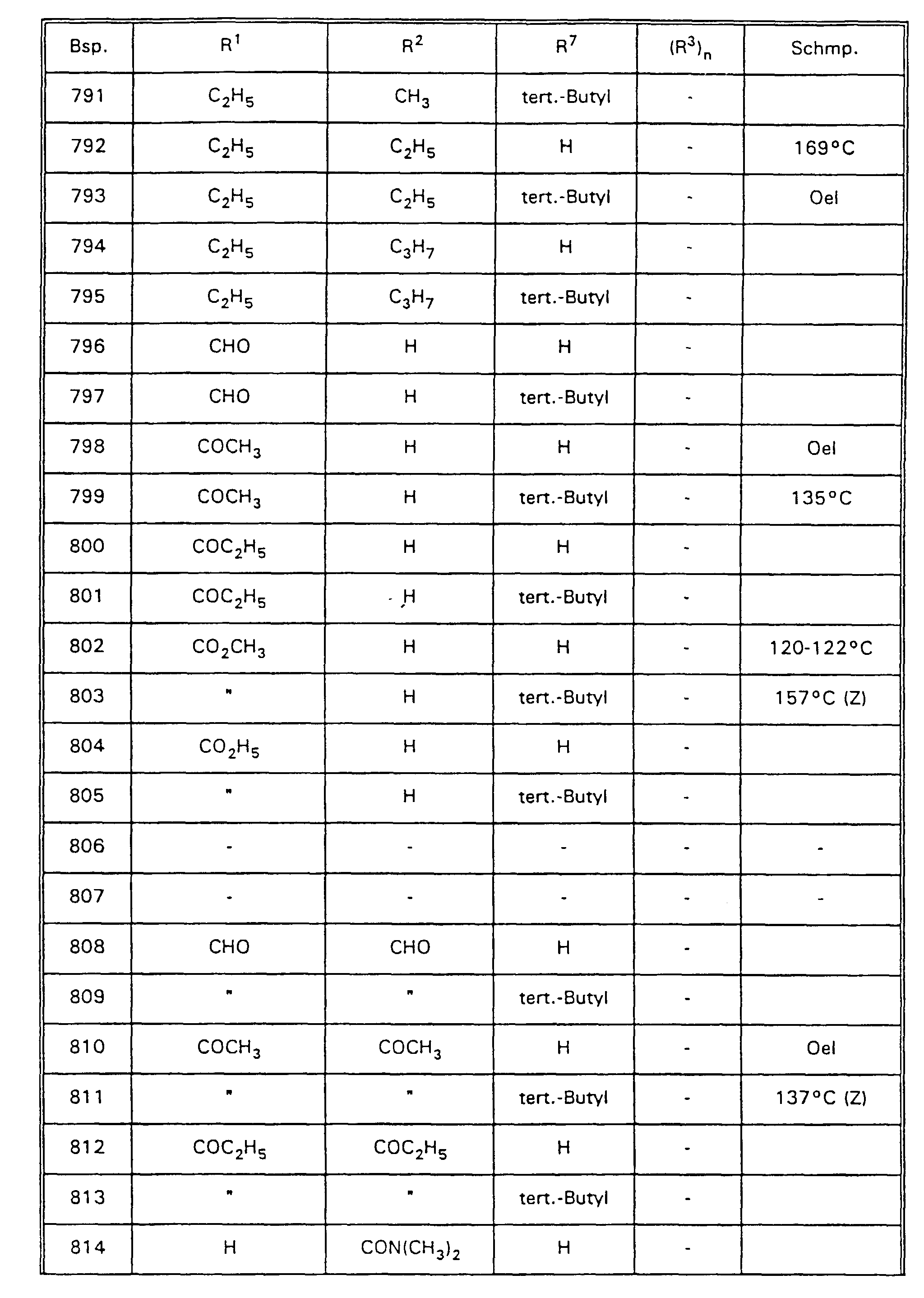

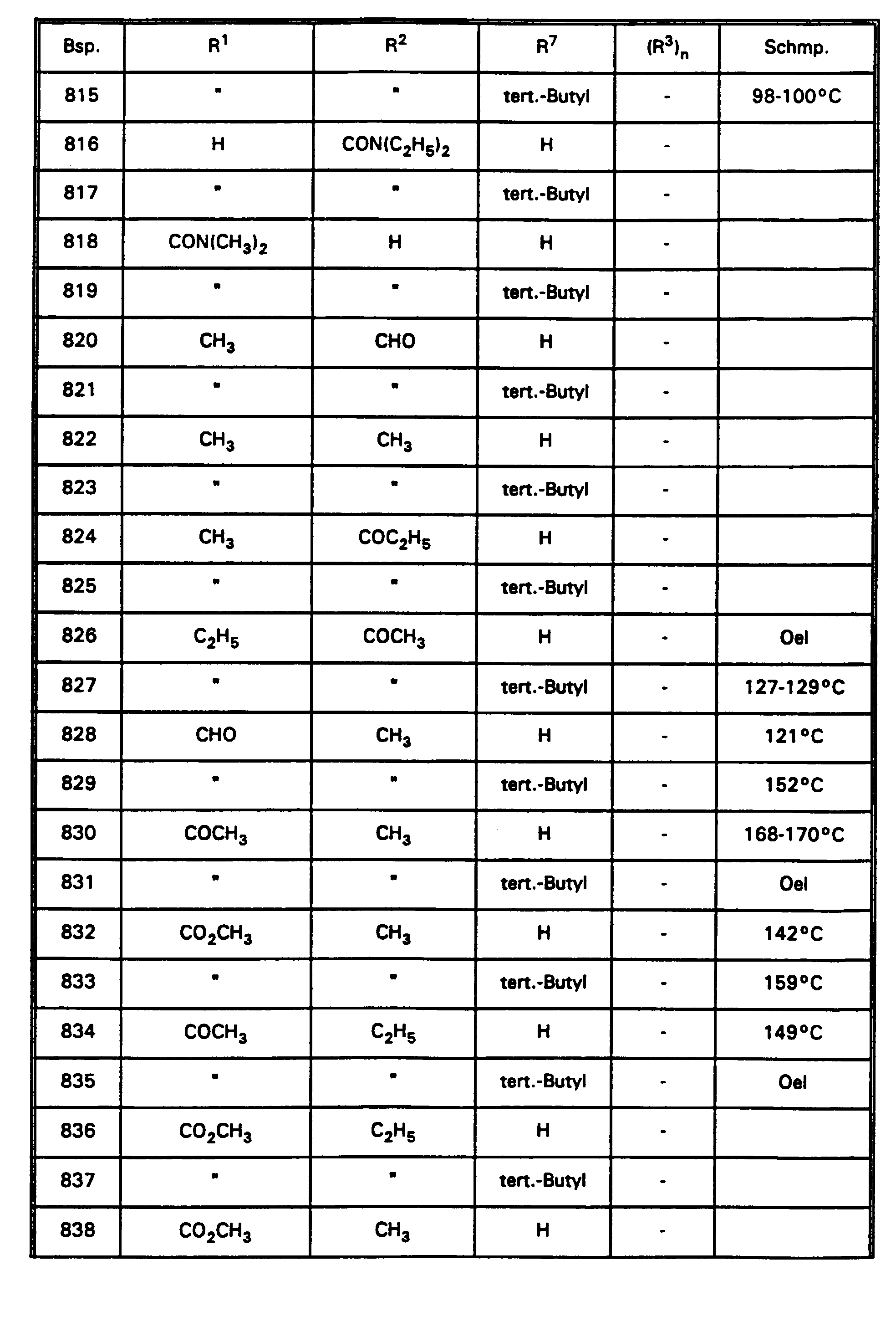

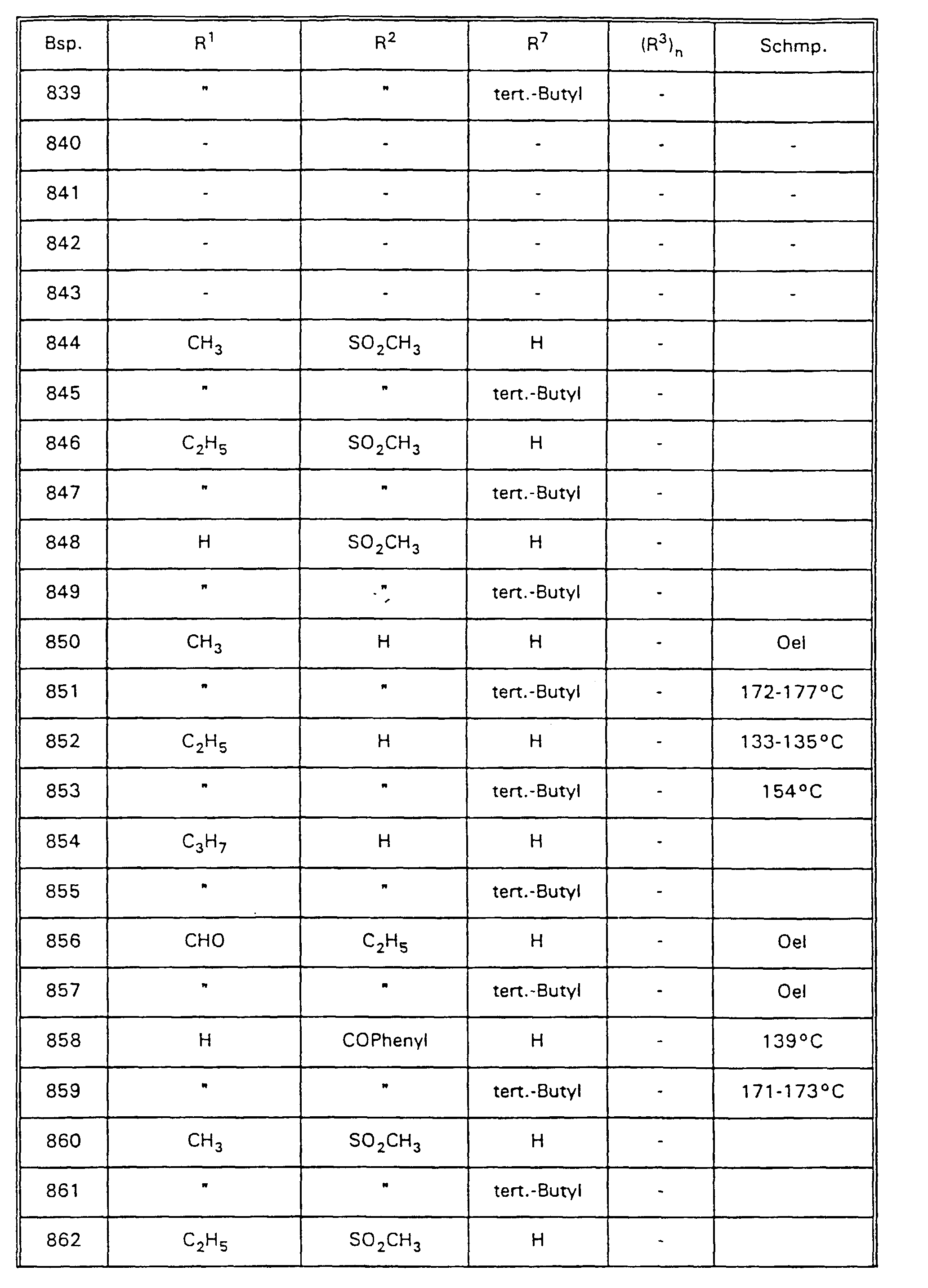

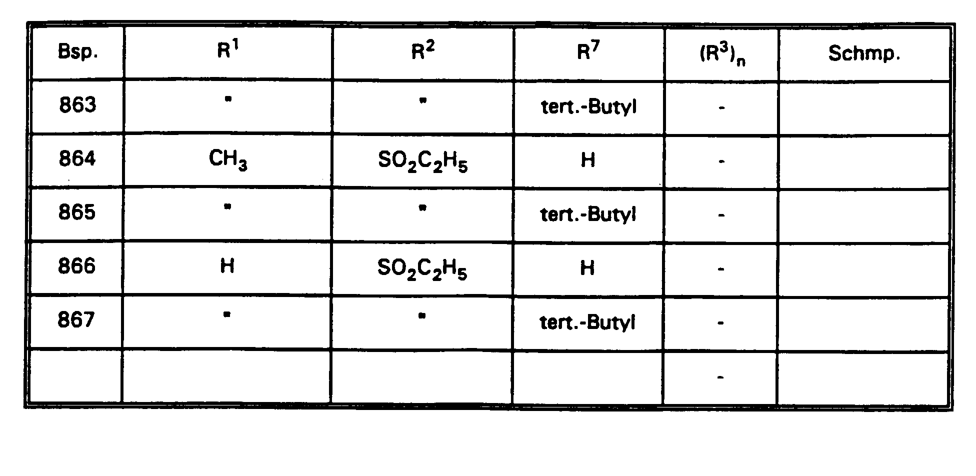

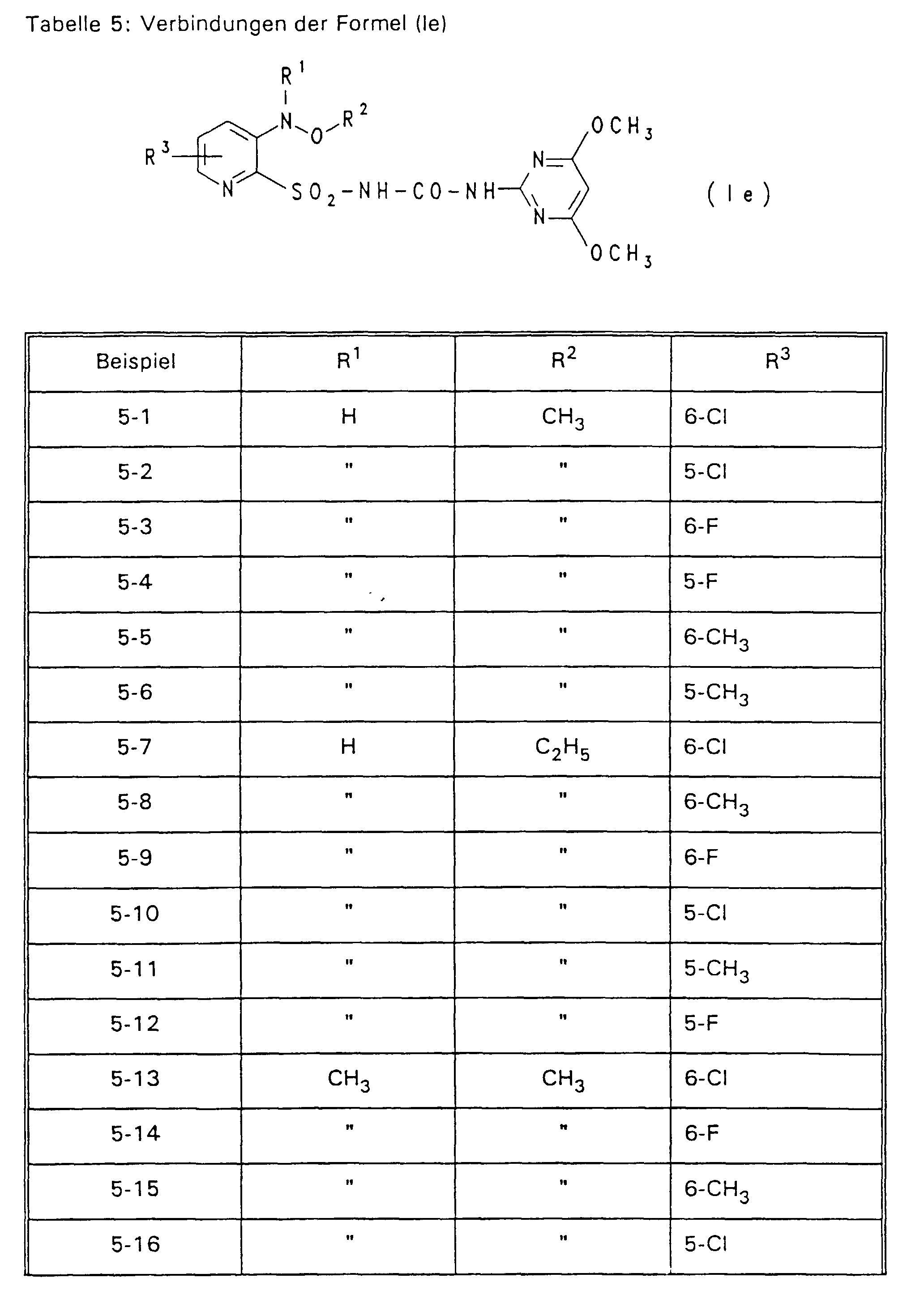

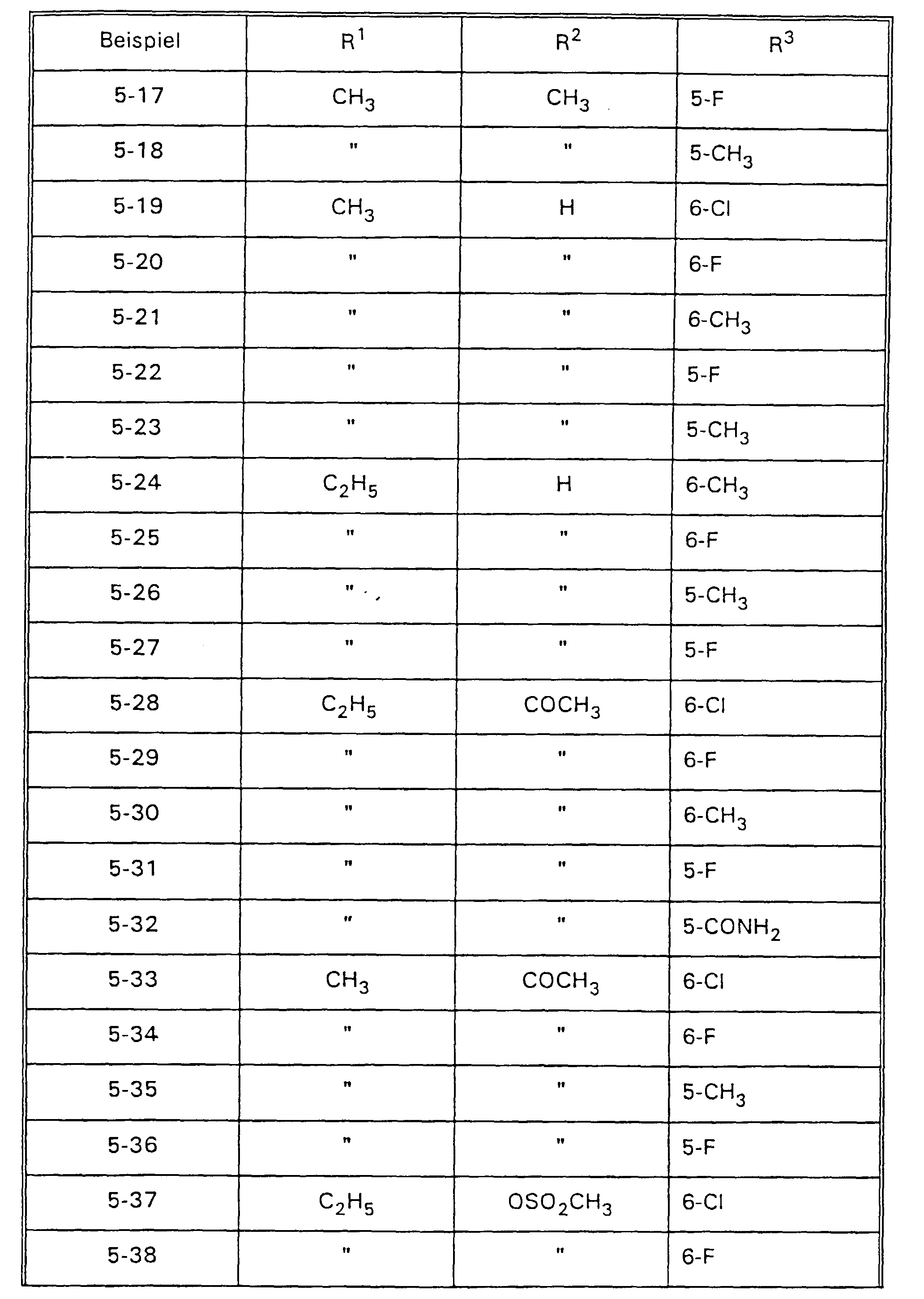

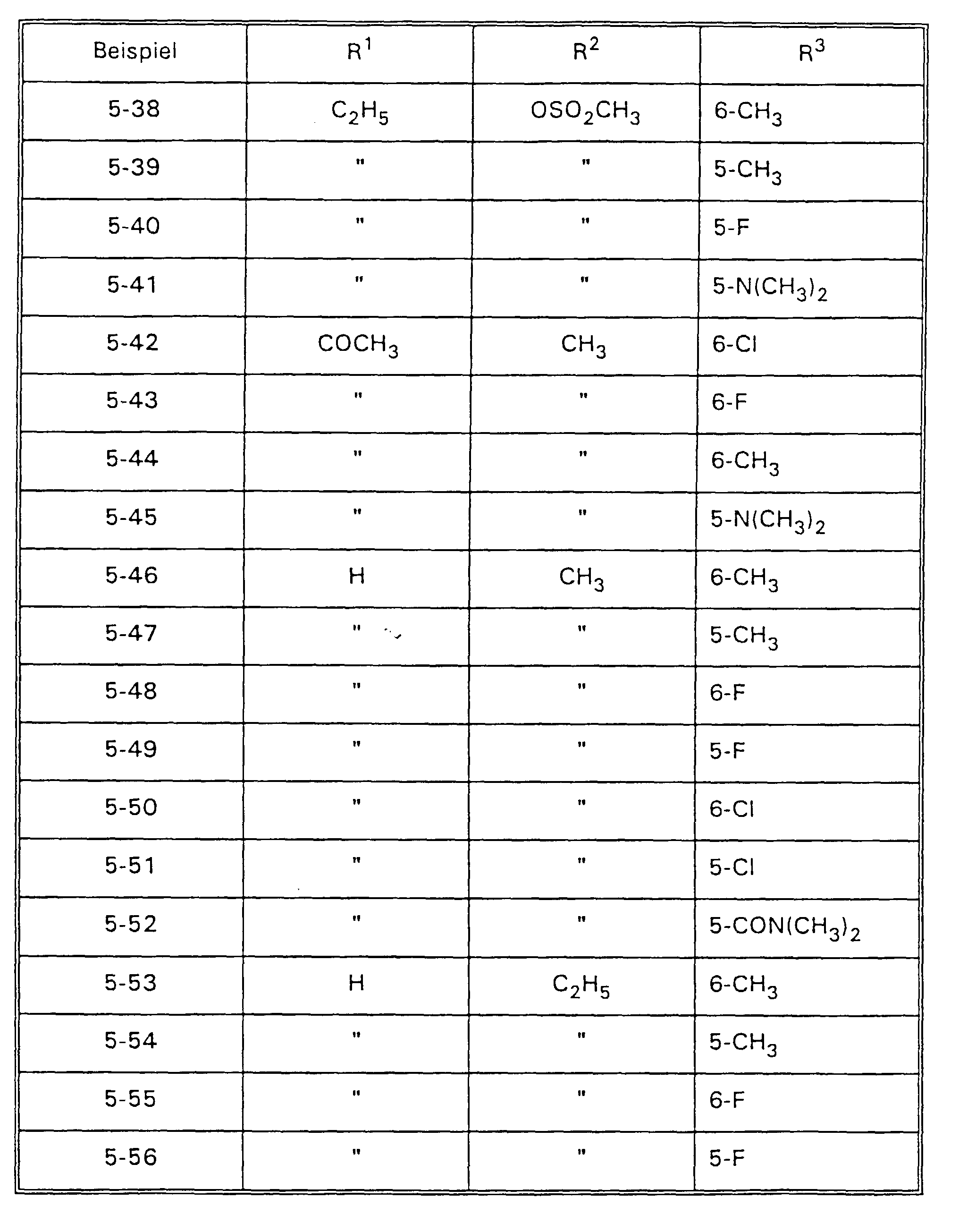

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird enthalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel micht und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.- Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (1), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| |
|---|
| 75 Gewichtsteile einer Verbindung der Formel (I), |
| 10 " ligninsulfonsaures Calcium, |
| 5 " Natriumlaurylsulfat, |
| 3 " Polyvinylalkohol und |
| 7 " Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| |
|---|
| 25 Gewichsteile einer Verbindung der Formel (I), |
| 5 " 2,2-dinaphthylmethan-6,6'-disulfonsaures Natrium, |
| 2 " oleoylmethyltaurinsaures Natirum, |
| 1 Gewichsteil Polyvinylalkohol, |
| 17 Gewichsteile Calciumcarbonat und |
| 50 " Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspension bzw. Emulsion mit einer Wasseraufwandmenge von umgerechnet 600 bis 800 I/ha in unterschiedlichen Dosierungen auf die Oberfläche der Abdeckerde appliziert. Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der obigen Beispiele (siehe Tabellen 1-3, 5) 1, 8, 15, 23, 29, 36, 43, 56, 69, 76, 90, 97, 111, 118, 125, 198, 333, 472, 500, 553, 639 und 647a sehr gute herbizide Wirkung gegen Schadplanzen wie Sinapis alba, Chrysanthemum segetum, Avena sativa, Stellaria media, Echinochloa crusgalli, Lolium multiflorum, Setaria spp., Abutilon theophrasti, Amaranthus retroflexus und Panicum miliaceum im Vorauflaufverfahren bei einer Aufwandmenge von 0,3 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 I/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen der genannten Beispiele (siehe Tabellen 1-3, 5) 1, 8, 15, 23, 29, 36, 43, 56, 69, 76, 90, 97, 111, 118, 125, 198, 333, 472, 500, 553, 639 und 647a sehr gute herbizide Wirkung gegen Schadplanzen wie Sinapis alba, Stellaria media, Echinochloa crus-galli, Lolium multiflorum, Chrysanthemum segetum, Setaria spp., Abutilon theophrasti, Amaranthus retroflexus und Panicum miliaceum, Avena sativa im Nachauflaufverfahren bei einer Aufwandmenge von 0,3 kg und weniger Aktivsubstanz pro Hektar.

## Patentansprüche

1. Verbindungen der Formel (I) oder deren Salze, 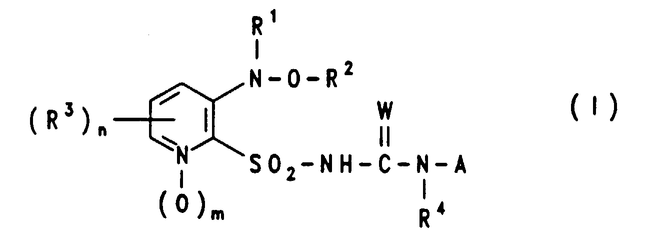 worin
R¹ H, (C₁-C₆)Alkyl, das unsubstituiert oder durch einen oder mehrere Reste aus der Gruppe Halogen, Nitro, (C₁-C₄)Alkoxy, (C₃-C₆)Cycloalkyl, Cyano, Aryl und substituiertes Aryl substituiert ist, (C₃-C₆)Cycloalkyl, das unsubstituiert oder durch einen oder mehrere Reste aus der Gruppe Halogen, (C₁-C₄)Alkyl und (C₁-C₄)Alkoxy substituiert ist, oder
(C₂-C₆)Alkenyl oder (C₂-C₆)Alkinyl, wobei jeder der beiden letztgenannten Reste unsubstituiert oder durch einen oder mehrere Reste aus der Gruppe Halogen, (C₁-C₄)Alkoxy und (C₃-C₆)Cycloalkyl substituiert ist, oder Aryl, substituiertes Aryl, oder einen Acylrest der Formel
-CO-R*,
worin
R* H, (C₁-C₆)Alkyl, (C₂-C₆)Alkenyl oder (C₂-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, Nitro, Cyano, Thiocyanato, Aryl und substituiertes Aryl substituiert ist, oder (C₁-C₄)Alkoxy, (C₂-C₄)Alkenyloxy, (C₂-C₄)Alkinyloxy oder (C₁-C₄)Alkylthio, wobei jeder der letztgenannten vier Reste unsubstituiert oder durch einen oder mehrere Reste aus der Gruppe Halogen, (C₁-C₄)Alkoxy, (C₁-C₄)Alkylthio, Aryl und substituiertes Aryl substituiert ist, oder (C₃-C₆)Cycloalkyl oder (C₃-C₆)Cycloalkoxy, wobei jeder der letztgenannten beiden Reste unsubstituiert oder durch einen oder mehrere Reste aus der Gruppe (C₁-C₄)Alkyl, (C₁-C₄)Alkoxy, (C₁-C₄)Alkylthio, (C₁-C₄)Haloalkyl, und Halogen substituiert ist, oder einen Rest der Formel NR^{a}R^{b} bedeutet,
R² H, (C₁-C₆)Alkyl, das unsubstituiert oder durch einen oder mehrere Reste aus der Gruppe Halogen, Nitro, (C₁-C₄)Alkoxy, (C₃-C₆)Cycloalkyl, Aryl und substituiertes Aryl substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, (C₁-C₄)Haloalkyl, Aryl und substituiertes Aryl substituiert ist, oder (C₂-C₆)Alkenyl oder (C₂-C₆)Alkinyl, wobei jeder der letztgenannten zwei Reste unsubstituiert oder durch einen oder mehrere Reste aus der Gruppe (C₁-C₄)Alkoxy, (C₁-C₄)Haloalkyl, (C₃-C₆)Cycloalkyl und Halogen substituiert ist, oder Aryl, substituiertes Aryl oder einen Rest der Formel R'R"R"'Si, worin R', R", R"' unabhängig voneinander (C₁-C₄)Alkyl bedeuten, oder einen Acylrest der Formel
-CO-R**,
worin
R** H, (C₁-C₆)Alkyl, (C₂-C₆)Alkenyl oder (C₂-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, Nitro, Cyano, Thiocyanato, Aryl und substituiertes Aryl substituiert ist, oder (C₁-C₄)Alkoxy, (C₂-C₄)Alkenyloxy oder (C₂-C₄)Alkinyloxy, wobei jeder der letztgenannten drei Reste unsubstituiert oder durch einen oder mehrere Reste aus der Gruppe Halogen, (C₁-C₄)Alkoxy, (C₁-C₄)Alkylthio, Aryl und substituiertes Aryl substituiert ist, oder (C₃-C₆)Cycloalkyl oder (C₃-C₆)Cycloalkoxy, wobei jeder der letztgenannten beiden Reste unsubstituiert oder durch einen oder mehrere Reste aus der Gruppe Halogen, (C₁-C₄)Alkyl, (C₁-C₄)Alkoxy, (C₁-C₄)Alkylthio und (C₁-C₄)Haloalkyl substituiert ist, oder einen Rest der Formel NR^{c}R^{d} bedeutet,
R³ (C₁-C₄)Alkyl, (C₁-C₃)Haloalkyl, Halogen, NO₂, CN, (C₁-C₃)Alkoxy, (C₁-C₃)Haloalkoxy, (C₁-C₃)Alkylthio, (C₁-C₃)Alkoxy-(C₁-C₃)alkyl, [(C₁-C₃)Alkoxy]-carbonyl, (C₁-C₃)Alkylamino, Di[(C₁-C₃)alkyl]-amino, (C₁-C₃)-Alkylsulfinyl, (C₁-C₃)Alkylsulfonyl, SO₂NR^{e}R^{f} oder C(O)NR^{g}R^{h},
R^{a}, R^{b}, R^{c}, R^{d}, R^{e}, R^{f}, R^{g} und R^{h} unabhängig voneinander H, (C₁-C₄)Alkyl, (C₃-C₆)Alkenyl, (C₃-C₆)Alkinyl, [(C₁-C₄)Alkyl]-carbonyl, Arylcarbonyl, das im Arylrest unsubstituiert oder durch einen oder mehrere Reste aus der Gruppe Halogen, (C₁-C₄)Alkyl und (C₁-C₄)Alkoxy substituiert ist, oder paarweise R^{a} und R^{b}, R^{c} und R^{d}, R^{e} und R^{f} bzw. R^{g} und R^{h} gemeinsam mit dem sie verbindenden N-Atom einen heterocyclischen gesättigten oder ungesättigten Ring mit 3 bis 7 Ringatomen, der neben dem N-Atom 1 oder 2 weitere Heteroatome aus der Gruppe N, O und S in den möglichen Oxidationsstufen enthalten kann und der unsubstituiert oder substituiert ist,
R⁴ H oder (C₁-C₄)Alkyl,
m 0 oder 1,
n 0, 1 oder 2,
A einen Rest der Formel 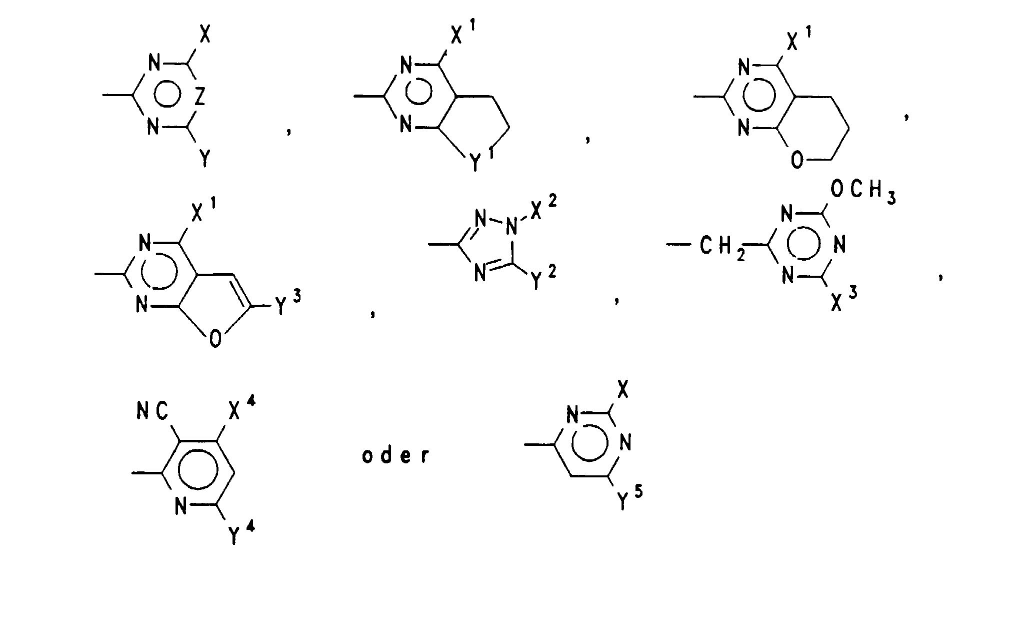
X,Y unabhängig voneinander H, Halogen, (C₁-C₃)Alkyl, (C₁-C₃)Alkoxy oder (C₁-C₃)Alkylthio, wobei die vorgenannten alkylhaltigen Reste unsubstituiert oder ein- oder mehrfach durch Halogen oder ein- oder zweifach durch (C₁-C₃)Alkoxy oder (C₁-C₃)Alkylthio substituiert sind, oder einen Rest der Formel NR⁵R⁶, (C₃-C₆)-Cycloalkyl, (C₂-C₄)Alkenyl, (C₂-C₄)Alkinyl, (C₃-C₄)Alkenyloxy oder (C₃-C₄)Alkinyloxy,
R⁵,R⁶ unabhängig voneinander H, (C₁-C₃)Alkyl oder (C₃-C₄)Alkenyl,
W O oder S,
Z CH oder N,
X¹ CH₃, OCH₃, OC₂H₅ oder OCF₂H,
Y¹ -O- oder -CH₂-,
X² CH₃, C₂H₅ oder CH₂CF₃,
Y² OCH₃, OC₂H₅, SCH₃, SC₂H₅, CH₃ oder C₂H₅,
X³ CH₃ oder OCH₃,
Y³ H oder CH₃,
X⁴ CH₃, OCH₃, OC₂H₅, CH₂OCH₃ oder Cl,
Y⁴ CH₃, OCH₃, OC₂H₅ oder Cl,
Y⁵ CH₃, C₂H₅, OCH₃ oder Cl
bedeuten.

2. Verbindungen oder deren Salze nach Anspruch 1, dadurch gekennzeichnet, daß
R¹ H, (C₁-C₄)Alkyl, das unsubstituiert oder durch einen oder mehrere Reste aus der Gruppe Halogen, (C₁-C₄)Alkoxy, (C₃-C₆)Cycloalkyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist, oder
(C₃-C₆)Cycloalkyl, (C₂-C₄)Alkenyl, (C₂-C₄)Haloalkenyl,(C₂-C₄)Alkinyl, Phenyl, substituiertes Phenyl oder einen Rest der Formel
-CO-R*
worin
R* H, (C₁-C₄)Alkyl, (C₂-C₄)Alkenyl oder (C₂-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylsulfonyl, Phenyl und substituiertes Phenyl substituiert ist, oder (C₁-C₄)Alkoxy, das unsubstituiert oder durch einen oder mehrere Reste aus der Gruppe Halogen, (C₁-C₄)Alkoxy, (C₁-C₄)Alkylthio, Phenyl und substituiertes Aryl substituiert ist, oder (C₃-C₆)Cycloalkyl oder (C₃-C₆)Cycloalkoxy, wobei jeder der letztgenannten beiden Reste unsubstituiert oder durch einen oder mehrere Reste aus der Gruppe (C₁-C₂)Alkyl, (C₁-C₂)Alkoxy und Halogen substituiert ist, oder einen Rest der Formel NR^{a}R^{b} bedeutet, bedeutet.

3. Verbindungen oder deren Salze nach Anspruch 1 oder 2, dadurch gekennzeichnet, daß
R¹ H, (C₁-C₄)Alkyl, das unsubstituiert oder durch 1 bis 3 Reste aus der Gruppe Halogen oder durch (C₁-C₂)Alkoxy oder (C₃-C₅)Cycloalkyl substituiert ist, oder einen Rest der Formel -CO-R* bedeutet, worin
R* H, (C₁-C₄)Alkyl, das unsubstituiert oder durch einen oder mehrere Halogenatome oder durch (C₁-C₂)Alkoxy substituiert ist, oder (C₂-C₄)Alkenyl, (C₂-C₃)Alkinyl, (C₃-C₅)Cycloalkyl, (C₁-C₄)Alkoxy, das unsubstituiert oder durch ein oder mehrere Halogenatome oder durch Phenyl substituiert ist, oder einen Rest der Formel NR^{a}R^{b}, worin R^{a} und R^{b} unabhängig voneinander H oder (C₁-C₄)Alkyl sind, bedeutet,
R² H, (C₁-C₄)Alkyl, das unsubstituiert oder durch einen oder mehrere Reste aus der Gruppe Halogen, (C₁-C₄)Alkoxy, (C₃-C₆)Cycloalkyl, Phenyl und substituiertes Phenyl substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy oder (C₁-C₄)Haloalkyl substituiert ist, oder (C₂-C₄)Alkenyl oder (C₂-C₄)Alkinyl, wobei jeder der letztgenannten zwei Reste unsubstituiert oder durch einen oder mehrere Reste aus der Gruppe (C₁-C₄)Alkoxy, (C₁-C₄)Haloalkyl und Halogen substituiert ist, oder Phenyl, substituiertes Phenyl oder einen Rest der Formel R'R"R"'Si, worin R', R", R"' unabhängig voneinander (C₁-C₄)Alkyl bedeuten, oder einen Acylrest der Formel
-CO-R**,
worin
R** H, (C₁-C₄)Alkyl, (C₂-C₄)Alkenyl oder (C₂-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)-Alkylsulfonyl, Phenyl und substituiertes Phenyl substituiert ist, oder (C₁-C₄)Alkoxy, (C₃-C₄)Alkenyloxy oder (C₃-C₄)Alkinyloxy, wobei jeder der letztgenannten drei Reste unsubstituiert oder durch einen oder mehrere Reste aus der Gruppe Halogen, (C₁-C₄)Alkoxy, (C₁-C₄)Alkylthio, Phenyl und substituiertes Phenyl substituiert ist, oder (C₃-C₆)Cycloalkyl oder (C₃-C₆)Cycloalkoxy, wobei jeder der letztgenannten beiden Reste unsubstituiert oder durch einen oder mehrere Reste aus der Gruppe Halogen, (C₁-C₄)Alkyl, (C₁-C₄)Alkoxy, und (C₁-C₄)Haloalkyl substituiert ist, oder einen Rest der Formel NR^{c}R^{d} bedeutet, und
R³ (C₁-C₃)Alkyl, (C₁-C₃)Haloalkyl, Halogen, NO₂, (C₁-C₃)Alkoxy, (C₁-C₃)Haloalkoxy, (C₁-C₂)Alkoxy-(C₁-C₂)alkyl, Acetyl, Propionyl, (C₁-C₂)Alkylamino, Di[(C₁-C₂)alkyl]-amino, (C₁-C₃)Alkylsulfonyl, SO₂NR^{e}R^{f} oder C(O)NR^{g}R^{h}
bedeuten.

4. Verbindungen oder deren Salze nach einem der Ansprüche 1 bis 3, dadurch gekennzeichnet, daß
R¹ H, (C₁-C₄)Alkyl, (C₁-C₄)Haloalkyl oder [(C₁-C₂)Alkoxy]-(C₁-C₂)alkyl, oder einen Rest der Formel -CO-R*, worin
R* H, (C₁-C₄)Alkyl, Halo-(C₁-C₄)alkyl, (C₂-C₄)Alkenyl, (C₂-C₄)Alkinyl, (C₁-C₄)Alkoxy, [(C₁-C₂)Alkoxy]-(C₁-C₂)alkyl, (C₃-C₅)Cycloalkyl oder einen Rest der Formel NR^{a}R^{b}, worin R^{a} und R^{b} unabhängig voneinander H oder (C₁-C₄)Alkyl bedeuten,
R² H, (C₁-C₄)Alkyl, (₁-C₄)Haloalkyl, [(C₁-C₂)Alkoxy]-(C₁-C₂)Alkyl oder einen Rest der Formel -CO-R**, worin
R** H, (C₁-C₄)Alkyl, (C₁-C₄)Haloalkyl, (C₃-C₄)Alkenyl, (C₃-C₄)Alkinyl, (C₁-C₄)Alkoxy, [(C₁-C₂)Alkoxy]-(C₁-C₂)Alkyl, (C₃-C₅)Cycloalkyl oder einen Rest der Formel NR^{c}R^{d} ist, worin R^{c} und R^{d} unabhängig voneinander H oder (C₁-C₄)Alkyl bedeuten,
R³ (C₁-C₄)Alkyl, Halogen, Nitro oder (C₁-C₄)Alkoxy
n 0 oder 1 und
A ein Rest der Formel 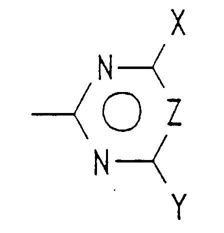 worin
einer der Reste X und Y (C₁-C₃)Alkyl, (C₁-C₃)Alkoxy, (C₁-C₃)Haloalkyl, (C₁-C₃)Haloalkoxy oder [(C₁-C₂)Alkoxy]-(C₁-C₂)alkyl ist
und der andere Rest Y bzw. X (C₁-C₃)Alkyl, (C₁-C₃)Alkoxy, (C₁-C₃)Alkylthio, wobei jeder der letztgenannten 3 Reste unsubstituiert oder ein- oder mehrfach durch Halogen oder ein- oder zweifach durch (C₁-C₃)Alkoxy oder (C₁-C₃)Alkylthio substituiert ist, oder Halogen oder ein Rest der Formel NR⁵R⁶, worin R⁵ und R⁶ unabhängig voneinander H, (C₁-C₂)Alkyl oder (C₃-C₄)Alkenyl sind, oder (C₃-C₆)Cycloalkyl, (C₂-C₄)Alkenyl, (C₂-C₄)Alkinyl, (C₃-C₄)Alkenyloxy oder (C₃-C₄)Alkinyloxy ist,
bedeuten.

5. Verfahren zur Herstellung der Verbindungen der Formel (I) oder deren Salze gemäß Anspruch 1, dadurch gekennzeichnet, daß man
a) eine Verbindung der Formel (II), 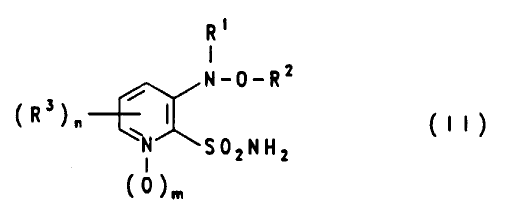 mit einem heterocyclischen Carbamat der Formel (III),
R°-O-CO-NR⁴-A (III)
worin R° gegebenenfalls substituiertes Phenyl oder (C₁-C₄)Alkyl bedeutet, umsetzt oder
b) ein Pyridylsulfonylcarbamat der Formel (IV), 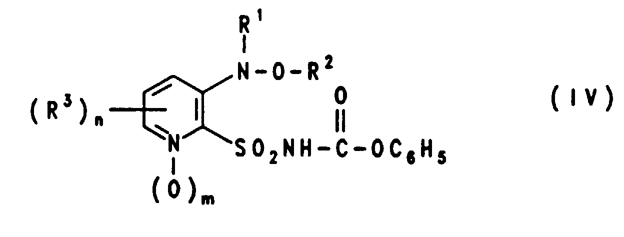 mit einem Aminoheterocyclus der Formel (V)
H-NR⁴-A (V)
umsetzt oder
c) ein Sulfonylisocyanat der Formel (Vl) 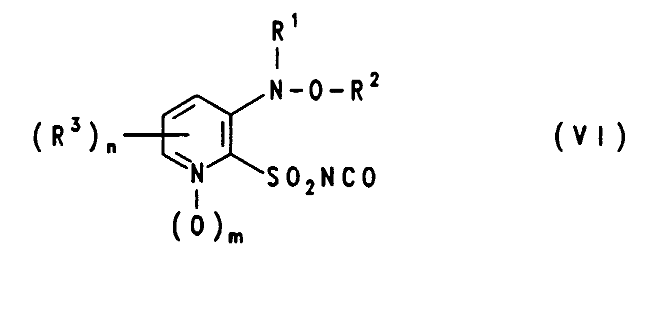 mit einem Aminoheterocyclus der Formel (V)
H-NR⁴-A (V)
umsetzt oder
d) in einer Eintopfreaktion zunächst einen Aminoheterocyclus der Formel H-NR⁴-A (V) in Gegenwart einer Base mit Phosgen umsetzt und das gebildete Intermediat mit einem Pyridinsulfonamid der Formel (II) umsetzt, oder
e) ein Sulfonamid der genannten Formel (II) mit einem (Thio-)lsocyanat der Formel (VII) 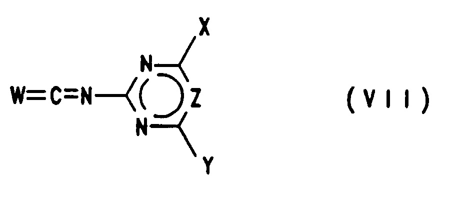 in Gegenwart einer Base umsetzt,
wobei in den Formeln (II)-(VII) die Reste R¹, R², R³, R⁴, A, W, X, Y und Z sowie m und n wie in Formel (I) definiert sind.

6. Herbizides oder pflanzenwachstumsregulierendes Mittel, dadurch gekennzeichnet, daß es mindestens eine Verbindung der Formel (I) oder deren Salze nach einem der Ansprüche 1 bis 4 und im Pflanzenschutz übliche Formulierungshilfsmittel enthält.

7. Verfahren zur Bekämpfung von Schadpflanzen oder zur Wachstumsregulierung von Pflanzen, dadurch gekennzeichnet, daß man ein wirksame Menge von mindestens einer Verbindung der Formel (I) oder deren Salze nach einem der Ansprüche 1 bis 4 auf die Schadpflanzen bzw. Pflanzen, deren Pflanzensamen oder die Fläche, auf der die Pflanzen wachsen, appliziert.

8. Verwendung der Verbindungen der Formel (I) oder deren Salze nach einem der Ansprüche 1 bis 4 als Herbizide oder Pflanzenwachstumsregulatoren.

9. Verbindungen der Formel (II), 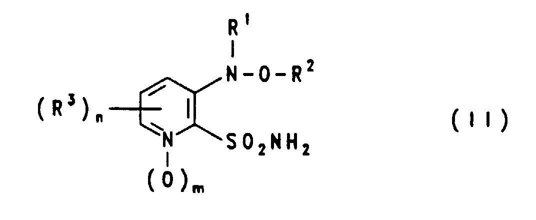 worin R¹, R², R³, n und m wie in Formel (I) nach einem der Anspüche 1 bis 4 definiert sind.

10. Verbindungen der Formel (VIII) 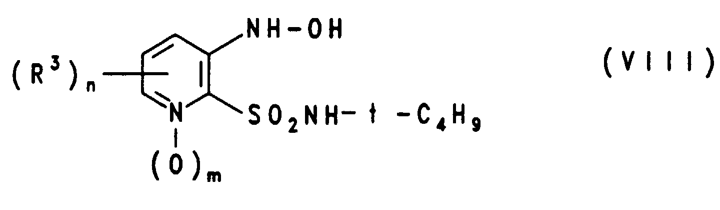 worin R¹, R², R³, n und m wie in Formel (I) nach einem der Anspüche 1 bis 4 definiert sind.

## Claims

1. A compound of the formula (I) or a salt thereof 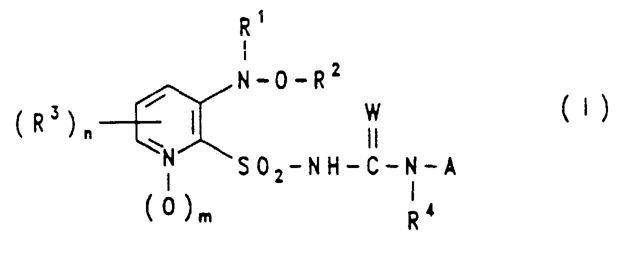 in which
R¹ is H, (C₁-C₆)alkyl which is unsubstituted or substituted by one or more radicals selected from the group consisting of halogen, nitro, (C₁-C₄)alkoxy, (C₃-C₆)cycloalkyl, cyano, aryl and substituted aryl, (C₃-C₆)cycloalkyl which is unsubstituted or substituted by one or more radicals selected from the group consisting of halogen, (C₁-C₄)alkyl and (C₁-C₄)alkoxy, or (C₂-C₆)alkenyl or (C₂-C₆)alkynyl, each of the two last-mentioned radicals being unsubstituted or substituted by one or more radicals selected from the group consisting of halogen, (C₁-C₄)alkoxy and (C₃-C₆)cycloalkyl, or is aryl, substituted aryl, or an acyl radical of the formula
-CO-R*
in which
R* is H, (C₁-C₆)alkyl, (C₂-C₆)alkenyl or (C₂-C₆)alkynyl, each of the three last-mentioned radicals being unsubstituted or substituted by one or more radicals selected from the group consisting of halogen, (C₁-C₄)alkoxy, (C₁-C₄)alkylthio, (C₁-C₄)alkylsulfinyl, (C₁-C₄)alkylsulfonyl, nitro, cyano, thiocyanato, aryl and substituted aryl, or is (C₁-C₄)alkoxy, (C₂-C₄)alkenyloxy, (C₂-C₄)alkynyloxy or (C₁-C₄)alkylthio, each of the last-mentioned four radicals being unsubstituted or substituted by one or more radicals selected from the group consisting of halogen, (C₁-C₄)alkoxy, (C₁-C₄)alkylthio, aryl and substituted aryl, or is (C₃-C₆)cycloalkyl or (C₃-C₆)cycloalkoxy, each of the lastmentioned two radicals being unsubstituted or substituted by one or more radicals selected from the group consisting of (C₁-C₄)alkyl, (C₁-C₄)alkoxy, (C₁-C₄)alkylthio, (C₁-C₄)haloalkyl, and halogen, or is a radical of the formula NR^{a}R^{b},
R² is H, (C₁-C₆)alkyl which is unsubstituted or substituted by one or more radicals selected from the group consisting of halogen, nitro, (C₁-C₄)alkoxy, (C₃-C₆)cycloalkyl, aryl and substituted aryl, or (C₃-C₆)cycloalkyl which is unsubstituted or substituted by one or more radicals selected from the group consisting of halogen, (C₁-C₄)alkyl, (C₁-C₄)alkoxy, (C₁-C₄)haloalkyl, aryl and substituted aryl, or (C₂-C₆)alkenyl or (C₂-C₆)alkynyl, each of the last-mentioned two radicals being unsubstituted or substituted by one or more radicals selected from the group consisting of (C₁-C₄)alkoxy, (C₁-C₄)haloalkyl, (C₃-C₆)cycloalkyl and halogen, or aryl, substituted aryl or a radical of the formula R'R"R"'Si, in which R', R" and R"' independently of one another are (C₁-C₄)alkyl, or an acyl radical of the formula
-CO-R**
in which
R** is H, (C₁-C₆)alkyl, (C₂-C₆)alkenyl or (C₂-C₆)alkynyl, each of the three last-mentioned radicals being unsubstituted or substituted by one or more radicals selected from the group consisting of halogen, (C₁-C₄)alkoxyl (C₁-C₄)alkylthio, (C₁-C₄)alkylsulfinyl, (C₁-C₄)alkylsulfonyl, nitro, cyano, thiocyanato, aryl and substituted aryl, or is (C₁-C₄)alkoxy, (C₂-C₄)alkenyloxy or (C₂-C₄)alkynyloxy, each of the last-mentioned three radicals being unsubstituted or substituted by one or more radicals selected from the group consisting of halogen, (C₁-C₄)alkoxy, (C₁-C₄)alkylthio, aryl and substituted aryl, or is (C₃-C₆)cycloalkyl or (C₃-C₆)cycloalkoxyl each of the last-mentioned two radicals being unsubstituted or substituted by one or more radicals selected from the group consisting of halogen, (C₁-C₄)alkyl, (C₁-C₄)alkoxy, (C₁-C₄)alkylthio and (C₁-c₄)haloalkyl, or is a radical of the formula NR^{c}R^{d},
R³ is (C₁-C₄)alkyl, (C₁-C₃)haloalkyl, halogen, NO₂, CN, (C₁-C₃)alkoxy, (C₁-C₃)haloalkoxy, (C₁-C₃)alkylthio, (C₁-C₃)alkoxy- (C₁-C₃)alkyl, [(C₁-C₃)alkoxy]carbonyl, (C₁-C₃)alkylamino, di[(C₁-C₃)alkyl]amino, (C₁-C₃)alkylsulfinyl, (C₁-C₃)alkylsulfonyl, SO₂NR^{e}R^{f} or C(O)NR^{g}R^{h},
R^{a}, R^{b}, R^{c}, R^{d}, R^{e}, R^{f}, R^{g} and R^{h} independently of one another are H, (C₁-C₄)alkyl, (C₃-C₆)alkenyl, (C₃-C₆)alkynyl, [(C₁-C₄)alkyl] carbonyl, arylcarbonyl which is unsubstituted in the aryl radical or substituted by one or more radicals selected from the group consisting of halogen, (C₁-C₄)alkyl and (C₁-C₄)alkoxy, or the pairs R^{a} and R^{b}, R^{c} and R^{d}, R^{e} and R^{f} or R^{g} and R^{h} together with the nitrogen atom linking them are a heterocyclic saturated or unsaturated ring having 3 to 7 ring atoms, which can contain, besides the nitrogen atom, 1 or 2 further heteroatons selected from the group consisting of N, O and S at the oxidation levels which are possible and which is unsubstituted or substituted,
R⁴ is H or (C₁-C₄)alkyl,
m is 0 or 1,
n is 0, 1 or 2,
A is a radical of the formula 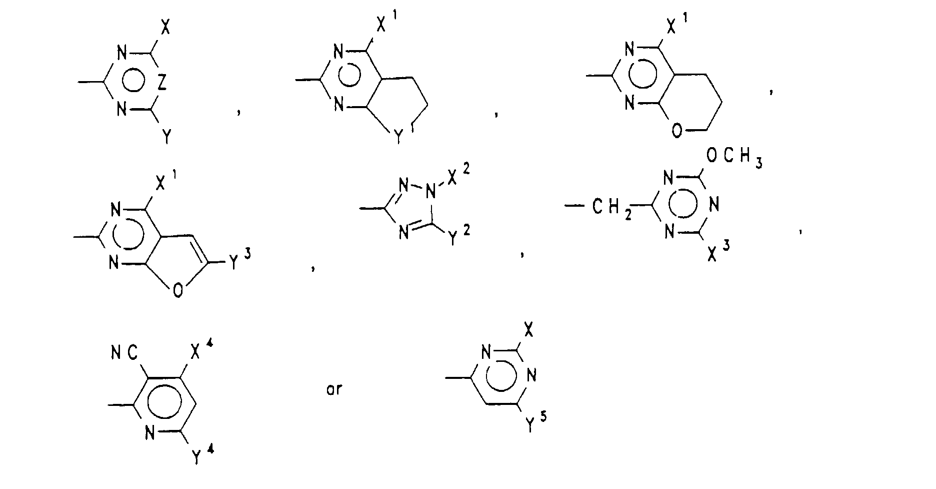
X and Y independently of one another are H, halogen, (C₁-C₃)alkyl, (C₁-C₃)alkoxy or (C₁-C₃)alkylthio, with the abovementioned alkyl-containing radicals being unsubstituted or mono- or polysubstituted by halogen or mono- or disubstituted by (C₁-C₃)alkoxy or (C₁-C₃)alkylthio, or are a radical of the formula NR⁵R⁶, (C₃-C₆)-cycloalkyl, (C₂-C₄)alkenyl, (C₂-C₄)alkynyl, (C₃-C₄)alkenyloxy or (C₃-C₄)alkynyloxy,
R⁵ and R⁶ independently of one another are H, (C₁-C₃)alkyl or (C₃-C₄)alkenyl,
W is O or S,
Z is CH or N,
X¹ is CH₃, OCH₃, OC₂H₅ or OCF₂H,
Y¹ is -O- or -CH₂-,
X² is CH₃, C₂H₅ or CH₂CF₃,
Y² is OCH₃, OC₂H₅, SCH₃, SC₂H₅, CH₃ or C₂H₅,
X³ is CH₃ or OCH₃,
Y³ is H or CH₃,
X⁴ is CH₃, OCH₃, OC₂H₅, CH₂OCH₃ or Cl,
Y⁴ is CH₃, OCH₃, OC₂H₅ or Cl and
Y⁵ is CH₃, C₂H₅, OCH₃ or Cl.

2. A compound or a salt thereof as claimed in claim 1, wherein
R¹ is H, (C₁-C₄)alkyl which is unsubstituted or substituted by one or more radicals selected from the group consisting of halogen, (C₁-C₄)alkoxy, (C₃-C₆)cycloalkyl or phenyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy and (C₁-C₄)alkylthio, or (C₃-C₆)cycloalkyl, (C₂-C₄)alkenyl, (C₂-C₄)haloalkenyl, (C₂-C₄)alkynyl, phenyl, substituted phenyl or a radical of the formula
- CO - R*
in which
R* is H, (C₁-C₄)alkyl, (C₂-C₄)alkenyl or (C₂-C₄)alkynyl, each of the three last-mentioned radicals being unsubstituted or substituted by one or more radicals selected from the group consisting of halogen, (C₁-C₄)alkoxy, (C₁-C₄)alkylsulfonyl, phenyl and substituted phenyl, or is (C₁-C₄)alkoxy which is unsubstituted or substituted by one or more radicals selected from the group consisting of halogen, (C₁-C₄)alkoxy, (C₁-C₄)alkylthio, phenyl and substituted aryl, or (C₃-C₆)cycloalkyl or (C₃-C₆)cycloalkoxy, each of the last-mentioned two radicals being unsubstituted or substituted by one or more radicals selected from the group consisting of (C₁-C₂)alkyl, (C₁-C₂)alkoxy and halogen, or is a radical of the formula NR^{a}R^{b}.

3. A compound or a salt thereof as claimed in claim 1 or 2, wherein
R¹ is H, (C₁-C₄)alkyl which is unsubstituted or substituted by 1 to 3 radicals selected from the group consisting of halogen or by (C₁-C₂)alkoxy or (C₃-C₅)cycloalkyl, or is a radical of the formula -CO-R* in which
R* is H, (C₁-C₄)alkyl which is unsubstituted or substituted by one or more halogen atoms or by (C₁-C₂)alkoxy, or (C₂-C₄)alkenyl, (C₂-C₃)alkynyl, (C₃-C₅)cycloalkyl, (C₁-C₄)alkoxy which is unsubstituted or substituted by one or more halogen atoms or by phenyl, or a radical of the formula NR^{a}R^{b}, in which R^{a} and R^{b} independently of one another are H or (C₁-C₄)alkyl,
R² is H, (C₁-C₄)alkyl which is unsubstituted or substituted by one or more radicals selected from the group consisting of halogen, (C₁-C₄)alkoxy, (C₃-C₆)cycloalkyl, phenyl and substituted phenyl, or (C₃-C₆)cycloalkyl which is unsubstituted or substituted by one or more radicals selected from the group consisting of halogen, (C₁-C₄)alkyl, (C₁-C₄)alkoxy or (C₁-C₄)haloalkyl, or (C₂-C₄)alkenyl or (C₂-C₄)alkynyl, each of the lastmentioned two radicals being unsubstituted or substituted by one or more radicals selected from the group consisting of (C₁-C₄)alkoxyl (C₁-C₄)haloalkyl and halogen, or is phenyl, substituted phenyl or a radical of the formula R'R"R"'Si, in which R', R" and R"' independently of one another are (C₁-C₄)alkyl, or an acyl radical of the formula
-CO-R**
in which
R** is H, (C₁-C₄)alkyl, (C₂-C₄)alkenyl or (C₂-C₄)alkynyl, each of the three last-mentioned radicals being unsubstituted or substituted by one or more radicals selected from the group consisting of halogen, (C₁-C₄)alkoxy, (C₁-C₄)alkylsulfonyl, phenyl and substituted phenyl, or is (C₁-C₄)alkoxy, (C₃-C₄)-alkenyloxy or (C₃-C₄)alkynyloxy, each of the last-mentioned three radicals being unsubstituted or substituted by one or more radicals selected from the group consisting of halogen, (C₁-C₄)alkoxy, (C₁-C₄)alkylthio, phenyl and substituted phenyl, or is (C₃-C₆)cycloalkyl or (C₃-C₆)cycloalkoxy, each of the last-mentioned two radicals being unsubstituted or substituted by one or more radicals selected from the group consisting of halogen, (C₁-C₄)alkyl,
(C₁-C₄)alkoxy and (C₁-C₄)haloalkyl, or a radical of the formula NR^{c}R^{d}, and
R³ is (C₁-C₃)alkyl, (C₁-C₃)haloalkyl, halogen, NO₂, (C₁-C₃)alkoxy, (C₁-C₃)haloalkoxy, (C₁-C₂)alkoxy-(C₁-C₂)alkyl, acetyl, propionyl, (C₁-C₂)-alkylamino, di[(C₁-C₂)alkyl]amino, (C₁-C₃)-alkylsulfonyl, SO₂NR^{e}R^{f} or C(O)NR^{g}R^{h}.

4. A compound or a salt thereof as claimed in any of claims 1 to 3, wherein
R¹ is H, (C₁-C₄)alkyl, (C₁-C₄)haloalkyl or [(C₁-C₂)-alkoxy]-(C₁-C₂)alkyl, or a radical of the formula -CO-R* in which
R* is H, (C₁-C₄)alkyl, halo-(C₁-C₄)alkyl, (C₂-C₄)-alkenyl, (C₂-C₄)alkynyl, (C₁-C₄)alkoxy, [(C₁-C₂)alkoxy]-(C₁-C₂)alkyl, (C₃-C₅)cycloalkyl or a radical of the formula NR^{a}R^{b} in which R^{a} and R^{b} independently of one another are H or (C₁-C₄)alkyl,
R² is H, (C₁-C₄)alkyl, (C₁-C₄)haloalkyl, [(C₁-C₂)alkoxy]-(C₁-C₂)alkyl or a radical of the formula -CO-R** in which
R** is H, (C₁-C₄)alkyl, (C₁-C₄)haloalkyl, (C₃-C₄)alkenyl, (C₃-C₄)alkynyl, (C₁-C₄)alkoxy, [(C₁-C₂)alkoxy]-(C₁-C₂)alkyl, (C₃-C₅)cycloalkyl or a radical of the formula NR^{c}R^{d}, in which R^{c} and R^{d} independently of one another are H or (C₁-C₄)alkyl,
R³ is (C₁-C₄)alkyl, halogen, nitro or (C₁-C₄)alkoxy n is 0 or 1 and
A is a radical of the formula 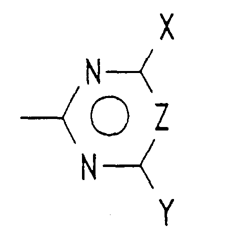 in which
one of the radicals X and Y is (C₁-C₃)alkyl, (C₁-C₃)-alkoxy, (C₁-C₃)haloalkyl, (C₁-C₃)haloalkoxy or [(C₁-C₂)alkoxy]-(C₁-C₂)alkyl and the other radical Y or X is (C₁-C₃)alkyl, (C₁-C₃)alkoxy or (C₁-C₃)alkylthio, each of the last-mentioned 3 radicals being unsubstituted or mono- or polysubstituted by halogen or mono- or disubstituted by (C₁-C₃)alkoxy or (C₁-C₃)alkylthio, or is halogen or a radical of the formula NR⁵R⁶, in which R⁵ and R⁶ independently of one another are H, (C₁-C₃)alkyl or (C₃-C₄)alkenyl, or (C₃-C₆)cycloalkyl, (C₂-C₄)alkenyl, (C₂-C₄)alkynyl, (C₃-C₄)alkenyloxy or (C₃-C₄)alkynyloxy.

5. A process for the preparation of a compound of the formula (I) or a salt thereof as defined in claim 1, which comprises
a) reacting a compound of the formula (II), 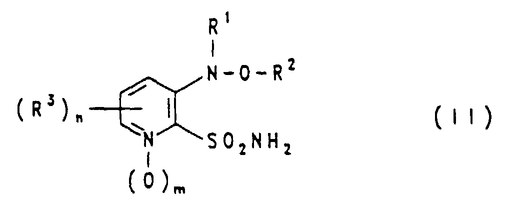 with a heterocyclic carbamate of the formula (III),
R°-O-CO-NR⁴-A (III)
in which R° is optionally substituted phenyl or (C₁-C₄)alkyl, or
b) reacting a pyridylsulfonylcarbamate of the formula (IV), 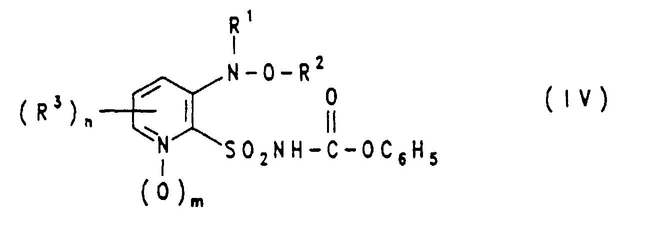 with an amino heterocycle of the formula (V)
H-NR⁴-A (V),
or
c) reacting a sulfonyl isocyanate of the formula (VI) 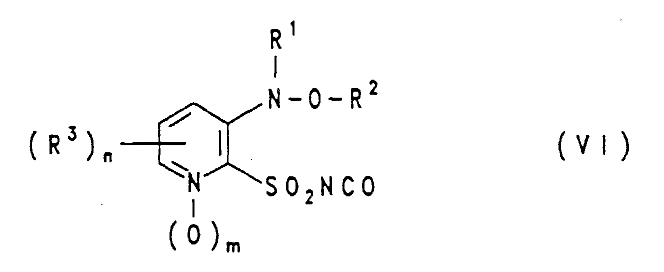 with an amino heterocycle of the formula (V)
H-NR⁴-A (V),
or
d) first reacting an amino heterocycle of the formula H-NR⁴-A (V) with phosgene in a one-pot reaction in the presence of a base, and reacting the intermediate formed with a pyridinesulfonamide of the formula (II), or
e) reacting a sulfonamide of the abovementioned formula (II) with a (thio)isocyanate of the formula (VII) 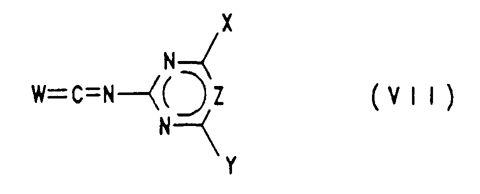 in the presence of a base,
the radicals R¹, R², R³, R⁴, A, W, X, Y and Z as well as m and n in the formulae (II)-(VII) being as defined in formula (I).

6. A herbicidal or plant growth-regulating composition, which comprises at least one compound of the formula (I) or a salt thereof as claimed in any of claims 1 to 4 and formulation auxiliaries conventionally used in crop protection.

7. A method of controlling harmful plants or of regulating the growth of plants, which comprises applying an effective amount of at least one compound of the formula (I) or a salt thereof as claimed in any of claims 1 to 4 to the harmful plants or to plants, the seeds of these plants or the area on which these plants grow.

8. The use of a compound of the formula (I) or a salt thereof as claimed in any of claims 1 to 4 as a herbicide or plant growth regulator.

9. A compound of the formula (II) 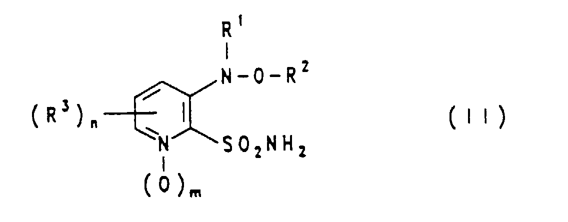 in which R¹, R², R³, n and m are as defined in formula (I) as claimed in any of claims 1 to 4.

10. A compound of the formula (VIII) 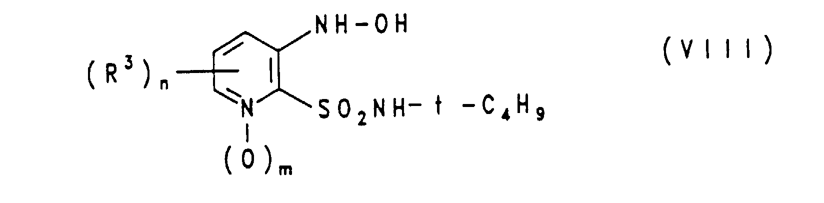 in which R¹, R², R³, n and m are as defined in formula (I) as claimed in any of claims 1 to 4.

## Revendications

1. Composés de formule (I), ou leurs sels, 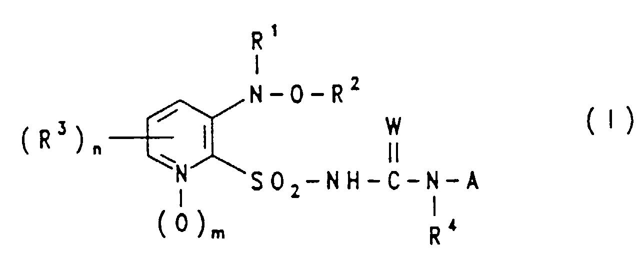 dans laquelle :
R¹ est H, un groupe alkyle en C₁-C₆, qui est non substitué ou est substitué par un ou plusieurs substituants choisis parmi l'ensemble comprenant les groupes halogéno, nitro, alcoxy en C₁-C₄, cycloalkyle en C₃-C₆, cyano, aryle et aryle substitué ; cycloalkyle en C₃-C₆, qui est non substitué ou est substitué par un ou plusieurs substituants choisis parmi l'ensemble comprenant les groupes halogéno, alkyle en C₁-C₄ et alcoxy en C₁-C₄ ; ou alcényle en C₂-C₆ ou alcynyle en C₂-C₆, où chacun des deux derniers radicaux mentionnés ci-dessus est non substitué ou est substitué par un ou plusieurs substituants choisis parmi l'ensemble comprenant les groupes halogéno, alcoxy en C₁-C₄ et cycloalkyle en C₃-C₆ ; ou encore aryle, aryle substitué, ou encore un radical acyle de formule :
-CO-R*
où :
R* est H ou un groupe alkyle en C₁-C₆, alcényle en C₂-C₆ ou alcynyle en C₂-C₆, où chacun des trois derniers radicaux est non substitué ou est substitué par un ou plusieurs substituants choisis parmi l'ensemble comprenant les groupes halogéno, alcoxy en C₁-C₄, alkylthio en C₁-C₄, alkylsulfinyle en C₁-C₄, alkylsulfonyle en C₁-C₄, nitro, cyano, thiocyanato, aryle et aryle substitué ; ou encore alcoxy en C₁-C₄, alcényloxy en C₂-C₄, alcynyloxy en C₂-C₄ ou alkylthio en C₁-C₄, où chacun des quatre derniers radicaux est non substitué ou est substitué par un ou plusieurs substituants choisis parmi l'ensemble comprenant les groupes halogéno, alcoxy en C₁-C₄, alkylthio en C₁-C₄, aryle ou aryle substitué ; ou encore cycloalkyle en C₃-C₆ ou cycloalcoxy en C₃-C₆, où chacun des deux derniers radicaux substitués ci-dessus est non substitué ou est substitué par un ou plusieurs substituants choisis parmi l'ensemble comprenant les groupes alkyle en C₁-C₄, alcoxy en C₁-C₄, alkylthio en C₁-C₄, halogénalkyle en C₁-C₄ ou halogéno ; ou encore un radical de formule NR^{a}R^{b},
R² est H ou un groupe alkyle en C₁-C₆, qui est non substitué ou est substitué par un ou plusieurs substituants choisis parmi l'ensemble comprenant les groupes halogéno, nitro, alcoxy en C₁-C₄, cycloalkyle en C₃-C₆, aryle et aryle substitué ; ou encore cycloalkyle en C₃-C₆, qui est non substitué ou est substitué par un ou plusieurs substituants choisis parmi l'ensemble comprenant les groupes halogéno, alkyle en C₁-C₄, alcoxy en C₁-C₄, halogénalkyle en C₁-C₄, aryle et aryle substitué ; ou encore alcényle en C₂-C₆ ou alcynyle en C₂-C₆, où chacun des deux derniers radicaux est non substitué ou est substitué par un ou plusieurs substituants choisis parmi l'ensemble comprenant les groupes alcoxy en C₁-C₄, halogénalkyle en C₁-C₄, cycloalkyle en C₃-C₆ et halogéno ; ou encore aryle, aryle substitué, ou un radical de formule R'R"R"'Si, où R', R", R"', indépendamment les uns des autres, représentent chacun un groupe alkyle en C₁-C₄ ; ou encore un radical acyle de formule :
-CO-R**
dans laquelle :
R** est H, un groupe alkyle en C₁-C₆, alcényle en C₂-C₆ ou alcynyle en C₂-C₆, où chacun des trois radicaux mentionnés ci-dessus est non substitué ou est substitué par un ou plusieurs substituants choisis parmi l'ensemble comprenant les groupes halogéno, alcoxy en C₁-C₄, alkylthio en C₁-C₄, alkylsulfinyle en C₁-C₄, alkylsulfonyle en C₁-C₄, nitro, cyano, thiocyanato, aryle et aryle substitué ; ou encore alcoxy en C₁-C₄, alcényloxy en C₂-C₄ ou alcynyloxy en C₂-C₄, où chacun des trois derniers radicaux ci-dessus est non substitué ou est substitué par un ou plusieurs substituants choisis parmi l'ensemble comprenant les groupes halogéno, alcoxy en C₁-C₄, alkylthio en C₁-C₄, aryle et aryle substitué ; ou encore cycloalkyle en C₃-C₆ ou cycloalcoxy en C₃-C₆, où chacun des deux derniers radicaux ci-dessus est non substitué ou est substitué par un ou plusieurs substituants choisis parmi l'ensemble comprenant les groupes halogéno, alkyle en C₁-C₄, alcoxy en C₁-C₄, alkylthio en C₁-C₄ et halogénalkyle en C₁-C₄, ou encore un radical de formule NR^{c}R^{d},
R³ est un groupe alkyle en C₁-C₄, halogénalkyle en C₁-C₃, halogéno, NO₂, CN, alcoxy en C₁-C₃, halogénalcoxy en C₁-C₃, alkylthio en C₁-C₃, (alcoxy en C₁-C₃)-(alkyle en C₁-C₃ ), (alcoxy en C₁-C₃)-carbonyle, alkylamino en C₁-C₃, di(alkyle en C₁-C₃)-amino, alkylsulfinyle en C₁-C₃, alkylsulfonyle en C₁-C₃, SO₂NR^{e}R^{f} ou C(O)NR^{g}R^{h},
R¹, R^{a}, R^{b}, R^{c}, R^{d}, R^{e}, R^{f} et R^{g} sont chacun indépendamment des autres H ou un groupe alkyle en C₁-C₄, alcényle en C₃-C₆, alcynyle en C₃-C₆, (alkyle en C₁-C₄)-carbonyle, arylcarbonyle, qui est non substitué dans le fragment aryle ou y est substitué par un ou plusieurs substituants choisis parmi l'ensemble comprenant les groupes halogéno, alkyle en C₁-C₄ et alcoxy en C₁-C₄ ; ou encore R^{a} et R^{b}, R^{c} et R^{d}, R^{e} et R^{f} ou R^{g} et R^{h} forment par paires, avec l'atome d'azote qui les relie, un noyau saturé ou insaturé hétérocyclique ayant de 3 à 7 atomes nucléaires, et qui, outre l'atome d'azote, peut contenir un ou deux autres hétéroatomes choisis parmi N, O et S, selon les états d'oxydation possibles, et qui est non substitué ou est substitué ;
R⁴ est H ou un groupe alkyle en C₁-C₄,
m vaut 0 ou 1,
n vaut 0, 1 ou 2,
A est un radical de formule : 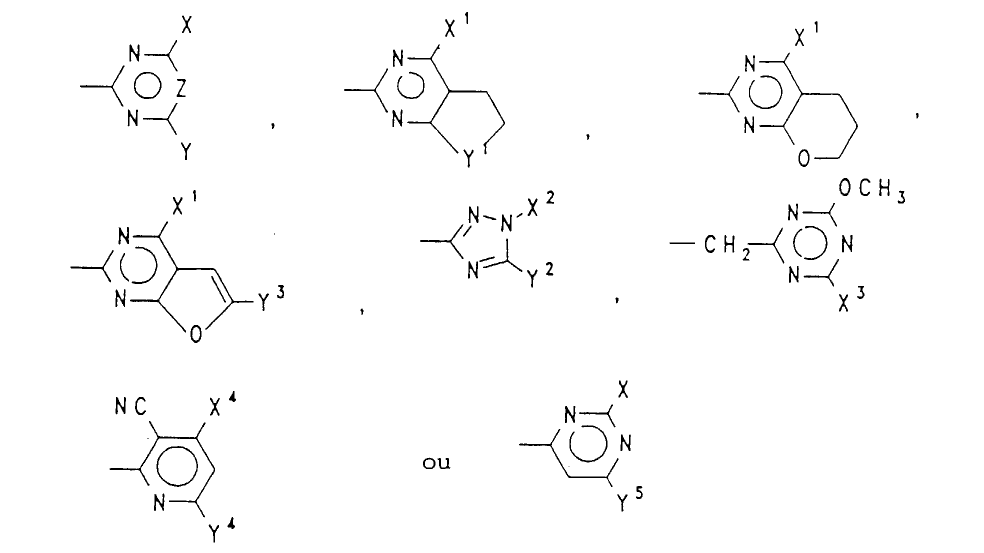
X, Y, indépendamment l'un de l'autre, sont chacun H ou un groupe halogéno, alkyle en C₁-C₃, alcoxy en C₁-C₃ ou alkylthio en C₁-C₃, où les deux derniers radicaux alkylés ci-dessus sont non substitués ou une ou plusieurs fois substitués par des groupes halogéno ou sont une ou deux fois substitués par des groupes alcoxy en C₁-C₃ ou alkylthio en C₁-C₃, ou encore un radical de formule NR⁵R⁶, cycloalkyle en C₃-C₆, alcényle en C₂-C₄, alcynyle en C₂-C₄, alcényloxy en C₃-C₄ ou alcynyloxy en C₃-C₄,
R⁵ et R⁶ sont chacun indépendamment de l'autre H ou un groupe alkyle en C₁-C₃ ou alcényle en C₃-C₄,
W est O ou S,
Z est CH ou N,
X¹ est CH₃, OCH₃, OC₂H₅ ou OCF₂H,
Y¹ est -O- ou -CH₂-.
X² est CH₃, C₂H₅ ou CH₂CF₃,
Y² est OCH₃, OC₂H₅, SCH₃, SC₂H₅, CH₃ ou C₂H₅,
X³ est CH₃ ou OCH₃,
Y³ est H ou CH₃,
X⁴ est CH₃, OCH₃, OC₂H₅, CH₂OCH₃ ou Cl,
Y⁴ est CH₃, OCH₃, OC₂H₅ ou Cl,
Y⁵ est CH₃, C₂H₅, OCH₃ ou Cl.

2. Composés ou leurs sels selon la revendication 1, caractérisés en ce que :
R¹ est H, un groupe alkyle en C₁-C₄, qui est non substitué ou est substitué par un ou plusieurs substituants choisis parmi l'ensemble comprenant les groupes halogéno, alcoxy en C₁-C₄, cycloalkyle en C₃-C₆ ou phényle qui est non substitué ou est substitué par un ou plusieurs substituants choisis parmi l'ensemble comprenant les groupes halogéno, alkyle en C₁-C₄, halogénalkyle en C₁-C₄, alcoxy en C₁-C₄, halogénalcoxy en C₁-C₄ et alkylthio en C₁-C₄, ou encore cycloalkyle en C₃-C₆, alcényle en C₂-C₄, halogénalcényle en C₂-C₄, alcynyle en C₂-C₄, phényle, phényle substitué, ou encore un radical de formule :
-CO-R*
dans laquelle :
R* est H, un groupe alkyle en C₁-C₄, alcényle en C₂-C₄ ou alcynyle en C₂-C₄, où chacun des trois derniers radicaux ci-dessus est non substitué ou est substitué par un ou plusieurs substituants choisis parmi l'ensemble comprenant les groupes halogéno, alcoxy en C₁-C₄, alkylsulfonyle en C₁-C₄, phényle et phényle substitué, ou encore alcoxy en C₁-C₄, qui est non substitué ou est substitué par un plusieurs substituants choisis parmi l'ensemble comprenant les groupes halogéno, alcoxy en C₁-C₄, alkylthio en C₁-C₄, phényle et aryle substitué, ou encore cycloalkyle en C₃-C₆ ou cycloalcoxy en C₃-C₆, où chacun des deux derniers radicaux ci-dessus est non substitué ou est substitué par un ou plusieurs substituants choisis parmi l'ensemble comprenant les groupes alkyle en C₁-C₂, alcoxy en C₁-C₂ et halogéno, ou encore un radical de formule NR^{a}R^{b}.

3. Composés ou leurs sels selon la revendication 1 ou 2, caractérisés en ce que :
R¹ est H ou un groupe alkyle en C₁-C₄, qui est non substitué ou est substitué par 1 à 3 substituants choisis parmi les halogènes et/ou les radicaux alcoxy en C₁-C₂ ou cycloalkyle en C₃-C₅, ou encore un radical de formule -CO-R*, dans laquelle
R* est H ou un groupe alkyle, qui est non substitué ou est substitué par un ou plusieurs atomes d'halogène ou groupes alcoxy en C₁-C₂, ou encore alcényle en C₂-C₄, alcynyle en C₂-C₃, cycloalkyle en C₃-C₅, alcoxy en C₁-C₄, qui est non substitué ou est substitué par un ou plusieurs atomes d'halogène ou groupes phényle, ou encore un radical de formule NR^{a}R^{b}, où R^{a} et R^{b}, indépendamment l'un de l'autre, sont chacun H ou un groupe alkyle en C₁-C₄,
R² est H, un groupe alkyle en C₁-C₄, qui est non substitué ou est substitué par un ou plusieurs substituants choisis parmi l'ensemble comprenant les groupes halogéno, alcoxy en C₁-C₄, cycloalkyle en C₃-C₆, phényle et phényle substitué ; ou encore cycloalkyle en C₃-C₆, qui est non substitué ou est substitué par un ou plusieurs substituants choisis parmi l'ensemble comprenant les groupes halogéno, alkyle en C₁-C₄, alcoxy en C₁-C₄ ou halogénalkyle en C₁-C₄ ; ou encore alcényle en C₂-C₄ ou alcynyle en C₂-C₄, où chacun des deux derniers radicaux ci-dessus est non substitué ou est substitué par un ou plusieurs substituants choisis parmi l'ensemble comprenant les groupes alcoxy en C₁-C₄, halogénalkyle en C₁-C₄ ou halogéno ; ou encore phényle, phényle substitué, ou un radical de formule R'R"R"'Si où R', R", R"' sont chacun indépendamment l'un de l'autre un groupe alkyle en C₁-C₄, ou encore un radical acyle de formule :
-CO-R**
dans laquelle :
R** est H, un groupe alkyle en C₁-C₄, alcényle en C₂-C₄ ou alcynyle en C₂-C₄, où les trois derniers radicaux mentionnés ci-dessus sont non substitués ou sont substitués par un ou plusieurs substituants choisis parmi l'ensemble comprenant les groupes halogéno, alcoxy en C₁-C₄, alkylsulfonyle en C₁-C₄, phényle et phényle substitué ; ou encore alcoxy en C₁-C₄, alcényloxy en C₃-C₄ ou alcynyloxy en C₃-C₄, où chacun des trois derniers radicaux ci-dessus est non substitué ou est substitué par un ou plusieurs radicaux choisis parmi l'ensemble comprenant les groupes halogéno, alcoxy en C₁-C₄, alkylthio en C₁-C₄, phényle et phényle substitué ; ou encore cycloalkyle en C₃-C₆ ou cycloalcoxy en C₃-C₆, où chacun des deux derniers radicaux ci-dessus est non substitué ou est substitué par un ou plusieurs substituants choisis parmi l'ensemble comprenant les groupes halogéno, alkyle en C₁-C₄, alcoxy en C₁-C₄ et halogénalkyle en C₁-C₄ ; ou encore un radical de formule NR^{c}R^{d}, et
R³ est un groupe alkyle en C₁-C₃, halogénalkyle en C₁-C₃, halogéno, NO₂, alcoxy en C₁-C₃, halogénalcoxy en C₁-C₃, (alcoxy en C₁-C₂)(alkyle en C₁-C₂), acétyle, propionyle, alkylamino en C₁-C₂, di(alkyle en C₁-C₂)amino, alkylsulfonyle en C₁-C₃, SO₂NR^{e}R^{f} ou C(O)NR^{g}R^{h}.

4. Composés ou leurs sels selon l'une des revendications 1 à 3, caractérisés en ce que :
R¹ est H ou un groupe alkyle en C₁-C₄, halogénalkyle en C₁-C₄ ou (alcoxy en C₁-C₂)(alkyle en C₁-C₂), ou encore un radical de formule -CO-R*, où
R* est H ou un groupe alkyle en C₁-C₄, halogénalkyle en C₁-C₄, alcényle en C₂-C₄, alcynyle en C₂-C₄, alcoxy en C₁-C₄, (alcoxy en C₁-C₂)(alkyle en C₁-C₂), cycloalkyle en C₃-C₅, ou encore un radical de formule NR^{a}R^{b}, où R^{a} et R^{b}, indépendamment l'un de l'autre, sont chacun H ou un groupe alkyle en C₁-C₄,
R² est H, un groupe alkyle en C₁-C₄, halogénalkyle en C₁-C₄, (alcoxy en C₁-C₄)-(alkyle en C₁-C₄) ou un radical de formule -CO-R** où :
R** est H ou un groupe alkyle en C₁-C₄, halogénalkyle en C₁-C₄, alcényle en C₃-C₄, alcynyle en C₃-C₄, alcoxy en C₁-C₄, (alcoxy en C₁-C₂)-(alkyle en C₁-C₂), cycloalkyle en C₃-C₅, ou encore un radical de formule NR^{c}R^{d} dans laquelle R^{c} et R^{d} sont chacun indépendamment l'un de l'autre H ou un groupe alkyle en C₁-C₄.
R³ est un groupe alkyle en C₁-C₄, halogéno, nitro ou alcoxy en C₁-C₄,
n vaut 0 ou 1, et
A est un résidu de formule : 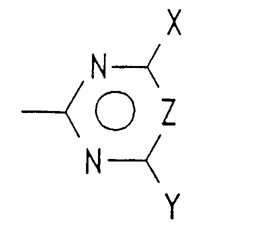 dans laquelle :
l'un des radicaux X et Y est un groupe alkyle en C₁-C₃, alcoxy en C₁-C₃, halogénalkyle en C₁-C₃, halogénalcoxy en C₁-C₃ ou (alcoxy en C₁-C₂)-(alkyle en C₁-C₂), et l'autre radical, respectivement Y et X, est un groupe alkyle en C₁-C₃, alcoxy en C₁-C₃, alkylthio en C₁-C₃, où chacun des trois derniers radicaux ci-dessus est non substitué ou est une ou plusieurs fois substitué par des halogènes ou une ou deux fois par des groupes alcoxy en C₁-C₃ ou alkylthio en C₁-C₃, ou encore un halogène, ou encore un radical de formule NR⁵R⁶, où R⁵ et R⁶, indépendamment l'un de l'autre, sont chacun H ou un groupe alkyle en C₁-C₃ ou alcényle en C₃-C₄, ou encore cycloalkyle en C₃-C₆, alcényle en C₂-C₄, alcynyle en C₂-C₄, alcényloxy en C₃-C₄ ou alcynyloxy en C₃-C₄.

5. Procédé de préparation des composés de formule (I) ou de leurs sels selon la revendication 1, caractérisé en ce que:
a) on fait réagir un composé de formule (II) : 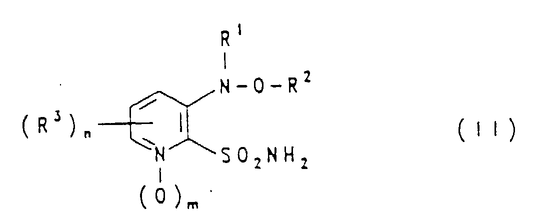 avec un carbamate hétérocyclique de formule (III) :
R^{o}-O-CO-NR⁴-A (III)
dans laquelle R^{o} est un groupe phényle éventuellement substitué ou alkyle en C₁-C₄, ou bien
b) on fait réagir un carbamate de pyridylsulfonyle de formule (IV) : 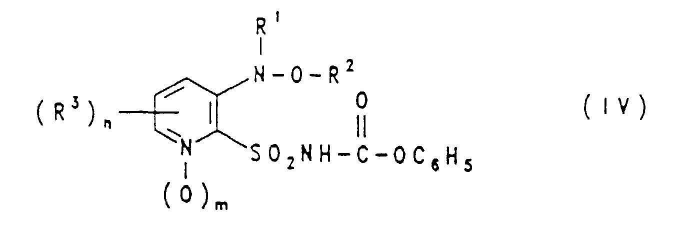 avec un composé aminohétérocyclique de formule (V) :
H-NR⁴-A (V)
ou bien
c) on fait réagir un isocyanate de sulfonyle de formule (VI) : 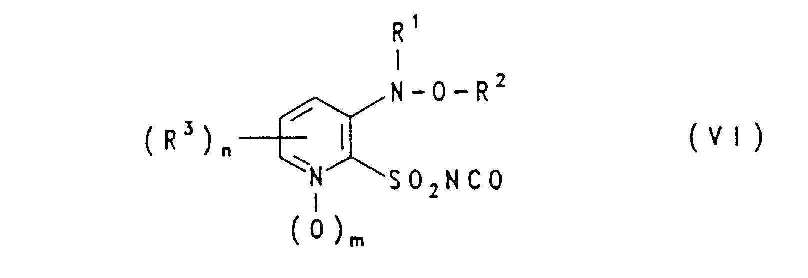 avec un composé aminohétérocyclique de formule (V) :
H-NR⁴-A (V)
ou bien
d) dans le cadre d'une réaction en un pot, on fait réagir d'abord un composé aminohétérocyclique de formule H-NR⁴-A (V) en présence d'une base telle par exemple que la triéthylamine, avec du phosgène, et on fait réagir l'intermédiaire ainsi formé avec un pyridinesulfonamide de formule (II), ou bien
e) on fait réagir un sulfonamide ayant la formule (II) ci-dessus avec un (thio)isocyanate de formule (VII) : 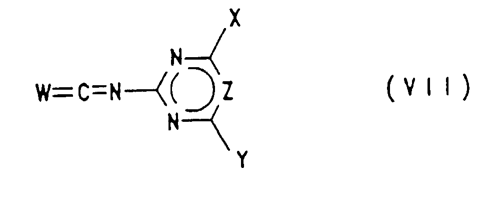 en présence d'une base, par exemple le carbonate de potassium ou la triéthylamine,
où dans les formules (II)-(VII), les radicaux R¹, R², R³, R⁴, A, W, X, Y et Z, ainsi que m et n, sont tels que définis dans la formule (I).

6. Herbicide ou agent régulateur de la croissance des végétaux, caractérisé en ce qu'il contient au moins un composé de formule (I) ou ses sels, selon l'une des revendications 1 à 4, et des adjuvants de formulation usuels en phytopharmacie.

7. Procédé pour maîtriser des plantes nuisibles ou pour réguler la croissance de végétaux, caractérisé en ce qu'on applique une quantité efficace d'au moins un composé de formule (I) ou de ses sels selon l'une des revendications 1 à 4 sur les plantes nuisibles ou sur les végétaux, leurs semences, ou sur la surface sur laquelle poussent les végétaux.

8. Utilisation des composés de formule (I) ou de leurs sels selon l'une des revendications 1 à 4, en tant qu'herbicides ou régulateurs de la croissance des végétaux.

9. Composés de formule (II) : 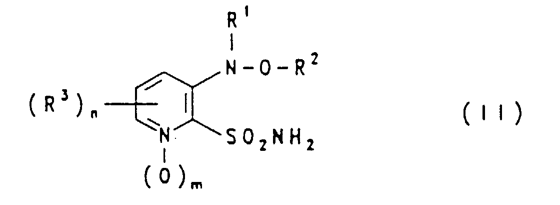 dans laquelle R¹, R², R³, n et m ont les significations données dans la formule (I) selon l'une des revendications 1 à 4.

10. Composés de formule (VIII) : 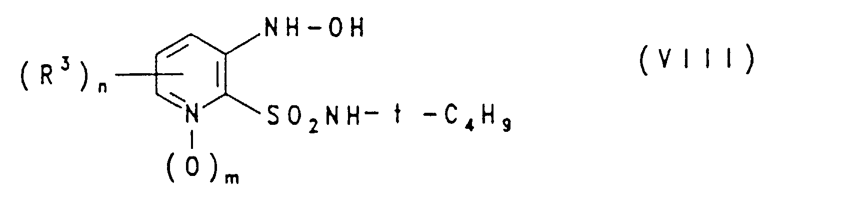 dans laquelle R¹, R², R³, n et m sont tels que définis dans la formule (I) selon l'une des revendications 1 à 4.
